# EUROPEAN PATENT APPLICATION

(11) **EP 3 037 533 A1**
(43) Date of publication of application: **29.06.2016**
(21) Application number: 14838190.8
(22) Date of filing: 20.08.2014
(51) Int. Cl.: C12N 15/09, A61K 39/395, A61P 31/16, C07K 16/10, C12N 1/15, C12N 1/19, C12N 1/21, C12N 5/10, C12P 21/08

(54) **ANTI-INFLUENZA VIRUS-NEUTRALIZING ANTIBODY**

(30) Priority: 23.08.2013 JP 2013172837
(71) Applicant: Fujita Health University, Aichi 470-1192 (JP); The Research Foundation for Microbial Diseases of Osaka University, Suita-shi, Osaka 565-0871 (JP); Perseus Proteomics Inc., Tokyo 153-0041 (JP)
(72) Inventor: KUROSAWA, Yoshikazu, Toyoake-shi Aichi 470-1192 (JP); OSHIMA, Nobuko, Toyoake-shi Aichi 470-1192 (JP); OKUNO, Yoshinobu, Kanonji-shi Kagawa 768-0061 (JP); MITOMO, Katsuyuki, Tokyo 153-0041 (JP); KOUDA, Katsushi, Tokyo 153-0041 (JP)
(74) Representative: Hoffmann Eitle
(86) International application number: PCT/JP2014/071812
(87) International publication number: WO 2015/025900

(57) **Abstract**

It is an object of the present invention to provide a novel antibody having a high binding activity and a high neutralizing activity on influenza viruses. The present invention provides an antibody, which neutralizes H1 influenza virus and/or H5 influenza virus, wherein the antibody has a heavy chain variable region having CDRs consisting of a defined heavy chain first complementarity-determining region (VH CDR1), a defined heavy chain second complementarity-determining region (VH CDR2) and a defined heavy chain third complementarity-determining region (VH CDR3), and a light chain variable region having CDRs consisting of a defined light chain second complementarity-determining region (VL CDR2) and a defined light chain third complementarity-determining region (VL CDR3).

## Description

### Technical Field

The present invention relates to an anti-influenza virus antibody that exhibits a neutralizing activity on influenza virus.

### Background Art

Influenza virus is an RNA enveloped virus that belongs to Orthomyxoviridae and has a particle size of approximately 100 nm in diameter, and it is divided into types A, B and C, based on the antigenicity of the internal protein thereof. The influenza virus consists of a ribonucleic acid (RNA) core associated with an internal nucleocapsid or nuclear protein, surrounded by a viral envelope, having a double-layered lipid structure, and an external glycoprotein. The inner layer of the viral envelope is mainly composed of a matrix protein, and a majority part of the outer layer is composed of a host-derived lipid substance. The RNA of the influenza virus has a segmental structure. Influenza, which spreads over the world, is caused by influenza A virus. The type A influenza virus has two types of envelope glycoproteins, hemagglutinin (HA) and neuraminidase (NA). Depending on a difference in the antigenicity, HA is divided into 16 subtypes, and NA is divided into 9 subtypes.

Recently, highly pathogenic avian influenza virus H5N1 has been prevalent over the world, and under the current circumstances, it will not be surprised even if a new virus that transmits from human to human will appear and will cause a pandemic. As measures for such situation, an international virus inspection system has been established, and a large stock of therapeutic agents such as Tamiflu and the development, production and stock of vaccines have been promoted. However, while the measures have been taken against such a new virus, many questions have remained ambiguous (e.g., When and in what a form a new-virus will appear? At that time, does Tamiflu or the like exhibit therapeutic effects? Is the developed vaccine effective on the new virus? When and to whom the vaccine is given? When a state of high alert will be initiated and terminated? etc.). This is because we are facing the first situation in human history, namely, preparation of vaccines and therapeutic agents against pathogens or viruses that have not yet appeared. In particular, as a problem on the development of vaccines, with regard to the influenza virus genome, many mutations have been introduced into a hemagglutinin gene every year, so that it has caused an antigenic drift (a change in the antigenicity), and this antigenic drift is considered to cause an epidemic spread of influenza. Accordingly, if a vaccine that is incompatible with the type of epidemic influenza were given, the preventive effects could not be anticipated. The reason why the development of antibody therapeutics (preventive agents) has not been conventionally attempted is that when a therapeutic agent has been developed, the developed agent would probably become useless because the properties of a virus that could have been neutralized by the developed agent have already changed due to the antigenic drift.

The present inventors have screened a phage display human antibody library produced from a large amount of B lymphocytes collected from a single subject, against twelve influenza virus H3N2 strains, which had been separated from 1968 to 2004. As a result, the inventors have found that a majority of clones exhibiting a neutralizing activity are anti-hemagglutinin antibodies, and that these anti-hemagglutinin antibodies are broadly divided into three groups, namely, antibodies specifically neutralizing the virus strains separated from 1968 to 1973, antibodies specifically neutralizing the virus strains separated from 1977 to 1993, and antibodies specifically neutralizing the virus strains separated from 1997 to 2003 (Non Patent Literature 1). These findings defy the common technical knowledge that the development of antibody therapeutics (preventive agents) against influenza virus is senseless. However, to date, such findings have not been studied vigorously for such a reason that the combination of a heavy chain with a light chain in a phage antibody library does not necessarily reflect the situation in a living organism.

Under such circumstances, three study groups have been succeeded, by themselves, in isolation of human monoclonal antibodies that neutralize H5 influenza virus (Patent Literatures 1 and 2, and Non Patent Literature 2 to 4). It have been demonstrated that these antibodies exhibit a neutralizing activity, not only on the type H5 influenza virus, but also on influenza viruses of other subtypes (e.g., type H1). However, 16 subtypes (H1 to H16) of hemagglutinin are broadly classified into two groups (Groups 1 and 2), depending on a difference in the antigenicity.

Moreover, an isolated antibody, which neutralizes at least one influenza virus selected from Group 1 consisting of influenza viruses of subtypes H1, H2, H5, H6, H8, H9, H11, H12, H13 and H16, and at least one influenza virus selected from Group 2 consisting of influenza viruses of subtypes H3, H4, H7, H10, H14 and H15, has been reported (Patent Literature 3).

### Prior Art Literatures

### Patent Literatures

Patent Literature 1: WO 2007/134327
Patent Literature 2: WO 2008/028946
Patent Literature 3: WO 2012/029997

### Non Patent Literatures

Non Patent Literature 1: Virology, Vol. 397, pp. 322-330, 2010
Non Patent Literature 2: Proc. Natl. Acad. Sci. USA., Vol. 105, pp. 5986-5991, 2008
Non Patent Literature 3: PLOS ONE, Vol. 3, PP. 5986-5991, e3942,2008
Non Patent Literature 4: Nature Structural & Molecular Biology, Vol. 16, pp. 265-273, 2009.

### Summary of Invention

### Object to be Solved by the Invention

In preparation for the coming pandemic of H5N1 influenza and the expected future pandemic of H7 and H9 viruses, it has been strongly desired to develop a preventive measure that is based on a novel concept which is more universal and reliable and is substituted for the conventional preventive concept against influenza viruses wherein a change in the antigenicity is predicted and a vaccine is designed. It is an object of the present invention to provide a novel antibody having a high binding activity and a high neutralizing activity on influenza viruses.

### Means for Solving the Object

The present inventors have conducted intensive studies directed towards achieving the aforementioned object. As a result, the inventors have succeeded in obtaining a novel antibody capable of neutralizing H1 influenza virus, thereby completing the present invention.

Specifically, the present invention provides the following inventions.
[1] An antibody, which neutralizes H1 influenza virus and/or H5 influenza virus, wherein the antibody has a heavy chain variable region having CDRs consisting of a heavy chain first complementarity-determining region (VH CDR1), a heavy chain second complementarity-determining region (VH CDR2) and a heavy chain third complementarity-determining region (VH CDR3), which are described in any one of the following (1) to (29), and a light chain variable region having CDRs consisting of a light chain first complementarity-determining region (VL CDR1), a light chain second complementarity-determining region (VL CDR2) and a light chain third complementarity-determining region (VL CDR3), which are described in any one of the following (1) to (29):
   (1) VH CDR1 of SEQ ID NO: 1, VH CDR2 of SEQ ID NO: 2, VH CDR3 of SEQ ID NO: 3, VL CDR1 of SEQ ID NO: 4, VL CDR2 of SEQ ID NO: 5, and VL CDR3 of SEQ ID NO: 6,
   (2) VH CDR1 of SEQ ID NO: 7, VH CDR2 of SEQ ID NO: 8, VH CDR3 of SEQ ID NO: 9, VL CDR1 of SEQ ID NO: 10, VL CDR2 of SEQ ID NO: 11, and VL CDR3 of SEQ ID NO: 12,
   (3) VH CDR1 of SEQ ID NO: 13, VH CDR2 of SEQ ID NO: 14, VH CDR3 of SEQ ID NO: 15, VL CDR1 of SEQ ID NO: 16, VL CDR2 of SEQ ID NO: 17, and VL CDR3 of SEQ ID NO: 18,
   (4) VH CDR1 of SEQ ID NO: 19, VH CDR2 of SEQ ID NO: 20, VH CDR3 of SEQ ID NO: 21, VL CDR1 of SEQ ID NO: 22, VL CDR2 of SEQ ID NO: 23, and VL CDR3 of SEQ ID NO: 24,
   (5) VH CDR1 of SEQ ID NO: 25, VH CDR2 of SEQ ID NO: 26, VH CDR3 of SEQ ID NO: 27, VL CDR1 of SEQ ID NO: 28, VL CDR2 of SEQ ID NO: 29, and VL CDR3 of SEQ ID NO: 30,
   (6) VH CDR1 of SEQ ID NO: 31, VH CDR2 of SEQ ID NO: 32, VH CDR3 of SEQ ID NO: 33, VL CDR1 of SEQ ID NO: 34, VL CDR2 of SEQ ID NO: 35, and VL CDR3 of SEQ ID NO: 36,
   (7) VH CDR1 of SEQ ID NO: 37, VH CDR2 of SEQ ID NO: 38, VH CDR3 of SEQ ID NO: 39, VL CDR1 of SEQ ID NO: 40, VL CDR2 of SEQ ID NO: 41, and VL CDR3 of SEQ ID NO: 42,
   (8) VH CDR1 of SEQ ID NO: 43, VH CDR2 of SEQ ID NO: 44, VH CDR3 of SEQ ID NO: 45, VL CDR1 of SEQ ID NO: 46, VL CDR2 of SEQ ID NO: 47, and VL CDR3 of SEQ ID NO: 48,
   (9) VH CDR1 of SEQ ID NO: 49, VH CDR2 of SEQ ID NO: 50, VH CDR3 of SEQ ID NO: 51, VL CDR1 of SEQ ID NO: 52, VL CDR2 of SEQ ID NO: 53, and VL CDR3 of SEQ ID NO: 54,
   (10) VH CDR1 of SEQ ID NO: 55, VH CDR2 of SEQ ID NO: 56, VH CDR3 of SEQ ID NO: 57, VL CDR1 of SEQ ID NO: 58, VL CDR2 of SEQ ID NO: 59, and VL CDR3 of SEQ ID NO: 60,
   (11) VH CDR1 of SEQ ID NO: 61, VH CDR2 of SEQ ID NO: 62, VH CDR3 of SEQ ID NO: 63, VL CDR1 of SEQ ID NO: 64, VL CDR2 of SEQ ID NO: 65, and VL CDR3 of SEQ ID NO: 66,
   (12) VH CDR1 of SEQ ID NO: 67, VH CDR2 of SEQ ID NO: 68, VH CDR3 of SEQ ID NO: 69, VL CDR1 of SEQ ID NO: 70, VL CDR2 of SEQ ID NO: 71, and VL CDR3 of SEQ ID NO: 72,
   (13) VH CDR1 of SEQ ID NO: 73, VH CDR2 of SEQ ID NO: 74, VH CDR3 of SEQ ID NO: 75, VL CDR1 of SEQ ID NO: 76, VL CDR2 of SEQ ID NO: 77, and VL CDR3 of SEQ ID NO: 78,
   (14) VH CDR1 of SEQ ID NO: 79, VH CDR2 of SEQ ID NO: 80, VH CDR3 of SEQ ID NO: 81, VL CDR1 of SEQ ID NO: 82, VL CDR2 of SEQ ID NO: 83, and VL CDR3 of SEQ ID NO: 84,
   (15) VH CDR1 of SEQ ID NO: 85, VH CDR2 of SEQ ID NO: 86, VH CDR3 of SEQ ID NO: 87, VL CDR1 of SEQ ID NO: 88, VL CDR2 of SEQ ID NO: 89, and VL CDR3 of SEQ ID NO: 90,
   (16) VH CDR1 of SEQ ID NO: 91, VH CDR2 of SEQ ID NO: 92, VH CDR3 of SEQ ID NO: 93, VL CDR1 of SEQ ID NO: 94, VL CDR1 of SEQ ID NO: 95, and VL CDR3 of SEQ ID NO: 96,
   (17) VH CDR1 of SEQ ID NO: 97, VH CDR2 of SEQ ID NO: 98, VH CDR3 of SEQ ID NO: 99, VL CDR1 of SEQ ID NO: 100, VL CDR2 of SEQ ID NO: 101, and VL CDR3 of SEQ ID NO: 102,
   (18) VH CDR1 of SEQ ID NO: 103, VH CDR2 of SEQ ID NO: 104, VH CDR3 of SEQ ID NO: 105, VL CDR1 of SEQ ID NO: 106, VL CDR2 of SEQ ID NO: 107, and VL CDR3 of SEQ ID NO: 108,
   (19) VH CDR1 of SEQ ID NO: 109, VH CDR2 of SEQ ID NO: 110, VH CDR3 of SEQ ID NO: 111, VL CDR1 of SEQ ID NO: 112, VL CDR2 of SEQ ID NO: 113, and VL CDR3 of SEQ ID NO: 114,
   (20) VH CDR1 of SEQ ID NO: 115, VH CDR2 of SEQ ID NO: 116, VH CDR3 of SEQ ID NO: 117, VL CDR1 of SEQ ID NO: 118, VL CDR2 of SEQ ID NO: 119, and VL CDR3 of SEQ ID NO: 120,
   (21) VH CDR1 of SEQ ID NO: 121, VH CDR2 of SEQ ID NO: 122, VH CDR3 of SEQ ID NO: 123, VL CDR1 of SEQ ID NO: 124, VL CDR2 of SEQ ID NO: 125, and VL CDR3 of SEQ ID NO: 126,
   (22) VH CDR1 of SEQ ID NO: 127, VH CDR2 of SEQ ID NO: 128, VH CDR3 of SEQ ID NO: 129, VL CDR1 of SEQ ID NO: 130, VL CDR2 of SEQ ID NO: 131, and VL CDR3 of SEQ ID NO: 132,
   (23) VH CDR1 of SEQ ID NO: 133, VH CDR2 of SEQ ID NO: 134, VH CDR3 of SEQ ID NO: 135, VL CDR1 of SEQ ID NO: 136, VL CDR2 of SEQ ID NO: 137, and VL CDR3 of SEQ ID NO: 138,
   (24) VH CDR1 of SEQ ID NO: 139, VH CDR2 of SEQ ID NO: 140, VH CDR3 of SEQ ID NO: 141, VL CDR1 of SEQ ID NO: 142, VL CDR2 of SEQ ID NO: 143, and VL CDR3 of SEQ ID NO: 144,
   (25) VH CDR1 of SEQ ID NO: 145, VH CDR2 of SEQ ID NO: 146, VH CDR3 of SEQ ID NO: 147, VL CDR1 of SEQ ID NO: 148, VL CDR2 of SEQ ID NO: 149, and VL CDR3 of SEQ ID NO: 150,
   (26) VH CDR1 of SEQ ID NO: 151, VH CDR2 of SEQ ID NO: 152, VH CDR3 of SEQ ID NO: 153, VL CDR1 of SEQ ID NO: 154, VL CDR2 of SEQ ID NO: 155, and VL CDR3 of SEQ ID NO: 156,
   (27) VH CDR1 of SEQ ID NO: 157, VH CDR2 of SEQ ID NO: 158, VH CDR3 of SEQ ID NO: 159, VL CDR1 of SEQ ID NO: 160, VL CDR2 of SEQ ID NO: 161, and VL CDR3 of SEQ ID NO: 162,
   (28) VH CDR1 of SEQ ID NO: 163, VH CDR2 of SEQ ID NO: 164, VH CDR3 of SEQ ID NO: 165, VL CDR1 of SEQ ID NO: 166, VL CDR2 of SEQ ID NO: 167, and VL CDR3 of SEQ ID NO: 168, and
   (29) VH CDR1 of SEQ ID NO: 169, VH CDR2 of SEQ ID NO: 170, VH CDR3 of SEQ ID NO: 171, VL CDR1 of SEQ ID NO: 172, VL CDR2 of SEQ ID NO: 173, and VL CDR3 of SEQ ID NO: 174.
[2] The antibody according to [1], wherein the heavy chain variable region and the light chain variable region, respectively, consist of amino acid sequences having the amino acid sequence numbers described in any one of the following (1) to (29):
   (1) SEQ ID NO: 176 and SEQ ID NO: 178,
   (2) SEQ ID NO: 180 and SEQ ID NO: 182,
   (3) SEQ ID NO: 184 and SEQ ID NO: 186,
   (4) SEQ ID NO: 188 and SEQ ID NO: 190,
   (5) SEQ ID NO: 192 and SEQ ID NO: 194,
   (6) SEQ ID NO: 196 and SEQ ID NO: 198,
   (7) SEQ ID NO: 200 and SEQ ID NO: 202,
   (8) SEQ ID NO: 204 and SEQ ID NO: 206,
   (9) SEQ ID NO: 208 and SEQ ID NO: 210,
   (10) SEQ ID NO: 212 and SEQ ID NO: 214,
   (11) SEQ ID NO: 216 and SEQ ID NO: 218,
   (12) SEQ ID NO: 220 and SEQ ID NO: 222,
   (13) SEQ ID NO: 224 and SEQ ID NO: 226,
   (14) SEQ ID NO: 228 and SEQ ID NO: 230,
   (15) SEQ ID NO: 232 and SEQ ID NO: 234,
   (16) SEQ ID NO: 236 and SEQ ID NO: 238,
   (17) SEQ ID NO: 240 and SEQ ID NO: 242,
   (18) SEQ ID NO: 244 and SEQ ID NO: 246,
   (19) SEQ ID NO: 248 and SEQ ID NO: 250,
   (20) SEQ ID NO: 252 and SEQ ID NO: 254,
   (21) SEQ ID NO: 256 and SEQ ID NO: 258,
   (22) SEQ ID NO: 260 and SEQ ID NO: 262,
   (23) SEQ ID NO: 264 and SEQ ID NO: 266,
   (24) SEQ ID NO: 268 and SEQ ID NO: 270,
   (25) SEQ ID NO: 272 and SEQ ID NO: 274,
   (26) SEQ ID NO: 276 and SEQ ID NO: 278,
   (27) SEQ ID NO: 280 and SEQ ID NO: 282,
   (28) SEQ ID NO: 284 and SEQ ID NO: 286, and
   (29) SEQ ID NO: 288 and SEQ ID NO: 290.
[3] The antibody according to [1] or [2], which is an antibody fragment selected from the group consisting of Fab, Fab', F(ab')₂, a single chain antibody (scFv), a dimerized V region (Diabody), a disulfide stabilized V region (dsFv), and a peptide comprising CDR.
[4] DNA encoding the antibody according to any one of [1] to [3].
[5] A recombinant vector comprising the DNA according to [4].
[6] A transformed cell line obtained by introducing the recombinant vector according to [5] into a host cell.
[7] A method for producing the antibody according to any one of [1] to [3], which comprises culturing the transformed cell line according to [6] in a medium, then allowing the transformed cell line to generate and accumulate the antibody according to any one of [1] to [3] in a culture, and then collecting the antibody from the culture.
[8] A pharmaceutical composition comprising the antibody according to any one of [1] to [3].
[9] A passive immunotherapeutic agent against influenza, which comprises the antibody according to any one of [1] to [3].
[10] A passive immunotherapy against influenza, which comprises administering the antibody according to any one of [1] to [3] to a living body.
[11] The antibody according to any one of [1] to [3], which is used as a passive immunotherapeutic agent against influenza.
[12] Use of the antibody according to any one of [1] to [3] for production of a passive immunotherapeutic agent against influenza.

### Advantageous Effects of Invention

According to the present invention, a human antibody, which has universality as a single antibody against all influenza viruses belonging to Group 1, and also has a neutralizing activity sufficient for the treatment of humans, can be provided. In addition, an antibody capable of retaining a neutralizing activity also on influenza viruses belonging to Group 2 is also provided. That is to say, by passive immunization with the aforementioned neutralizing antibody, it becomes possible to effectively prevent or treat influenza with the antibody of the present invention, not only when an antigenic drift occurs, but also when currently unknown influenza, which will appear upon the occurrence of an antigen sift, becomes pandemic (for example, the pandemic of avian influenza).

### Brief Description of Drawings

[Figure 1] Figure 1 shows the schedule of vaccination and blood collection.
[Figure 2] Figure 2 shows the neutralization kinetic activity of representative clones.

### Embodiments for Carrying out the Invention

In the present description, the influenza virus includes the currently known subtypes of Group 1, and also subtypes, which will be isolated and identified in the future. The currently known influenza virus subtypes include subtypes each consisting of the combination of a type selected from hemagglutinin H1 to H16 and a type selected from neuraminidase N1 to N9.

The influenza virus is broadly divided into two groups depending on a difference in the antigenicity of hemagglutinin. In the present description, the group consisting of influenza viruses of types H1, H2, H5, H6, H8, H9, H11, H12, H13 and H16 is defined as Group 1, and the group consisting of influenza viruses of types H3, H4, H7, H10, H14 and H15 is defined as Group 2. Upon the category selection of these groups, the type of neuraminidase is not considered. Even regarding novel subtypes to be isolated and identified in the figure, the novel subtypes are classified into Group 1 or Group 2, depending on the similarity of the amino acid sequence of hemagglutinin.

The antibody of the present invention is preferably an antibody, which neutralizes at least one influenza virus selected from the aforementioned Group 1 (types H1, H2, H5, H6, H8, H9, H11, H12, H13 and H16) and at least one influenza virus selected from the aforementioned Group 2 (types H3, H4, H7, H10, H14 and H15). The neutralizing antibody of the present invention more preferably neutralizes at least H1 and/or H5 influenza viruses, among the influenza viruses of Group 1. The neutralizing antibody of the present invention particularly preferably neutralizes all of H1 to H16 influenza viruses.

The neutralizing antibody of the present invention can be produced by a method comprising the following steps (1) to (5):
(1) a step of providing an antibody library comprising antibody clones derived from approximately 10⁸ or more B cells collected from a single subject;
(2) a step of allowing influenza virus of any subtype of Group 1, a hemagglutinin protein of the virus, or an extracellular domain thereof to come into contact as an antigen with the antibody library of the step (1), and comprehensively selecting antibody clones that react with the antigen;
(3) a step of recovering an antibody molecule from each antibody clone selected in the step (2);
(4) a step of examining each antibody obtained in the step (3), in terms of its neutralizing activity on at least one influenza virus selected from Group 1 and at least one influenza virus selected from Group 2; and
(5) a step of allowing a clone that produces an antibody neutralizing influenza virus to produce the antibody, and then recovering the produced antibody.

The type of a donor, from which B cells serving as antibody-producing cells for production of an antibody library are collected, is not particularly limited, as long as it is any given mammal (e.g., a human, a swine, a horse, etc.) or any given avian (e.g., a chicken, a canard, etc.), which has been infected with influenza virus. Depending on a target to which the neutralizing antibody of the present invention is to be given by passive immunization, an animal of the same type as the target can be appropriately selected. The donor is preferably a human. In a case where the donor is a human, the age and sex of the donor, the presence or absence of vaccination, and the like are not particularly limited. Since it is desired for the donor to have been infected with influenza virus many times, the donor is preferably 20 years of age and older, more preferably 30 years of age and older, even more preferably 40 years of age and older, and particularly preferably 50 years of age and older, but the age of the donor is not limited thereto. A human who has not had an onset of influenza A for a certain period of time in the past is more desirable.

The amount of blood sampling for collection of B cells that are to be used in production of a common antibody library is approximately 20 to 30 mL, and the amount of B cells contained in this amount of blood is approximately 10⁷ cells. In all groups, from which a human neutralizing antibody reacting against highly pathogenic avian influenza virus H5N1 has been isolated, an ordinary amount of blood has been collected from each of a plurality of donors, and the collected blood samples have been then gathered to produce an antibody library of approximately 10¹⁰ clones. In contrast, the present inventors have intended to thoroughly (comprehensively) obtain antibodies that bind to hemagglutinin of a certain subtype, and have attempted to produce an antibody library with a size that reflects all antibody repertoires. That is, in general, the amount of blood collected from a human once is at most approximately 200 to 300 ml. Thus, the amount of B cells that can be collected by this method is at most approximately 10⁸ cells. Hence, the present inventors have collected from a single subject, B cells, which are contained in blood in an amount larger than the aforementioned amount, according to apheresis. The antibody library used for the purpose of the present invention is preferably constituted with 10⁹ or more B cells. For example, in order to collect approximately 10⁹ B cells from a single human body, B cells may be collected from approximately 3 L of blood according to apheresis.

Antibody-producing cells used to produce an antibody library may further comprise antibody-producing cells derived from another subject, as long as the cells contain approximately 10⁸ or more B cells, and preferably 10⁹ or more B cells, which are derived from the single subject. Specifically, monocytes corresponding to approximately 3 L of blood have been collected, for example, by apheresis, and thereafter, B cells can be isolated and recovered from the monocytes, for example, by Ficoll-Paque density gradient.

Examples of the antibody library include a phage display library, a library obtained by immortalization of B cells with EB virus, and a hybridoma library obtained by fusing B cells with myeloma cells, but the antibody library is not limited thereto. Preferably, a phage display library can be used.

Examples of the method for producing a phage display human antibody library in the present description include the following methods, but the production method is not limited thereto.

The type of the phage used herein is not particularly limited. In general, a filamentous phage (Ff bacteriophage) is preferably used. An example of a method of presenting a foreign protein on the surface of a phage is a method which comprises forming a fusion protein of a foreign protein and any coat protein of g3p (cp3) and g6p (cp6) to g9p (cp9), and allowing the foreign protein to express and/or present in the form of the fusion protein on the coat protein. A method of fusing a foreign protein with the N-terminal side of cp3 or cp8 has been often applied. Examples of a phage display vector include: 1) a vector, by which a foreign gene is introduced into a phage in a form in which the foreign gene is fused with a coat protein gene in the phage genome, and the coat protein is then presented on the surface of the phage in the form of a totally fusion protein with the foreign protein; 2) a vector, by which a gene encoding a fusion protein is inserted into a phage, separately from a wild-type coat protein gene, and allowing the fusion protein and the wdd-type coat protein to simultaneously express on the surface of the phage; and 3) a vector, by which *Escherichia coli* comprising a phagemid vector having a gene encoding a fusion protein is infected with a helper phage having a wild-type coat protein gene, and phage particles simultaneously expressing the fusion protein and the wild-type coat protein are generated. In the case of 1) above, if a large foreign protein is fused with a coat protein, infectivity would be lost. In such a case, the vector 2) or 3) is used to produce an antibody library.

Examples of the specific vector include those described in Holt et al. (Curr. Opin. Biotechno., 11: 445-449, 2000). For example, pCESl (see J.Biol. Chem., 274:18218-18230,1999) is a Fab expression type phagemid vector, in which DNA encoding a *k*L chain constant region downstream of a cp3 signal peptide, DNA encoding C_{H3} downstream of a cp3 signal peptide, a His-tag, a C-myc tag, and a cp3 coding sequence chain via an amber stop codon (TAG) are disposed under the control of one lactose promoter. When this vector is introduced into *Escherichia coli* having an amber mutation, Fab is presented on a cp3 coat protein. However, when this vector is allowed to express in an HB2151 strain or the like, which does not have such an amber mutation, a soluble Fab antibody is generated. Moreover, as a scFv expression type phagemid vector, pHEN1 (J. Mol. Biol., 222:581-597,1991) or the like is used.

On the other hand, examples of a helper phage include M13-K07 and VCSM13. Furthermore, another phage display vector, which has been prepared by ligating a sequence comprising a codon encoding cysteine to each of the 3'-end of an antibody gene and the 5'-end of a coat protein gene, and allowing the two genes to express simultaneously and separately (not as a fusion protein), so that it has been designed to present an antibody on the coat protein on the phage surface via an S-S bond caused by the introduced cysteine residues, is also used (CysDisplay1M technique of Morphosys).

Examples of the antibody library prepared in the present description include a naive/non-immunized library, a synthetic library, and an immunized library.

The naive/non-immunized library is a library prepared by obtaining V_{H} and V_{L} genes possessed by a normal animal by RT-PCR, and then randomly cloning these genes into the aforementioned phage display vector. In general, mRNA derived from lymphocytes from the peripheral blood, bone marrow, tonsil or the like of a normal animal (preferably, peripheral blood lymphocytes), or the like is used as a template. In order to eliminate the bias of the V gene, such as disease history, only the amplified mRNA derived from IgM, in which class switch caused by antigen sensitization does not occur, is particularly referred to as a "naive library." Representative examples of such a naive library include the library of CAT (see J. Mol. Biol., 222: 581-597, 1991; Nat. Biotechnol.,14:309-314,1996), the library ofMRC (see Annu. Rev. Immunol., 12:433-455, 1994), and the library of Dyax (see J. Biol. Chom. (changed to "Chem."), 1999 (as mentioned above); Proc. Natl. Acad. Sci. USA,14: 7969-7974, 2000).

The synthetic library is prepared by selecting a specific functional antibody gene in human B cells, replacing a V gene fragment, for example, an antigen-binding region thereof close to CDR, with DNA encoding a random amino acid sequence having an appropriate length, and converting it to a library. Since such a library can be constituted by a combination of V_{H} and V_{L} genes generating originally functional scFv or Fab, this library is considered to be excellent in terms of antibody expression efficiency and stability. Representative examples of the synthetic library include the HuCAL library of Morphosys (see J. Mol. Biol., 296: 57-86,2000), the library of Biolnvent (see Nat Biotechnol,18: 852, 2000), and the library of Crucell (see Proc. Natl. Acad. sci. USA, 92: 3938,1995; J. Immumol. Methods, 272: 219-233, 2003). In the case of using such a synthetic library, it is desirable to use a VH1-69 or VH1-e gene fragment as a V gene fragment of the heavy chain variable region.

The immunized library is obtained by preparing mRNA from lymphocytes collected from a human whose blood antibody titer to a target antigen has been increased, such as a human who has received vaccination, or human lymphocytes, which have been artificially immunized with a target antigen by an *in vitro* immunization method, or the like, as in the case of the aforementioned naive/non-immunized library, then amplifying V_{H} and V_{L} genes by an RT-PCR method, and then converting it to a library. Since an antibody gene of interest is originally comprised in the library, an antibody of interest can be obtained even from a library with a relatively small size. However, antibodies specific to the subtypes of the virus immunized by vaccination are amplified. Thus, in the case of a human, if influenza viruses of hemagglutinin subtypes H1 to H3, regarding which a large number of antibodies reacting therewith are predicted to be present in the human body, are vaccinated to the human, it is likely that an antibody having a narrow range of neutralizing activity, which can neutralizes only specific isolated strains in the subtypes, would be amplified, and that a neutralizing antibody of interest would disappear. Accordingly, in the case of vaccination, it is preferable to use a vaccine for influenza virus of a subtype, regarding which a wide range of infection has not been reported so far (e.g., H5, H9, etc., in the case of a human).

As a diversity of libraries increases, it is better. However, in reality, taking into consideration the number of phages handled by the following panning operation (10¹¹ to 10¹³ phages) and the number of phages necessary for isolation and amplification of clones by an ordinary panning operation (100 to 1,000 phages/clone), approximately 10⁸ to 10¹¹ clones are appropriate. Preferably, 10⁹ clones and 10⁶ clones are suitable for V_{H} and V_{L} genes, respectively, and the number of Fab or scFV clones is 10¹⁰ to 10¹¹_{.}

As an example of a method of preparing an antibody library by immortalization with EB virus, there is a method described in PLos Medicine 4 (5): e178 0928-9936 (2007), but the method is not limited thereto. A majority of people have been infected with EB virus as a subclinical infection of infectious mononucleosis, and thus, have been immunized with the EB virus. However, since viral particles are generated in the case of using ordinary EB virus, appropriate purification should be carried out. It is also preferable to use, as a system that does not involve virus contamination, a recombinant EB virus, which retains an ability to immortalize B lymphocytes but does not have an ability to replicate viral particles (e.g., a defect in a switch gene for transition from a latent infection condition to a lytic infection condition, etc.).

Since marmoset-derived B95-8 cells secrete EB virus, if a culture supernatant thereof were used, B lymphocytes could be easily transformed. These cells are cultured, for example, in a medium containing serum and penicillin/streptomycin (P/S) (e.g., RPMI 1640) or in a serum-free medium containing a cell growth factor, and a culture supernatant is then separated from the cultured cells by filtration, centrifugation, etc. Thereafter, antibody-producing B lymphocytes are suspended at a suitable concentration (e.g., approximately 10⁷ cells/mL) in the culture supernatant, and the obtained mixture is then incubated at a temperature of generally 20°C to 40°C, and preferably 30°C to 37°C, in general, for approximately 0.5 to 2 hours, so as to obtain antibody-producing B cell line. In a case where human antibody-producing cells are provided as mixed lymphocytes, since a majority of people have T lymphocytes exhibiting cytotoxicity to EB virus-infected cells, in order to increase transformation frequency, it is preferable to previously remove the T lymphocytes, for example, by forming an E rosette from the T lymphocytes and sheep erythrocytes, etc. In addition, soluble antigen-binding sheep erythrocytes are mixed with antibody-producing B lymphocytes, and a rosette is then separated by applying density gradient such as Percoll, so that lymphocytes specific to the target antigen can be selected. Moreover, by adding an excessive amount of antigen, antigen-specific B lymphocytes are capped, and they do not present IgG on the surface thereof. Thus, when they are mixed with anti-IgG antibody-binding sheep erythrocytes, only antigen-non-specific B lymphocytes form a rosette. Accordingly, by collecting a rosette-non-formation layer from the mixture by applying density gradient such as Percoll, antigen-specific B lymphocytes can be selected.

Antibody-secreting cells, which have obtained infinite proliferative capacity as a result of the transformation, can be fused again with mouse or human myeloma cells to stably maintain their antibody-secreting capacity. Examples of the mouse myeloma cells include NS-1, P3U1, SP2/0, and AP-1. Examples of the human myeloma cells include SKO-007, GM 1500-6TG-2, LICR-LON-HMy2, and UC729-6.

As a method of preparing an antibody library by cell fusion, preparation of hybridomas for general production of monoclonal antibodies can be applied. That is to say, B cells collected from a donor are fused with the aforementioned myeloma cells to prepare antibody-producing hybridomas.

The fusion operation can be carried out by a known method, for example, the method of Kohler and Milstein [Nature, Vol. 256, p. 495 (1975)]. Examples of a fusion promoter include polyethylene glycol (PEG) and Sendai virus. Preferably, PEG or the like is used. The molecular weight of PEG is not particularly limited. PEG1000 to PEG6000, which are low toxic and have a relatively low viscosity, are preferable. The concentration of PEG is, for example, approximately 10% to 80%, and preferably approximately 30% to 50%. As a solution for diluting PEG, a serum-free medium (e.g., RPMI 1640), a complete medium containing approximately 5% to 20% serum, various types of buffers such as a phosphate buffered saline (PBS) or a Tris buffer, can be used. As desired, DMSO (e.g., approximately 10% to 20%) can also be added. The pH of the fused solution is, for example, approximately 4 to 10, and preferably, approximately 6 to 8.

The preferred ratio of the number of B cells and the number of myeloma cells is generally approximately 1:1 to 20:1, and cell fusion can be efficiently carried out by incubating these cells at a temperature of generally 20°C to 40°C, preferably 30°C to 37°C, and generally for 1 to 10 minutes.

The screening and breeding of hybridomas are generally carried out in a medium for animal cells (e.g., RPMI 1640) containing 5% to 20% FCS, or a cell growth factor-containing serum-free medium, to which HAT (hypoxanthine, aminopterin, and thymidine) has been added. The concentrations of hypoxanthine, aminopterin, and thymidine are, for example, approximately 0.1 mM, approximately 0.4 µM, and approximately 0.016 mM, respectively. For selection of human or mouse hybridomas, ouabain resistance can be used. Since a human cell line has higher sensitivity to ouabain than a mouse cell line, by adding ouabain at a concentration of approximately 10⁻¹ to 10⁻³ M to the medium, unfused human cells can be eliminated.

For selection of hybridomas, it is preferable to use feeder cells or a certain type of cell culture supernatant. As such feeder cells, allogeneic cell species having a limited survival period, which help the appearance of hybridomas and die by themselves, or cells capable of producing a large amount of growth factors useful for the appearance of hybridomas, the proliferation potency of which has been reduced by radiation exposure or the like, etc. can be used. Examples of the mouse feeder cells include splenic cells, macrophages, blood, and thymocytes. Examples of the human feeder cells include peripheral blood monocytes. Examples of the cell culture supernatant include the primary culture supernatants of various types of the aforementioned cells, and the culture supernatants of various established cells.

A step of selecting an antibody reacting with a target antigen by a phage display method is referred to as "panning." Specifically, a carrier, on which influenza virus of any subtype in Group 1, or a hemagglutinin protein of the virus, or an extracellular domain thereof is immobilized, is allowed to come into contact with a phage library, and non-bound phages are then removed by washing. Thereafter, the bound phages are eluted from the carrier, and are then allowed to infect *Escherichia coli*, so that the phages are allowed to proliferate therein. By repeating a series of the operations about two to four times, phages presenting antigen-specific antibodies are concentrated. In the case of the present invention, influenza virus serving as an antigen may be inactivated by a treatment with formalin.

The cDNA sequence encoding hemagglutinin of each subtype is known, and recombinant hemagglutinin of a desired subtype can be produced by applying a common genetic recombination technique. Moreover, the trimer extracellular domain structure of hemagglutinin can be prepared according to the method described in Nature, Vol. 289, PP. 373-378,1981.

Examples of a carrier, on which an antigen is immobilized, include various types of carriers used in general antigen-antibody reactions or affinity chromatography, including insoluble polysaccharides such as agarose, dextran or cellulose, synthetic resins such as polystyrene, polyacrylamide or silicone a microplate consisting of glass or metal, a tube, a membrane, a column, beads, and a sensor chip of surface plasmon resonance (SPR). For immobilization of an antigen, physical adsorption may be used, or a method involving chemical binding, which is generally used to insolubilize or immobilize a protein, enzyme or the like, may also be used. For example, a biotin-(strept)avidin system or the like is preferably used. For the washing of non-bound phages, a blocking solution such as a BSA solution (once or twice), PBS containing a surfactant such as Tween (three to five times), and the like can be successively used. It has also been reported that the use of a citrate buffer (PHS) or the like is preferable. For elution of specific phages, an acid (e.g., 0.1 M hydrochloric acid, etc.) is generally used. It is also possible to carry out cleavage with specific protease (For example, a gene sequence encoding a trypsin cleavage site can be introduced into a connected portion between an antibody gene and a coat protein gene. In this case, since a wild-type coat protein is presented on the surface of the eluted phage, even if all of the coat protein is expressed as a fusion protein, infection to *Escherichia coli* and/or proliferation become possible.), competitive elution with a soluble antigen, or elution by reduction of S-S bond (For example, in the aforementioned CySDisplay^{™}, after completion of the panning, the antibody is dissociated from the coat protein using a suitable reducing agent, so that an antigen-specific phage can be recovered.) can also be carried out. In the case of elution with an acid, the eluted phage is neutralized with Tris buffer or the like, and it is then allowed to infect *Escherichia coli,* and after completion of the culture, phages are recovered according to an ordinary method.

It is also possible that a hemagglutinin trimer is allowed to express on a cell membrane using an enzyme display, instead of immobilizing an antigen on a carrier.

After phages presenting an antigen-specific antibody have been concentrated by panning, these phages are allowed to infect *Escherichia coli,* and the *Escherichia coli* are then inoculated on a plate, followed by cloning. The phages are recovered again, and the antigen-binding activity is then measured by using an antibody titer measurement method (e.g., ELISA, RIA, NA, etc.), FACS (fluorescence activated cell sorting) or SPR (Surface Plasmon Resonance).

With regard to a step of allowing the above-obtained phage antibody clone to infect *Escherichia coli,* and then recovering an antibody from a culture supernatant, for-example, in the case of using a phage display vector in which an amber stop codon is introduced into a connected portion between an antibody gene and a coat protein gene, if the phage is allowed to infect *Escherichia coli* having no amber mutations (e.g., the strain HB2151), soluble antibody molecules are generated, and they are then secreted into a periplasm or a medium. Accordingly, the cell wall is solubilized with lysozyme or the like, and an extracellular fraction is recovered, and it can be then purified using the same purification technique as described above. If a His-tag or a c-myc tag has been previously introduced, the fraction can be easily purified using IMAC or an anti-c-myc antibody column. Moreover, if cleavage with specific protease is used for panning, since an antibody molecule is separated from the surface of a phage by the action of the protease, the same purification operation as described above is carried out, so as to purify an antibody of interest. In the case of the present invention, the neutralizing activity of an IgG-type complete antibody is approximately 100 to 1,000 times higher than that of a Fab-type antibody. Thus, as described in the Examples later, plasmid DNA is recovered from the obtained phage clone, a sequence corresponding to a domain binding to the Fc of IgG is then added thereto by genetic engineering, and *Escherichia coli* is then transformed with the resulting DNA, followed by culture. Thereafter, an antibody recovered from the culture supernatant is purified with an IgG Sepharose column, and it is then subjected to the test of neutralizing activity.

As a method of selecting an antibody of interest from an antibody-producing cell line obtained by immortalization with EB virus or cell fusion, for example, the aforementioned antigen is fluorescently labeled, and is then allowed to react with immortalized cells or fusion cells, and thereafter, cells binding to the antigen are separated from the resulting cells using a fluorescence-activated cell sorter (FACS), so as to select the antibody of interest. In this case, since hybridomas, which produce antibodies binding to target antigens or immortalized B cells, can be directly selected, it is possible to greatly reduce efforts for cloning.

For the cloning of hybridomas producing monoclonal antibodies binding to target antigens, various methods can be applied.

Since aminopterin inhibits many cellular functions, it is preferable to remove it from the medium as soon as possible. However, human hybridomas are generally maintained in an aminopterin-added medium for approximately 4 to 6 weeks after cell fusion. Hypoxanthine and thymidine are desirably removed from the medium one or more weeks after the removal of aminopterin. After a clone has appeared and the diameter thereof has become approximately 1 mm, it becomes possible to measure the amount of the antibody in the culture supernatant.

The amount of an antibody can be measured, for example, by a method which comprises adding a culture supernatant of hybridomas to a solid phase (e.g., a microplate) on which a target antigen, a derivative thereof, or a partial peptide thereof is adsorbed directly or together with a carrier, then adding an anti-immunoglobulin IgG antibody (which is an antibody reacting with IgG derived from an animal of the same species as the animal, from which the original antibody-producing cells have been derived) or protein A, which has been labeled with a radioactive substance (e.g., ¹²⁵I, ¹³¹I, ³H, or ¹⁴C), enzyme (e.g., β-galactosidase, β-glucosidase_{;} alkaline phosphatase, peroxidase, or malate dehydrogenase), a fluorescent substance (e.g., fluorescamine, fluorescein isothiocyanate), a luminescent substance (e.g., luminol, a luminol derivative, luciferin, or lucigenin) or the like, and then detecting an antibody reacting with the target antigen bound to the solid phase, or a method which comprises adding a culture supernatant of hybridomas to a solid phase, on which an anti-IgG antibody or protein A has been adsorbed, then adding a target antigen, a derivative thereof, or a partial peptide thereof, which has been labeled with the same labeling agent as described above to the solid phase, and then detecting an antibody reacting with the target antigen bound to the solid phase.

As a cloning method, a limiting dilution method is generally used. In addition, cloning using soft agar or cloning involving FACS (as described above) can also be applied. Cloning involving a limiting dilution method can be carried out, for example, by the following procedures, but is not limited thereto.

The amount of an antibody is measured as described above, and positive wells are then selected. Suitable feeder cells are selected, and are then added to a 96-well plate. Cells are sucked out of the antibody-positive cells, and they are then suspended at a density of 30 cells/mL in a complete medium (e.g., RPMI 1640 containing 10% FCS (Fetal Bovine/Calf Serum) and P/S). Thereafter, 0.1 mL (3 cells/well) of the cell suspension is added to the well plate, to which the feeder cells have been added. The remaining cell suspension is diluted to a density of 10 cells/mL, and it is then dispersed on another well (1 cell/well) in the same manner as described above. The further remaining cell suspension is diluted to a density of 3 cells/mL, and it is then dispersed on another well (0.3 cell/well). The cells are cultured for approximately 2 to 3 weeks, until visible clones have appeared, and thereafter, the amount of the antibody is measured. Positive wells are selected, and are then cloned again. Since it is relatively difficult to clone human cells, a plate containing the cell suspension at a density of 10 cells/well is also prepared. In general, monoclonal antibody-producing hybridomas can be obtained by two times of subcloning operations. In order to confirm the stability thereof, it is desired to regularly carry out re-cloning operations for further several months.

Hybridomas can be cultured *in vitro* or *in vivo.*

An example of the *in vitro* culture method is a method of gradually scaling-up the above-obtained monoclonal antibody-producing hybridomas from the well plate, while the cell density is maintained at approximately 10⁵ to 10⁶ cells/mL, and while the FCS concentration is gradually decreased.

An example of the *in vivo* culture method is a method which comprises injecting mineral oil into the abdominal cavity of a mouse (which is tissue compatible with the parent strain of hybridomas) to induce plasmocytoma (MOPC), then 5 to 10 days later, intraperitoneally injecting 10⁶ to 10⁷ hybridomas to the mouse, and then 2 to 5 weeks later, collecting ascites from the mouse under anesthesia.

As with general separation and purification of a polyclonal antibody, separation and purification of a monoclonal antibody are carried out by an immunoglobulin separation purification method [e.g., a salting-out method, an alcohol precipitation method, an isoelectric point precipitation method, electrophoresis, an adsorption-desorption method using an ion exchanger (e.g., DEAE and QEAE), an ultracentrifugation method, a gel filtration method, a specific purification method which comprises collecting only the antibody using an antigen-bound solid phase or an active adsorbent such as protein A or protein G, and then dissociating the bond to obtain the antibody, etc.].

As stated above, hybridomas are cultured *in vivo* or *ex vivo* of a warm-blooded animal, and an antibody is then collected from the body fluid or culture thereof, so that a monoclonal antibody binding to influenza virus of a specific hemagglutinin subtype can be screened.

An example of the method of testing the neutralizing activity in the present invention is a focus formation inhibition test (J. Clin. Miorobiol. Vol. 28, PP 1308-1313 (1990)). That is to say, influenza virus is allowed to come into contact with host cells in the presence or absence of a test antibody, and based on whether or not focus formation caused by viral infection to the host cells is significantly inhibited by the test antibody, the presence or absence of a neutralizing activity and the degree thereof are determined.

The subtype of influenza virus, regarding which the neutralizing activity of a test antibody is to be examined, is not particularly limited. Such influenza virus subtypes preferably include at least H1 influenza virus from Group 1.

Using a clone that produces an antibody molecule that has been confirmed to neutralize at least one influenza virus selected from Group 1 as a result of the neutralizing activity test, the antibody molecule can be produced in a large amount. When the used antibody library is a phage display library, a phage clone presenting Fab or scFv of the neutralizing antibody of interest is allowed to infect *Escherichia coli,* and the *Escherichia coli* is then cultured, so that a Fab-type antibody or a scFv-type antibody may be prepared. However, for the purpose of significantly increasing the neutralizing activity, it is more preferable to convert these antibodies to IgG-type antibodies. For example, Fab can be converted to IgG by cutting fragments encoding VHCH1 and VLCL from phage DNA, inserting them into a plasmid comprising a fragment encoding an Fc region so as to construct a plasmid comprising DNA encoding a heavy chain and a light chain thereof, then transfecting animal cells such as CHO cells with this plasmid, followed by performing a culture, and then allowing an IgG-type antibody to secrete into the culture supernatant. The obtained antibody can be purified and/or recovered by a known method.

Moreover, when the used antibody library consists of EB virus-immortalized B cells or hybridomas obtained by cell fusion, an antibody molecule is produced *in vitro* or *in vivo*, as described above, and it is then purified by an ordinary method, so as to recover the antibody.

The affinity of the obtained neutralizing antibody for an antigen can be enhanced *in vitro* by imitating the steps adopted by an immune system (somatic mutation and selection). Examples of a method of introducing a mutation into an antibody gene include a chain shuffling method, random mutagenesis involving *Escherichia coli* that is easily mutated as a result of deficiency in a repair system thereof or error-prone PCR, and CDR walking. A neutralizing antibody with an improved affinity for an antigen can be selected by screening for a high-affinity neutralizing antibody through a mutation library prepared by mutation introduction. Examples of the method that can be used herein include: 1) a method of setting the concentration of an antigen used for selection at low and recovering a high-affinity phage; 2) a method of setting strict washing conditions and recovering an antibody phage that is hardly removed from an antigen; and 3) a method of using an antagonistic reaction.

A majority of the neutralizing antibodies of the present invention, which have been obtained as described above, utilize a VH1-69 or VH1-e gene as a heavy chain V region. This is a characteristic that is shared by various antibodies neutralizing influenza viruses of a plurality of subtypes in Group 1, which has been reported so far.

When the characteristics of the previously reported neutralizing antibodies against human influenza are verified at a monoclonal antibody level, the target epitope is the head of influenza hemagglutinin (HA) of the influenza, and in general, the antibodies have a neutralizing activity only on specific strains and some subtype strains having high homology with the specific strains. This is because the antigenicity and immunogenicity of influenza to human are extremely high at the head of HA, and mutation sites have been concentrated to the HA during the evolutional process of influenza.

Accordingly, in order to obtain a versatile neutralizing antibody having a wide neutralizing ability capable of coping with continuous mutations in a single subtype, it is effective to produce an antibody reacting with a portion in which antigenicity and immunogenicity are considered to be low.

In the present invention, the inventors have produced an antibody reacting with a stem portion, in which a mutation hardly occurs, and have succeeded in making an invention relating to an antibody capable of coping with not only continuous mutations in a single subtype but also discontinuous mutations over several subtypes. Previously, the present inventors had produced an antibody reacting with a sialic acid pocket, which is only a site to be hardly mutated in the HA head of influenza, and thus, the inventors had succeeded in producing a monoclonal antibody having a neutralizing activity on viruses ranging over several subtypes (Patent Literature 3, Ohshima N et al.," 2011 J Virol 85 (21)).

In the case of an antibody, during the differentiation process of B cells, reorganization of an immunoglobulin gene, namely, recombination of V, D and J regions of a heavy chain variable domain or recombination of V and J regions of a light chain variable domain occurs, and thereafter, a somatic cell mutation is induced to the nucleotide sequence of a variable region. As a result, an antibody having a variable domain having a higher affinity for an antigen can be produced. Accordingly, the neutralizing antibody of the present invention, which is an antibody clone derived from B cells, may also include a neutralizing antibody having an amino acid sequence comprising a somatic cell mutation of the original immunoglobulin gene. Moreover, upon formation of an antigen-antibody complex of a neutralizing antibody and an influenza virus antigen, the contact points with hemagglutinin molecules are all heavy chain variable domains. Thus, a site substantially contributing to the affinity of the neutralizing antibody for influenza virus has been anticipated to be a heavy chain variable domain. Furthermore, since the complementarity-determining region 3 (CDR 3) does not contribute so much to the binding of the neutralizing antibody of the present invention with an antigen, the complementarity-determining regions 1 and 2, which are present in the heavy chain V region, are more important. In the present description, the term "heavy chain variable domain V region (light chain variable domain V region)" is used to mean a V region constituting the variable domain of a heavy chain (light chain) after completion of the reorganization, and it may be, for example, a region including the framework regions 1,2 and 3 and the complementarity-determining regions 1 and 2. Further, the heavy chain (light chain) variable domain indicates a domain in the Fab region, which is not a constant domain, and it may be, for example, a region including the framework regions 1, 2 and 3 and the complementarity-determining regions 1,2 and 3.

Specific examples of the present invention include antibodies, the heavy chain variable domain and the light chain variable domain of which consist of amino acid sequences shown in the amino acid sequence numbers described in any one of the following (1) to (29):
(1) VH CDR1 of SEQ ID NO: 1, VH CDR2 of SEQ ID NO: 2, VH CDR3 of SEQ ID NO: 3, VL CDR1 of SEQ ID NO: 4, VL CDR2 of SEQ ID NO: 5, and VL CDR3 of SEQ ID NO: 6,
(2) VH CDR1 of SEQ ID NO: 7, VH CDR2 of SEQ ID NO: 8, VH CDR3 of SEQ ID NO: 9, VL CDR1 of SEQ ID NO: 10, VL CDR2 of SEQ ID NO: 11, and VL CDR3 of SEQ ID NO: 12,
(3) VH CDR1 of SEQ ID NO: 13, VH CDR2 of SEQ ID NO: 14, VH CDR3 of SEQ ID NO: 15, VL CDR1 of SEQ ID NO: 16, VL CDR2 of SEQ ID NO: 17, and VL CDR3 of SEQ ID NO: 18,
(4) VH CDR1 of SEQ ID NO: 19, VH CDR2 of SEQ ID NO: 20, VH CDR3 of SEQ ID NO: 21, VL CDR1 of SEQ ID NO: 22, VL CDR2 of SEQ ID NO: 23, and VL CDR3 of SEQ ID NO: 24,
(5) VH CDR1 of SEQ ID NO: 25, VH CDR2 of SEQ ID NO: 26, VH CDR3 of SEQ ID NO: 27, VL CDR1 of SEQ ID NO: 28, VL CDR2 of SEQ ID NO: 29, and VL CDR3 of SEQ ID NO: 30,
(6) VH CDR1 of SEQ ID NO: 31, VH CDR2 of SEQ ID NO: 32, VH CDR3 of SEQ ID NO: 33, VL CDR1 of SEQ ID NO: 34, VL CDR2 of SEQ ID NO: 35, and VL CDR3 of SEQ ID NO: 36,
(7) VH CDR1 of SEQ ID NO: 37, VH CDR2 of SEQ ID NO: 38, VH CDR3 of SEQ ID NO: 39, VL CDR1 of SEQ ID NO: 40, VL CDR2 of SEQ ID NO: 41, and VL CDR3 of SEQ ID NO: 42,
(8) VH CDR1 of SEQ ID NO: 43, VH CDR2 of SEQ ID NO: 44, VH CDR3 of SEQ ID NO: 45, VL CDR1 of SEQ ID NO: 46, VL CDR2 of SEQ ID NO: 47, and VL CDR3 of SEQ ID NO: 48,
(9) VH CDR1 of SEQ ID NO: 49, VH CDR2 of SEQ ID NO: 50, VH CDR3 of SEQ ID NO: 51, VL CDR1 of SEQ ID NO: 52, VL CDR2 of SEQ ID NO: 53, and VL CDR3 of SEQ ID NO: 54,
(10) VH CDR1 of SEQ ID NO: 55, VH CDR2 of SEQ ID NO: 56, VH CDR3 of SEQ ID NO: 57, VL CDR1 of SEQ ID NO: 58, VL CDR2 of SEQ ID NO: 59, and VL CDR3 of SEQ ID NO: 60,
(11) VH CDR1 of SEQ ID NO: 61, VH CDR2 of SEQ ID NO: 62, VH CDR3 of SEQ ID NO: 63, VL CDR1 of SEQ ID NO: 64, VL CDR2 of SEQ ID NO: 65, and VL CDR3 of SEQ ID NO: 66,
(12) VH CDR1 of SEQ ID NO: 67, VH CDR2 of SEQ ID NO: 68, VH CDR3 of SEQ ID NO: 69, VL CDR1 of SEQ ID NO: 70, VL CDR2 of SEQ ID NO: 71, and VL CDR3 of SEQ ID NO: 72,
(13) VH CDR1 of SEQ ID NO: 73, VH CDR2 of SEQ ID NO: 74, VH CDR3 of SEQ ID NO: 75, VL CDR1 of SEQ ID NO: 76, VL CDR2 of SEQ ID NO: 77, and VL CDR3 of SEQ ID NO: 78,
(14) VH CDR1 of SEQ ID NO: 79, VH CDR2 of SEQ ID NO: 80, VH CDR3 of SEQ ID NO: 81, VL CDR1 of SEQ ID NO: 82, VL CDR2 of SEQ ID NO: 83, and VL CDR3 of SEQ ID NO: 84,
(15) VH CDR1 of SEQ ID NO: 85, VH CDR2 of SEQ ID NO: 86, VH CDR3 of SEQ ID NO: 87, VL CDR1 of SEQ ID NO: 88, VL CDR2 of SEQ ID NO: 89, and VL CDR3 of SEQ ID NO: 90,
(16) VH CDR1 of SEQ ID NO: 91, VH CDR2 of SEQ ID NO: 92, VH CDR3 of SEQ ID NO: 93, VL CDR1 of SEQ ID NO: 94, VL CDR2 of SEQ ID NO: 95, and VL CDR3 of SEQ ID NO: 96,
(17) VH CDR1 of SEQ ID NO: 97, VH CDR2 of SEQ ID NO: 98, VH CDR3 of SEQ ID NO: 99, VL CDR1 of SEQ ID NO: 100, VL CDR2 of SEQ ID NO: 101, and VL CDR3 of SEQ ID NO: 102,
(18) VH CDR1 of SEQ ID NO: 103, VH CDR2 of SEQ ID NO: 104, VH CDR3 of SEQ ID NO: 105, VL CDR1 of SEQ ID NO: 106, VL CDR2 of SEQ ID NO: 107, and VL CDR3 of SEQ ID NO: 108,
(19) VH CDR1 of SEQ ID NO: 109, VH CDR2 of SEQ ID NO: 110, VH CDR3 of SEQ ID NO: 111, VL CDR1 of SEQ ID NO: 112, VL CDR2 of SEQ ID NO: 113, and VL CDR3 of SEQ ID NO: 114,
(20) VH CDR1 of SEQ ID NO: 115, VH CDR2 of SEQ ID NO: 116, VH CDR3 of SEQ ID NO: 117, VL CDR1 of SEQ ID NO: 118, VL CDR2 of SEQ ID NO: 119, and VL CDR3 of SEQ ID NO: 120,
(21) VH CDR1 of SEQ ID NO: 121, VH CDR2 of SEQ ID NO: 122, VH CDR3 of SEQ ID NO: 123, VL CDR1 of SEQ ID NO: 124, VL CDR2 of SEQ ID NO: 125, and VL CDR3 of SEQ ID NO: 126,
(22) VH CDR1 of SEQ ID NO: 127, VH CDR2 of SEQ ID NO: 128, VH CDR3 of SEQ ID NO: 129, VL CDR1 of SEQ ID NO: 130, VL CDR2 of SEQ ID NO: 131, and VL CDR3 of SEQ ID NO: 132,
(23) VH CDR1 of SEQ ID NO: 133, VH CDR2 of SEQ ID NO: 134, VH CDR3 of SEQ ID NO: 135, VL CDR1 of SEQ ID NO: 136, VL CDR2 of SEQ ID NO: 137, and VL CDR3 of SEQ ID NO: 138,
(24) VH CDR1 of SEQ ID NO: 139, VH CDR2 of SEQ ID NO: 140, VH CDR3 of SEQ ID NO: 141, VL CDR1 of SEQ ID NO: 142, VL CDR2 of SEQ ID NO: 143, and VL CDR3 of SEQ ID NO: 144,
(25) VH CDR1 of SEQ ID NO: 145, VH CDR2 of SEQ ID NO: 146, VH CDR3 of SEQ ID NO: 147, VL CDR1 of SEQ ID NO: 148, VLCDR2 of SEQ ID NO: 149, and VL CDR3 of SEQ ID NO: 150,
(26) VH CDR1 of SEQ ID NO: 151, VH CDR2 of SEQ ID NO: 152, VH CDR3 of SEQ ID NO: 153, VL CDR1 of SEQ ID NO: 154, VL CDR2 of SEQ ID NO: 155, and VL CDR3 of SEQ ID NO: 156,
(27) VH CDR1 of SEQ ID NO: 157, VH CDR2 of SEQ ID NO: 158, VH CDR3 of SEQ ID NO: 159, VL CDR1 of SEQ ID NO: 160, VL CDR2 of SEQ ID NO: 161, and VL CDR3 of SEQ ID NO: 162,
(28) VH CDR1 of SEQ ID NO: 163, VH CDR2 of SEQ ID NO: 164, VH CDR3 of SEQ ID NO: 165, VL CDR1 of SEQ ID NO: 166, VL CDR2 of SEQ ID NO: 167, and VL CDR3 of SEQ ID NO: 168, and
(29) VH CDR1 of SEQ ID NO: 169, VH CDR2 of SEQ ID NO: 170, VH CDR3 of SEQ ID NO: 171, VL CDR1 of SEQ ID NO: 172, VL CDR2 of SEQ ID NO: 173, and VL CDR3 of SEQ ID NO: 174.

Moreover, with regard to the antibodies described in the above (1) to (29), the nucleotide sequences and amino acid sequences of individual heavy chains and light chains are shown in the following Table 1.

**[Table 1]**

| ID | Antibody | Nucleotide sequence of heavy chain | Amino acid sequence of heavy chain | Nucleotide sequence of light chain | Amino acid sequence of light chain |
|---|---|---|---|---|---|
| F080-227 | 1 | SEQ ID NO:175 | SEQ ID NO: 176 | SEQ ID NO:177 | SEQ ID NO:178 |
| F081-007 | 2 | SEQ ID NO: 179 | SEQ ID NO:180 | SEQ ID NO:181 | SEQ ID NO: 182 |
| F081-008 | 3 | SEQ ID NO: 183 | SEQ ID NO:184 | SEQ ID NO: 185 | SEQ ID NO:186 |
| F081-107 | 4 | SEQ ID NO: 187 | SEQ ID NO:188 | SEQ ID NO: 189 | SEQ ID NO: 190 |
| F081-264 | 5 | SEQ ID NO: 191 | SEQ ID NO:192 | SEQ ID NO:193 | SEQ ID NO:194 |
| F081-281 | 6 | SEQ ID NO: 195 | SEQ ID NO:196 | SEQ ID NO:197 | SEQ ID NO:198 |
| F082-117 | 7 | SEQ ID NO: 199 | SEQ ID NO: 200 | SEQ ID NO: 201 | SEQ ID NO:202 |
| F082-220 | 8 | SEQ ID NO: 203 | SEQ ID NO:204 | SEQ ID NO: 205 | SEQ ID NO:206 |
| F082-237 | 9 | SEQ ID NO: 207 | SEQ ID NO: 208 | SEQ ID NO:209 | SEQ ID NO:210 |
| F082-243 | 10 | SEQ ID NO: 211 | SEQ ID NO: 212 | SEQ ID NO: 213 | SEQ ID NO: 214 |
| F082-338 | 11 | SEQ ID NO: 215 | SEQ ID NO: 216 | SEQ ID NO: 217 | SEQ ID NO: 218 |
| F083-103 | 12 | SEQ ID NO: 219 | SEQ ID NO: 220 | SEQ ID NO: 221 | SEQ ID NO: 222 |
| F083-115 | 13 | SEQ ID NO: 223 | SEQ ID NO: 224 | SEQ ID NO: 225 | SEQ ID NO: 226 |
| F083-249 | 14 | SEQ ID NO: 227 | SEQ ID NO: 228 | SEQ ID NO: 229 | SEQ ID NO: 230 |
| F083-301 | 15 | SEQ ID NO: 231 | SEQ ID NO: 232 | SEQ ID NO: 233 | SEQ ID NO: 234 |
| F083-302 | 16 | SEQ ID NO: 235 | SEQ ID NO: 236 | SEQ ID NO: 237 | SEQ ID NO: 238 |
| F083-305 | 17 | SEQ ID NO: 239 | SEQ ID NO: 240 | SEQ ID NO: 241 | SEQ ID NO: 242 |
| F083-307 | 18 | SEQ ID NO: 243 | SEQ ID NO: 244 | SEQ ID NO: 245 | SEQ ID NO: 246 |
| F083-308 | 19 | SEQ ID NO: 247 | SEQ ID NO: 248 | SEQ ID NO: 249 | SEQ ID NO: 250 |
| F083-311 | 20 | SEQ ID NO: 251 | SEQ ID NO: 252 | SEQ ID NO: 253 | SEQ ID NO: 254 |
| F083-318 | 21 | SEQ ID NO: 255 | SEQ ID NO: 256 | SEQ ID NO: 257 | SEQ ID NO: 258 |
| F083-328 | 22 | SEQ ID NO: 259 | SEQ ID NO: 260 | SEQ ID NO: 261 | SEQ ID NO: 262 |
| F083-336 | 23 | SEQ ID NO: 263 | SEQ ID NO: 264 | SEQ ID NO: 265 | SEQ ID NO: 266 |
| F083-349 | 24 | SEQ ID NO: 267 | SEQ ID NO: 268 | SEQ ID NO: 269 | SEQ ID NO: 270 |
| F083-351 | 25 | SEQ ID NO: 271 | SEQ ID NO: 272 | SEQ ID NO: 273 | SEQ ID NO: 274 |
| F083-354 | 26 | SEQ ID NO: 275 | SEQ ID NO: 276 | SEQ ID NO: 277 | SEQ ID NO: 278 |
| F083-366 | 27 | SEQ ID NO: 279 | SEQ ID NO: 280 | SEQ ID NO: 281 | SEQ ID NO: 282 |
| F083-373 | 28 | SEQ ID NO: 283 | SEQ ID NO: 284 | SEQ ID NO: 285 | SEQ ID NO: 286 |
| F083-389 | 29 | SEQ ID NO: 287 | SEQ ID NO: 288 | SEQ ID NO: 289 | SEQ ID NO: 290 |

The amino acid sequences of the heavy chains of the above-mentioned 29 types of antibodies of the present invention are shown in Table 2, and the amino acid sequences of the light chains thereof are shown in Table 3.

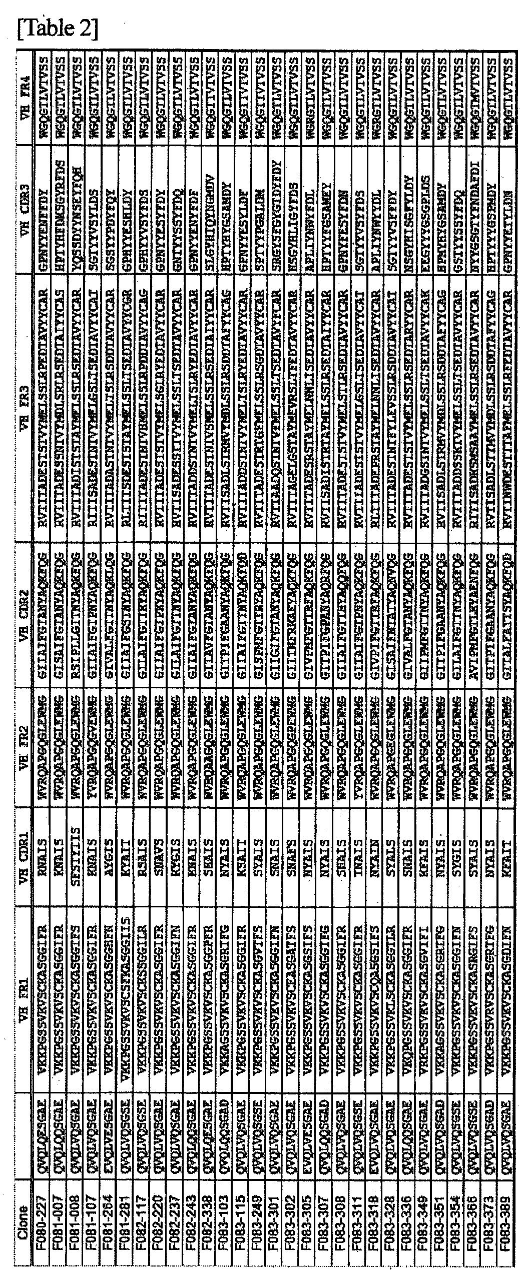

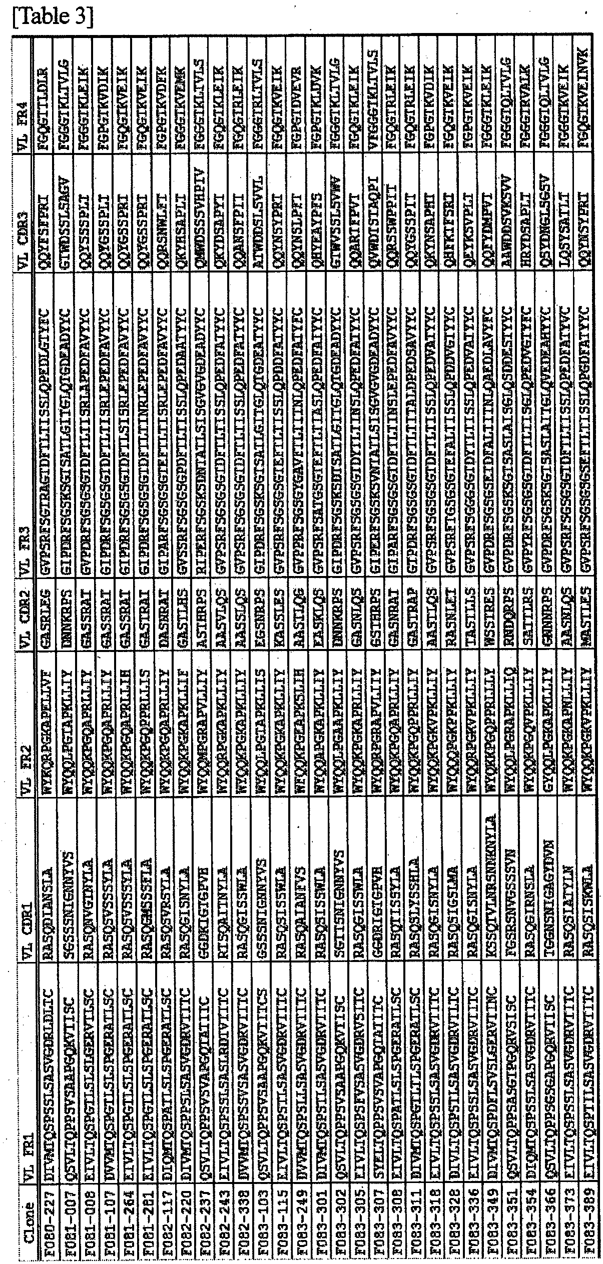

If an epitope recognized by the neutralizing antibody of the present invention were clarified, a peptide comprising the amino acid sequence of the epitope (an amino acid sequence having antigenicity) would be useful as a vaccine for influenza virus, and also, a nucleic acid (gene) comprising a nucleotide sequence encoding a peptide having the antigenicity would be useful as a reagent used for influenza tests or a reagent kit for the tests. An immunologically reactive epitope can be determined by a known method. Examples of such a method of specifying an immunologically reactive epitope include: 1) a method of examining the reactivity of a limited decomposition product prepared by subjecting hemagglutinin to an enzymatic treatment or a chemical treatment with an IgG-type antibody of the neutralizing antibody obtained by the present invention; and 2) a method of examining the reactivity of an overlapping peptide synthesized with reference to amino acid sequence database with an IgG-type antibody of the neutralizing antibody obtained by the present invention.

### (Production of the antibody of the present invention)

An H chain or L chain expression vector is produced, and it is then allowed to express in a host cell. Thereafter, a supernatant containing the secreted antibody is recovered and is then purified, so that an antibody can be obtained.

Specifically, DNA encoding VH is ligated to another DNA molecule encoding a heavy chain constant region (CH1, CH2 and CH3), so as to obtain a full-length heavy chain gene. The sequence of a human heavy chain constant region gene is known in the present technical field (for example, Kabat, E. A. et al., (1991) Sequences of Proteins of Immunological Interest, 5th edition, U. S. Department of Health and Human Services, NIH Publication No. 91-3242), and a DNA fragment including such a region can be obtained by standard PCR amplification. The heavy chain constant region may be the constant region of IgG1, IgG2, IgG3, IgG4, IgA, IgE, IgM or IgD. The most preferred constant region is that of IgG1 or IgG2. The constant region sequence of IgG1 may include any given various alleles or allotypes known to be generated among different individuals, such as Gm (1), Gm (2), Gm (3) or Gm (17). These allotypes correspond to a substitution of amino acids naturally-occurring in the constant region of IgG1.

DNA encoding VL is ligated to another DNA molecule encoding the light chain constant region CL, so as to obtain a full-length L chain gene (and a Fab light chain gene). The sequence of a human light chain constant region gene is known in the present technical field (for example, Kabat, E. A. et al., (1991) Sequences of Proteins of Immunological Interest, 5th edition, U. S. Department of Health and Human Services, NIH Publication No. 91-3242), and a DNA fragment including such a region can be obtained by standard PCR amplification. The light chain constant region may be the constant region of κ or λ. The κ constant region may include any given various alleles known to be generated among different individuals, such as Inv (1), Inv (2) or Inv (3). The λ constant region may be derived from any one of the three λ genes.

The thus obtained DNA encoding an H chain or L chain is inserted into a vector to produce an expression vector, and the produced expression vector is then allowed to express in a host cell. Thereafter, a supernatant containing the secreted antibody is recovered and purified, so that an antibody can be obtained. Examples of the expression vector include a plasmid, retrovirus, adenovirus, adeno-associated virus (AAV), plant viruses such as cauliflower mosaic virus or tobacco mosaic virus, a cosmid, YAC, and EBV-derived episome. An expression vector and an expression regulatory sequence are selected, so that they are suitable for a host cell used for expression. An antibody light chain gene and an antibody heavy chain gene can be inserted into different vectors, or the two genes can also be inserted into a single expression vector. An antibody gene is inserted into an expression vector by a standard method (for example, ligation of a complementary restriction site on an antibody gene fragment to a vector, or blunt-ended ligation applied when no restriction sites are present):

A favorable vector encodes a functionally completed human CH or CL immunoglobulin sequence having a suitable restriction site, which has been produced by an engineering approach such that any given VH or VL sequence can be easily inserted and then expressed as described above. In such a vector, splicing generally takes place between a splice donor site in the inserted J region and a splice acceptor site preceding a human C domain, or such splicing also takes place in a splice region existing in a human CH exon. Polyadenylation and transcription termination take place in a natural chromosomal site downstream of a coding region. A recombinant expression vector can also encode a host cell-derived signal peptide that promotes the secretion of an antibody chain. An antibody chain gene can be cloned into a vector, such that a signal peptide can be ligated in-frame to the amino terminus of an immunoglobulin chain. The signal peptide may be either an immunoglobulin signal peptide or a heterogeneous signal peptide (namely, it may be a non-immunoglobulin protein-derived signal peptide).

An expression vector used for the antibody of the present invention may also have sequences such as a sequence for regulating replication of the vector in a host cell (e.g. a replication origin) or a selective marker gene sequence, in addition to an antibody gene and a regulatory sequence. The selective marker gene promotes selection of a host cell into which a vector has been introduced. For instance, the selective marker generally imparts resistance to drugs such as G418, hygromycin or methotrexate to a host cell into which the vector has been introduced. Preferred selective marker genes include a dihydrofolate reductase (DHFR) gene (used in methotrexate selection/amplification as a dhfr-host cell), a neomycin phosphotransferase gene (used in G418 selection), and a glutamate synthase gene.

A host cell is transformed with an antibody gene expression vector produced by the above-described method. Any type of cell may be used as a host cell, as long as it can produce the antibody of the present invention. Examples of such a host cell include bacteria, yeast, animal cells, insect cells, and plant cells. Among these cells, animal cells are preferable. Examples of the animal cells include Chinese hamster ovary cells CHO/dhfr(-) and CHO/DG44, monkey-derived cells COS (A. Wright & S. L. Morrison, J. Immunol. 160,3393-3402 (1998)), and SP2/O cells (mouse myeloma) (K. Motmans et al., Eur. J. Cancer Prev. 5,512-51199 (1996), R. P. Junghans et al., Cancer Res. 50,1495-1502 (1990)). For transformation, a lipofectin method (R. W. Malone et al., Proc. Natl. Acad. Sci. USA 86, 6007 (1989), P. L. Felgner et al., Proc. Natl. Acad. Sci. USA 84, 7413 (1987)), an electroporation method, a calcium phosphate method (F. L. Graham & A. J. van der Eb, Virology 52,456-467 (1973)), a DEAE-Dextran method, and the like are preferably applied.

A transformant is cultured, and an antibody is then separated from the cells of the transformant or a culture medium thereof For separation/purification of the antibody, methods such as centrifugation, ammonium sulfate fractionation, salting-out, ultrafiltration, affinity chromatography, ion exchange chromatography and gel filtration chromatography can be used by appropriately combining them.

### (Antibody fragments)

An antibody fragment can be produced based on the antibody of the present invention, or based on the sequence information of a gene encoding the antibody of the present invention. Examples of the antibody fragment include Fab, Fab', F(ab')₂, scFv, and dsFv antibodies.

Fab is obtained by digesting IgG by papain in the presence of cysteine. It is an antibody fragment with a molecular weight of approximately 50,000, which is constituted with L chain and H chain variable regions, and an H chain fragment consisting of a CH1 domain and a portion of a hinge region. In the present invention, the above-described antibody can be obtained by papain digestion. In addition, Fab can also be prepared by incorporating DNA encoding a portion of the H chain and the L chain of the above-described antibody into a suitable vector, then performing transformation with the resulting vector, and then obtaining it from the transformant.

Fab' is an antibody fragment with a molecular weight of approximately 50,000, which is obtained by cleaving a disulfide bond between the H chains of the below-mentioned F(ab')₂. In the present invention, Fab' can be obtained by digesting the above-described antibody by pepsin, and then cleaving a disulfide bond with a reducing agent. In addition, as with Fab, Fab' can also be prepared by genetic engineering using DNA encoding the Fab'.

F(ab')₂ is obtained by digesting IgG by pepsin, and is an antibody fragment with a molecular weight of approximately 100,000, which is obtained by binding, via a disulfide bond, one fragment (Fab') constituted with L chain and H chain variable regions and an H chain fragment consisting of a CH1 domain and a portion of a hinge region, to the other fragment (Fab'). In the present invention, F(ab')₂ can be obtained by digesting the above-described antibody by pepsin. In addition, as with Fab, F(ab')₂ can also be prepared by genetic engineering using DNA encoding the F(ab')₂.

scFv is an antibody fragment obtained by ligating the C-terminus of one chain of Fv consisting of an H chain variable region and an L chain variable region to the N-terminus of the other chain thereof, using a suitable peptide linker, so as to form a single chain. (GGGGS)₃ having high flexibility can be used, for example, as such a peptide linker. For instance, DNA encoding the H chain variable region and L chain variable region of the above-described antibody and DNA encoding a peptide linker are used to construct DNA encoding a scFv antibody, and the thus constructed DNA is then incorporated into a suitable vector. Thereafter, scFv can be prepared from a transformant obtained by transformation with the aforementioned vector.

dsFv is a Fv fragment obtained by introducing a Cys residue into a suitable site in each of an H chain variable region and an L chain variable region, and then stabilizing the H chain variable region and the L chain variable region by a disulfide bond. The site in each chain, into which the Cys residue is to be introduced, can be determined based on a conformation predicted by molecular modeling. In the present invention, for example, a conformation is predicted from the amino acid sequences of the H chain variable region and L chain variable region of the above-described antibody, and DNA encoding each of the H chain variable region and the L chain variable region, into which a mutation has been introduced based on such prediction, is then constructed. The thus constructed DNA is incorporated into a suitable vector. Thereafter, dsFv can be then prepared from a transformant obtained by transformation with the aforementioned vector.

Further, it is also possible to ligate scFv antibody or dsFv antibody using a suitable linker, or to fuse an antibody fragment with streptavidin, so as to multimerize the antibody fragment.

### (Pharmaceutical composition)

Since the neutralizing antibody of the present invention is capable of neutralizing influenza viruses of all hemagglutinin subtypes over the framework of groups (which is not an antibody capable of neutralizing influenza viruses beyond the framework of groups), it can be an effective preventive and/or therapeutic means, not only for seasonal influenza caused by antigenic drift, but also for pandemic influenza caused by antigenic shift. With regard to the range of the present neutralizing antibody (which may also be effective for antigenic shift, if the antigenic shift is a shift in Group 1), passive immunization can be carried out on influenza viruses of subtypes in Group 1 by administering the neutralizing antibody, and therapeutic effects on patients who have developed influenza as a result of infection with any given influenza virus, and preventive effects on subjects who have a risk of the onset of influenza or infection with influenza virus can be anticipated. Moreover, since the neutralizing antibody of the present invention has originally existed in a human body, it is considered that the present neutralizing antibody does not have a risk of side effects.

The neutralizing antibody of the present invention can be used as a passive immunotherapeutic agent against influenza, by itself, or after it has been mixed with a pharmaceutically acceptable carrier to prepare a pharmaceutical composition.

Herein, as pharmaceutically acceptable carriers, various types of organic or inorganic carrier substances, which have been commonly used as materials for formulations, are used. These pharmaceutically acceptable carriers are mixed as excipients, solvents (dispersers), solubilizers, suspending agents, stabilizers, isotonization agents, buffers, pH adjusters, soothing agents, and the like, with the neutralizing antibody of the present invention. In addition, as necessary, formulation additives, such as preservatives and antioxidants, can also be used.

Preferred examples of the excipient include lactose, saccharose, D-mannitol, D-sorbitol, starch, pregelatinized starch, dextrin, crystalline cellulose, low-substituted hydroxypropyl cellulose, sodium carboxymethyl cellulose, gum Arabic, Pullulan, light anhydrous silicic acid, synthetic aluminum silicate, and magnesium aluminometasilicate.

Preferred examples of the solvent include water for injection, a normal saline, a Ringer solution, alcohol, propylene glycol, polyethylene glycol, sesame oil, corn oil, olive oil, and cottonseed oil.

Preferred examples of the solubilizer include polyethylene glycol, propylene glycol, D-mannitol, trehalose, benzyl benzoate, ethanol, tris-aminomethane, cholesterol, triethanolamine, sodium carbonate, sodium citrate, sodium salicylate, and sodium acetate.

Preferred examples of the suspending agent include: surfactants such as stearyl triethanolamine, sodium lauryl sulfate, laurylaminopropionic acid, lecithin, benzalkonium chloride, benzethonium chloride and glyceryl monostearate; hydrophilic polymers such as polyvinyl alcohol, polyvinyl pyrrolidone, sodium carboxymethyl cellulose, methyl cellulose, hydroxymethyl cellulose, hydroxyethyl cellulose and hydroxypropyl cellulose; and polysorbates and polyoxyethylene hardened castor oil.

Preferred examples of the stabilizer include human serum albumin (HSA), sodium pyrosulfite, Rongalit, and sodium hydrogen metabisulfite.

Preferred examples of the isotonization agent include sodium chloride, glycerin, D-mannitol, D-sorbitol, and glucose.

Preferred examples of the buffer include buffer solutions such as phosphate, acetate, carbonate, and citrate.

Preferred examples of the pH adjuster include acids and bases, such as hydrochloric acid and sodium hydroxide.

A preferred example of the soothing agent is benzyl alcohol.

Preferred examples of the preservative include p-hydroxybenzoate esters, chlorobutanol, benzyl alcohol, phenethyl alcohol, dehydroacetic acid, and sorbic acid.

Preferred examples of the antioxidant include sulfite and ascorbate.

Examples of the dosage form of the above-described pharmaceutical composition include injections (e.g., a subcutaneous injection, an intravenous injection, an intramuscular injection, an intraperitoneal injection, an intra-arterial injection, etc.) and injection-type formulations such as drops.

The pharmaceutical composition can be produced by a method commonly used in the technical field of formulations, for example, a method described in Japanese Pharmacopoeia. Hereafter, specific methods for producing formulations will be described in detail. The content of an antibody in a pharmaceutical composition is different depending on the dosage form, the applied dose, etc. It is, for example, approximately 0.1% to 100% by weight.

For example, an injection is produced by dissolving, suspending or emulsifying an antibody, together with a disperser (e.g., polysorbate 80, polyoxyethylene hardened castor oil 60, polyethylene glycol, carboxymethyl cellulose, sodium alginate, etc.), a preservative (e.g., methylparaben, propylparaben, benzyl alcohol, chlorobutanol, phenol, etc.), an isotonization agent (e.g., sodium chloride, glycerin, D-mannitol, D-sorbitol, glucose, etc.) and the like, in an aqueous solvent (e.g., distilled water, normal saline, Ringer's solution, etc.) or in an oily solvent (e.g., vegetable oil such as olive oil, sesame oil, cottonseed oil or corn oil, propylene glycol, etc.). At this time, additives such as a solubilizer (e.g., sodium salicylate, sodium acetate, etc.), a stabilizer (e.g., human blood albumin, etc.), a soothing agent (e.g., benzyl alcohol, etc.) may be used, as desired. Such an injection is subjected to a sterilization treatment such as filtration sterilization using a membrane filter or the like, as necessary, and thereafter, it is generally filled into a suitable vessel such as an ampule.

Regarding such an injection, the aforementioned liquid agent is processed into powders according to vacuum drying or the like, and the particles can be then used by being dissolved (dispersed) before use. Examples of the vacuum drying method include a freeze-drying method and a method using a speed-back concentrator (SAVANT). When such freeze-drying is carried out, it is preferable that a sample that has been cooled to -10°C or lower be used, and that the sample be freeze-dried in a flask in a laboratory, industrially using a tray, or in a vial. When such a speed-back concentrator is used, freeze-drying is carried out at approximately 0°C to 30°C; and at a degree of vacuum of approximately 20 mmHg or less, and preferably approximately 10 mmHg or less. It is preferable that a buffer such as phosphate be added to a liquid agent to be dried, and that the pH be set at approximately 3 to 10. The powdery formulation obtained by vacuum drying is stable for a long period of time, and it can be used as an injection by being dissolved in water for injection, normal saline, Ringer's solution, etc., or being dispersed in olive oil, sesame oil, cottonseed oil, corn oil, propylene glycol, etc., before use.

Other therapeutic drugs can also be used in combination with the above-described antibody, as necessary. Examples of such a therapeutic drug include Tamiflu, Relenza, and Amantadine.

Otherwise, other therapeutic drugs can also be bound to the above-described antibody, as necessary. The antibody transports a drug to a site at which influenza virus is present, or a periphery thereof, and it also inhibits invasion of the virus into cells. On the other hand, the drug kills the virus, or it treats, alleviates or ameliorates the symptoms of influenza. The drug that is used herein includes all drugs, which are used as or are to be used as therapeutic drugs for influenza. Such a drug is, for example, a synthetic or natural, low-molecular-weight or high-molecular-weight, protein or non-protein, or nucleic acid or nucleotide substance. The binding of the antibody to the drug is preferably carried out via a linker. The linker comprises, for example, a substituted or non-substituted aliphatic alkylene chain, and at both ends thereof, it comprises a group capable of binding to a functional group of the antibody or drug, such as an N-hydroxysuccinimide group, an ester group, a thiol group, an imidocarbonate group, or an aldehyde group (Kotai Kogaku Nyumon (Antibody Engineering Manual), Chijinshokan Co., Ltd., 1994).

As necessary, in order to facilitate the transport of a pharmaceutical drug into a cell, the drug can also be encapsulated into a liposome. Examples of a preferred liposome include a positively charged liposome, positively charged cholesterol, and a transmembrane peptide binding liposome (Mamoru NAKANISHI et al., Tanpakushitsu Kakusan Koso (Protein, Nucleic acid and Enzyme 44: 1590-1596 (1999), Shiro NIKI, Kagaku to Seibutsu (Chemistry and Organisms) 43: 649-653 (2005), Clinical Cancer research 59: 4325-4333 (1999), etc.).

The neutralizing antibody of the present invention is administered via parenteral administration, such as intravenous administration, intraperitoneal administration, intramuscular administration, subcutaneous administration, or transdermal administration.

The amount of the active ingredient of the antibody is 100 to 2,500 µg/ml per dose, or it is 1.0 to 10 mg per kg of the body weight of an adult human patient. However, the amount of the active ingredient is not limited to thereto.

The number of administrations is, for example, once 1 to 2 weeks for one to several administrations, or once 2 to 3 weeks for approximately 2 months.

The neutralizing antibody of the present invention is not only used for prevention and/or treatment of influenza for humans, but it is also administered to avian species such as a chicken, or to non-human mammals such as a swine or a horse, so that it can also be used for prevention and/or treatment of influenza for such animals. Thereby, the risk of infection of humans with influenza can be reduced before it happens. Even in a case where the present neutralizing antibody is applied to these animals, the same formulation method as described above can be applied.

Hereinafter, the present invention will be described more in detail in the following examples. However, these examples are not intended to limit the scope of the present invention.

### Examples

### [Materials and Methods]

### Materials

### < Antigen virus for screening >

The following influenza viruses were used in the present study: A/H1N1pdm: A/California/7/2009pdm (Ca109), and A/Brisbane/59/2007 (Bri07).

The terms in the parentheses indicate abbreviations of virus strains.

### Characterization (for ELISA, HI activity, and neutralizing activity)

The following influenza viruses were used in the present study: A/H1N1pdm: A/California/7/2009pdm (Ca109), A/Suita/1/2009pdm (Sui09); A/H1N1: A/New Caledonia/20/1999 (NC99), A/Solomon Islands/3/2006 (SI06), A/Brisbane/59/2007 (Bri07). A/H3N2: A/Panama/2007/1999. A/H5N1: A/Indonesia/5/2005/PR8-IBCDC-RG2. The terms in the parentheses indicate abbreviations of virus strains.

### < Construction of phage antibody library >

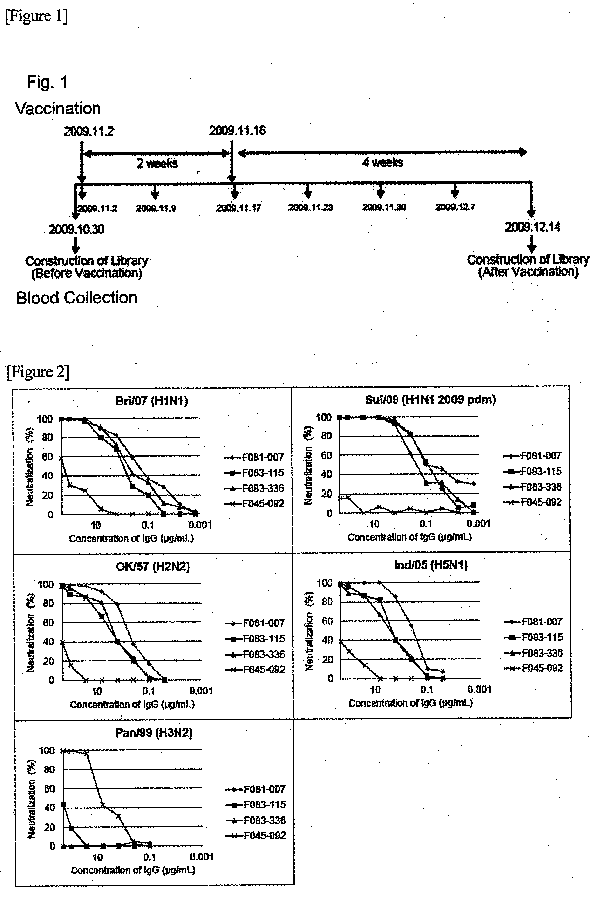

The schedule of vaccination and blood collection is shown in Figure 1.

In 2009, a donor bone in 1947 was inoculated with the pandemic vaccine strain A/California/7/2009 twice (November 2 and 16, 2009). Blood collection from the donor was carried out, twice before the vaccinations (October 30 and November 2), once after the first vaccination (November 9), and five times after the second vaccination (November 17, 23 and 30, and December 7 and 14). From the blood collected on October 30 (before the vaccinations) and on December 14 (after the vaccinations), an enormous phage antibody library was prepared.

Such a phage antibody library was constructed according to the previously reported method (Okada J, et al., 2010. Virology 397: 322-330). The monocytes of the donor born in 1947 before and after the vaccinations were recovered from 3 L of blood according to apheresis. The cells comprised 8.0 x 10⁸ B lymphocytes before the vaccinations and 1.2 x 109 B lymphocytes after the vaccinations. The enormous antibody library was constructed by a phage display method, as previously reported (Marks JD, et al., 1991. J. Mol. Biol. 222: 581-597). With regard to the size of the library, from the blood collected before the vaccinations, the heavy chain consisted of 1.6 x 10⁹ clones, the light chain consisted of 2.0 x 10⁹ clones, and Fab consisted of 1.4 x 10¹⁰ clones. From the blood collected after the vaccinations, the heavy chain consisted of 3.2 x 10⁹ clones, the light chain consisted of 1.3 x 10⁹ clones, and Fab consisted of 2.6 x 10¹⁰ clones.

### Antibody-obtaining method

### < Screening of library >

Clones binding to viral particles were selected by a panning method, as previously reported (Iba Y, et al.,1997. Gene 194: 35-46). The viral particles H1N1 of the strain Cal09 or Bri07, which had been treated with formalin, were used as antigens in a screening operation. After two or three times of panning operations, *Escherichia coli* (DH12S) was infected with the eluted phage, and it was then cultured in an LB plate containing 100 µg/ml ampicillin and 0.2% glucose. Thereafter, *Escherichia coli* colonies containing phagemid were isolated, and they were then cultured at 30°C overnight in a 2 x YT medium containing 100 µg/ml ampicillin, 0.05% glucose and 1 mM isopropyl-β-D-thiogalactopyranoside (IPTG). During the culture of the *Ercherichia coli*, a Fab-cp3-type antibody was secreted into the medium (Morino K, et al., 2001. J. Immunol. Meth. 257: 175-184). ELISA was carried out using the culture supernatant containing the Fab-cp3-type antibody, and the binding activity of the antibody to the virus strains H1N1 and H3N2 used in the screening was measured. Clones only binding to the virus strain H1 were selected and were subjected to a further analysis.

### Antibody evaluation method

### < ELISA >

A 96-well maxisorp immunoplate (Nunc) was coated with viral particles that had been treated with formalin, and the Fab-cp3-type antibody contained in the culture supernatant of *Escherichia coli* was then added to each well. The well was incubated with a rabbit anti-cp3 antibody (MBL), and it was further incubated with peroxidase-conjugated goat anti-rabbit IgG (H + L chain; MBL). A HRP substrate (OPD, Wako Pure Chemical Industries, Ltd.) was added to each well, so that a color was developed. The peroxidase reaction was terminated by addition of sulfuric acid, and the absorbance of the sample at a wavelength of 492 nm was then measured.

### < Sequence analysis >

The nucleotide sequence of the VH fragment of the isolated antibody clone was determined by employing GenomeLab Dye Terminator Cycle Sequencing - Quick Start Kit (Beckman Coulter) and CEQ2000 DNA Analysis System (Beckman Coulter). As a VH sequence primer, T7ETZ (5'-TAATACGACTCACTATAGGG 3') was used.

### < Western blotting >

A vaccine strain, which had been inactivated with formalin, was subjected to SDS-PAGE under non-reduction conditions, and it was then transcribed on Immobilon-P membrane (Millipore). The membrane was incubated with the Fab-cp3-type antibody contained in the culture supernatant of *Escherichia coli.* The membrane was incubated with rabbit anti-cp3 Ab (MBL), and was further incubated with peroxidase-conjugated goat anti-rabbit IgG (H + L chain; MBL). A band was detected using ECL Plus Western blotting Detection Regents (GE Healthcare) and Chemical-luminescence imaging system (Light Capture; ATTO).

### < Measurement of virus-neutralizing activity >

A focus reduction neutralization assay was carried out (Okuno Y, et al., 1990. J. Clin. Microbiol. 28:1308-13131). A Fab-PP-type antibody (200 or 500 µg/ml) was mixed with an equal amount of influenza virus (100 FFU), and the obtained mixture was then added to MDCK cells, which had been allowed to grow on a 96-well plate. After completion of incubation, the cells were washed with serum-free MEM, and were then cultured at 37°C for 15 hours in MEM containing 0.4% BSA. Thereafter, the cultured cells were fixed with ethanol, and were then allowed to react with a PAP (peroxidase and anti-peroxidase) complex. The number of focuses, which were formed with one or more cells, was counted, and a focus reduction rate was shown as a result.

### < Hemagglutination inhibition (HI) assay >

The HI test was carried out by the previously mentioned method Okuno Y, et al., 1990. J. Clin. Microbiol. 28:1308-13131). The purified 160µg/ml Fab-PP-type antibody was subjected to stepwise dilution, and it was then incubated with virus of 4 HA units per well. Thereafter, 0.75% Guinea pig serum was added to each well, and it was then incubated at room temperature for 30 to 60 minutes. The lowest concentration (µg/ml) of the Fab-PP-type antibody that inhibited hemagglutination was shown as a result.

### Antibody production methods (Fab and IgG)

### < Production of purified Fab-PP-type antibody >

A Fab-cp3-type antibody was converted to a Fab-PP-type antibody (wherein P represents a single Fc-binding domain of protein A) according to a previously reported method (Ito W, et al., 1993. J. Biol. Chem. 268: 20668-20675), and the Fab-PP-type antibody was then purified with IgG Sepharose (GE Healthcare).

### < Production of IgG-type antibody >

### (1) Production of plasmid expressing IgG antibody

The gene of a phage antibody (scFv) is disposed in the order of VH-VL, and VH and VL have an scFv structure in which they are connected with each other by the following linker.
Linker (SEQ ID NO: 291):
Gly Gly Gly Gly Ser Gly Gly Gly Gly Ser Gly Gly Gly Gly Ser

On the other hand, the gene of a phage antibody (Fab) is disposed in the order of VH-CH1-His and VL-CL-PIII, and the aforementioned connecting linker is not present.

First, a human germline gene, which was assumed to be used in VH and VL, was searched on IMGT (*).
(*) IMGT: http://wwwimgt.org

Using the search results of IMGT as references, the phage antibody was converted to an IgG antibody. A VH sequence was connected with the constant region of human IgG1. Regarding VL, a gene, a deficient portion of which was connected with the closest germline gene, was produced. An H chain gene and an L chain gene, in which Nhel was added to the 5'-terminal side and EcoRI was added to the 3'-terminal side, were totally synthesized by GenScript. The synthesized heavy chain and light chain genes were each incorporated into different expression vectors. That is to say, the artificially synthesized H chain and L chain genes were each cleaved with NheI and EcoRI, and they were then incorporated into the NheI and EcoRI sites of the expression vector pCAGGS, so as to obtain an antibody H chain expression vector and an antibody L chain expression vector. After construction of these expression vectors, they were allowed to forcibly express inhuman cells, thereby obtaining antibodies (see the content of Japanese Patent No. 4870348).

### [Results]

The activities of representative clones are shown in Table 4.

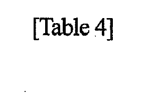

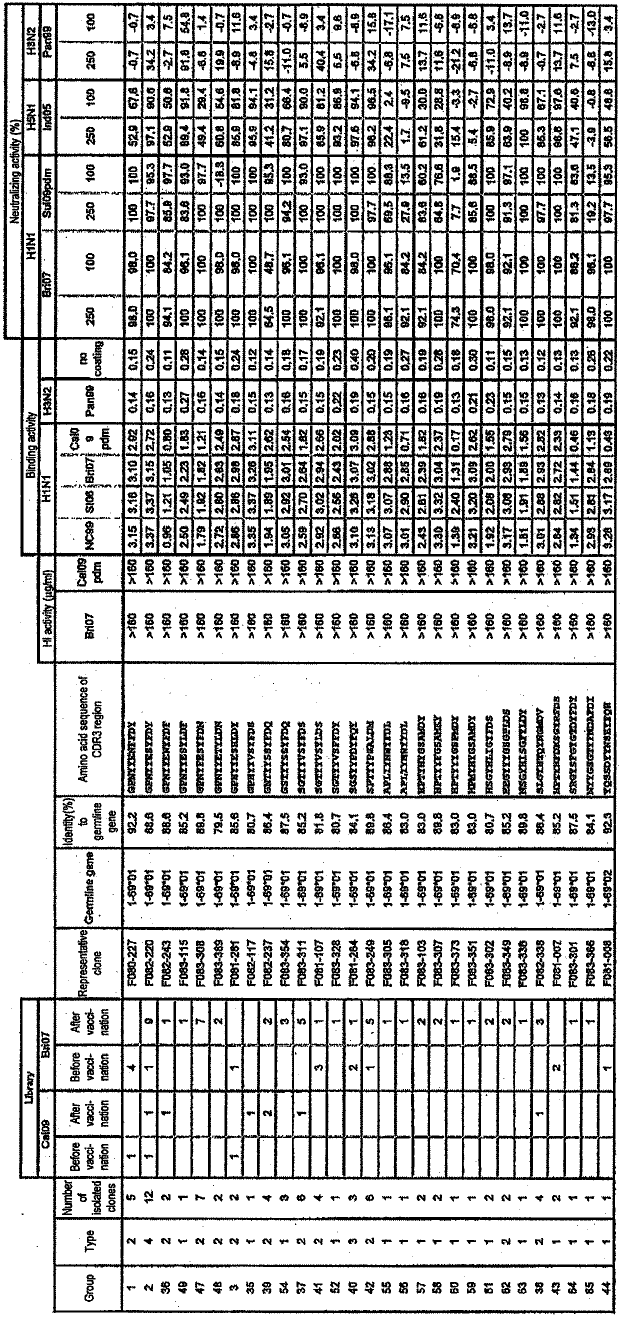

The first three lines in Table 4 indicate the group number of clones isolated by screening, the number of clone types included in each group, and the number of the isolated clones. Germline genes used by the clones were identified by comparing the VH amino acid sequences of all of the germline genes with those of representative clones, and the obtained identity was defined by percentage. The amino acid sequences of the CDR3 regions were shown. The binding activities of the clones to 4 types of H1N1 strains (NC99, SI06, Bri07 and Ca109) and 1 type of H3N2 strain (Pan99) were detected by enzyme-linked immunosorbent assay (ELISA). The binding activity was indicated by the absorbance at 492 nm. The neutralizing activities of the Fab-PP-type antibodies at a concentration of 100 or 250 µg/ml on the strains H1N1, H5N1 and H3N2 were measured by a focus reduction method. The reduction rate was defined by percentage. The HI activities of the Fab-PP antibodies were measured with respect to the H1N1 strains (Bri07 and Ca109). The lowest concentration (µg/ml) of the Fab-PP-type antibody to inhibit hemagglutination was shown.

### < Analysis of neutralization kinetics of antibody obtained in the present invention >

### Measurement of neutralization kinetics of IgG-type antibody to influenza virus strains

A purified, completely-humanized IgG antibody was adjusted to concentrations of 200 µg/mL and 100 µg/mL with MEM containing 0.4% BSA, and the 100 µg/mL solution was further subjected to four-step dilution. To this antibody solution, an equal amount of influenza virus containing approximately 100 focus forming units was added, and the obtained mixture was then subjected to a neutralization reaction at 37°C for 1 hour. MDCK cells, which had been cultured in a 96-well plate, were washed with PBS(-), the neutralization reaction solution was then added by 30 µL/well to the MDCK cells. Thereafter, the obtained mixture was reacted at 37°C for 1 hour. After that, the reaction solution was removed, and the cells were then washed with PBS(-) once. MEM containing 0.4% BSA was added by 50 µL/well to the resulting cells, and the obtained mixture was then cultured in the presence of CO2 at 37°C for 16 hours. Thereafter, the culture solution was removed, and the resulting cells were fixed with ethanol and were then dried. The infected cells were stained by an enzyme antibody technique using a peroxidase - anti-peroxidase complex (PAP method). The number of the infected cells was counted, and the infection inhibition rate was then calculated.

### Influenza virus strains

The following virus strains were used in the measurement of neutralizing activity. The terms in the parentheses indicate abbreviations.
H1N1; A/Bribane/59/2007 (Bri/07)
H1N12009 pandemic; A/Suita/1/2009 (Sui/09)
H2N2; A/Okuda/1957 (OK/57)
H5N1; A/Indonesia/5/2005 (H5N1)/PR-IBCDC-RG2 (clade2.1) (Ind/05)
H3N2; A/Panama/2007/1999 (Pan/99)

### Completely humanized IgG antibodies subjected to measurement of neutralizing

F081-007, F083-115, F083-336, and F045-092

### (Concerning the control antibody F045-092 used in the present example)

Approximately 3 L of component blood was collected from a human born in 1973 according to apheresis.

After production of a phage antibody library, the library was screened against the influenza virus strain H3N2, and as a result, an anti-HA (hemagglutinin) antibody clone was isolated.

This antibody exhibits an extremely strong neutralizing activity against a wide range of H3 virus strains from 1968 to 2011, and it is highly likely that the binding region has, as an epitope, the receptor binding pocket of a hemagglutinin head domain.

Moreover, this antibody also exhibits a neutralizing activity on H1, H2 and H5 virus strains. That is to say, this antibody is a versatile antibody capable of neutralizing a wide range of influenza viruses belonging to, at least, Group 1 and Group 2 (Patent Literature 3).

### Results

All of the three types of antibodies (F081-007, F083-115, and F083-336), which had been subjected to the measurement, exhibited a high neutralizing activity on the studied virus strains (H1N1, H2N2 and H5N1) belonging to Group 1, which was 90 to 900 times, at an IC50 value, higher than that of the F045-092 antibody.

In particular, the IC50 values of these three antibodies were 0.09 - 0.23 µLg/mL with respect to the H1N1 pandemic strain (Sui/09), although the neutralizing activity of the F045-092 antibody on this pandemic strain was not detected.

Moreover, the F081-007 antibody exhibited an extremely high neutralizing activity on all of the strains, and particularly, it exhibited an IC50 value that was approximately 900 times higher than the F045-092 antibody on the H5N1 strain (Ind/05) (0.36 µg/mL).

Furthermore, the neutralizing activity of the F083-115 antibody on the H3N2 strain was detected, although the activity was weak (IC50 value: 225µg/mL):

From these results, it was demonstrated that the antibody of the present invention has overwhelmingly strong neutralization kinetics, in particular, on Group 1, in comparison to the antibody described in Patent Literature 3 as a prior art (Table 5 and Figure 2).

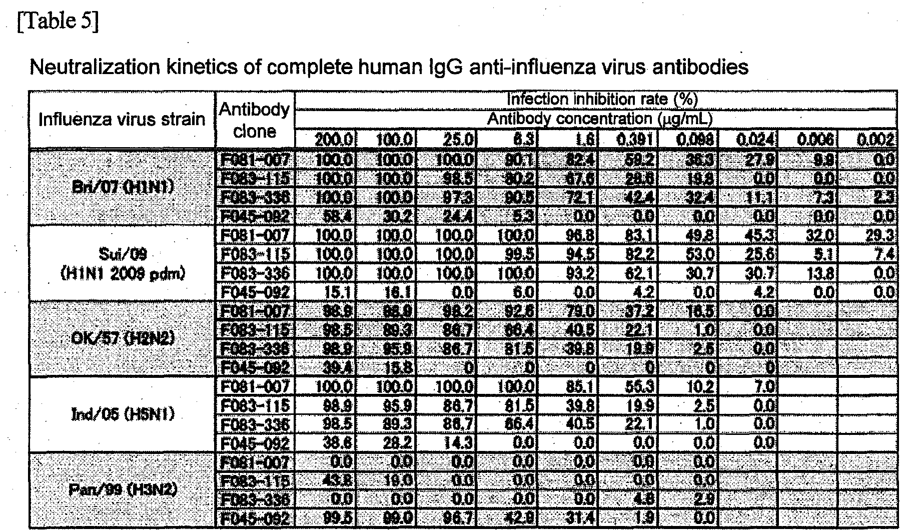

## Claims

1. An antibody, which neutralizes H1 influenza virus and/or H5 influenza virus, wherein the antibody has a heavy chain variable region having CDRs consisting of a heavy chain first complementarity-determining region (VH CDR1), a heavy chain second complementarity-determining region (VH CDR2) and a heavy chain third complementarity-determining region (VH CDR3), which are described in any one of the following (1) to (29), and a light chain variable region having CDRs consisting of a light chain first complementarity-determining region (VL CDR1), a light chain second complementarity-determining region (VL CDR2) and a light chain third complementarity-determining region (VL CDR3), which are described in any one of the following (1) to (29):
(1) VH CDR1 of SEQ ID NO: 1, VH CDR2 of SEQ ID NO: 2, VH CDR3 of SEQ ID NO: 3, VL CDR1 of SEQ ID NO: 4, VL CDR2 of SEQ ID NO: 5, and VL CDR3 of SEQ ID NO: 6,
(2) VH CDR1 of SEQ ID NO: 7, VH CDR2 of SEQ ID NO: 8, VH CDR3 of SEQ ID NO: 9, VL CDR1 of SEQ ID NO: 10, VL CDR2 of SEQ ID NO: 11, and VL CDR3 of SEQ ID NO: 12,
(3) VH CDR1 of SEQ ID NO: 13, VH CDR2 of SEQ ID NO: 14, VH CDR3 of SEQ ID NO: 15, VL CDR1 of SEQ ID NO:16, VL CDR2 of SEQ ID NO: 17, and VL CDR3 of SEQ ID NO: 18,
(4) VH CDR1 of SEQ ID NO: 19, VH CDR2 of SEQ ID NO: 20, VH CDR3 of SEQ ID NO: 21, VL CDR1 of SEQ ID NO: 22, VL CDR2 of SEQ ID NO: 23, and VL CDR3 of SEQ ID NO: 24,
(5) VH CDR1 of SEQ ID NO: 25, VH CDR2 of SEQ ID NO: 26, VH CDR3 of SEQ ID NO: 27, VL CDR1 of SEQ ID NO: 28, VL CDR2 of SEQ ID NO: 29, and VL CDR3 of SEQ ID NO: 30,
(6) VH CDR1 of SEQ ID NO: 31, VH CDR2 of SEQ ID NO: 32, VH CDR3 of SEQ ID NO: 33, VL CDR1 of SEQ ID NO: 34, VL CDR2 of SEQ ID NO: 35, and VL CDR3 of SEQ ID NO: 36,
(7) VH CDR1 of SEQ ID NO: 37, VH CDR2 of SEQ ID NO: 38, VH CDR3 of SEQ ID NO: 39, VL CDR1 of SEQ ID NO: 40, VL CDR2 of SEQ ID NO: 41, and VL CDR3 of SEQ ID NO: 42,
(8) VH CDR1 of SEQ ID NO: 43, VH CDR2 of SEQ ID NO: 44, VH CDR3 of SEQ ID NO: 45, VL CDR1 of SEQ ID NO: 46, VL CDR2 of SEQ ID NO: 47, and VL CDR3 of SEQ ID NO: 48,
(9) VH CDR1 of SEQ ID NO: 49, VH CDR2 of SEQ ID NO: 50, VH CDR3 of SEQ ID NO: 51, VL CDR1 of SEQ ID NO: 52, VL CDR2 of SEQ ID NO: 53, and VL CDR3 of SEQ ID NO: 54,
(10) VH CDR1 of SEQ ID NO: 55, VH CDR2 of SEQ ID NO: 56, VH CDR3 of SEQ ID NO: 57, VL CDR1 of SEQ ID NO: 58, VL CDR2 of SEQ ID NO: 59, and VL CDR3 of SEQ ID NO: 60,
(11) VH CDR1 of SEQ ID NO: 61, VH CDR2 of SEQ ID NO: 62, VH CDR3 of SEQ ID NO: 63, VL CDR1 of SEQ ID NO: 64, VL CDR2 of SEQ ID NO: 65, and VL CDR3 of SEQ ID NO: 66,
(12) VH CDR1 of SEQ ID NO: 67, VH CDR2 of SEQ ID NO: 68, VH CDR3 of SEQ ID NO: 69, VL CDR1 of SEQ ID NO: 70, VL CDR2 of SEQ ID NO: 71, and VL CDR3 of SEQ ID NO: 72,
(13) VH CDR1 of SEQ ID NO: 73, VH CDR2 of SEQ ID NO: 74, VH CDR3 of SEQ ID NO: 75, VL CDR1 of SEQ ID NO: 76, VL CDR2 of SEQ ID NO: 77, and VL CDR3 of SEQ ID NO: 78,
(14) VH CDR1 of SEQ ID NO: 79, VH CDR2 of SEQ ID NO: 80, VH CDR3 of SEQ ID NO: 81, VL CDR1 of SEQ ID NO: 82, VL CDR2 of SEQ ID NO: 83, and VL CDR3 of SEQ ID NO: 84,
(15) VH CDR1 of SEQ ID NO: 85, VH CDR2 of SEQ ID NO: 86, VH CDR3 of SEQ ID NO: 87, VL CDR1 of SEQ ID NO: 88, VL CDR2 of SEQ ID NO: 89, and VL CDR3 of SEQ ID NO: 90,
(16) VH CDR1 of SEQ ID NO: 91, VH CDR2 of SEQ ID NO: 92, VH CDR3 of SEQ ID NO: 93, VL CDR1 of SEQ ID NO: 94, VL CDR2 of SEQ ID NO: 95, and VL CDR3 of SEQ ID NO: 96,
(17) VH CDR1 of SEQ ID NO: 97, VH CDR2 of SEQ ID NO: 98, VH CDR3 of SEQ ID NO: 99, VLCDR1 of SEQ ID NO:100, VL CDR2 of SEQ ID NO:101, and VL CDR3 of SEQ ID NO: 102,
(18) VH CDR1 of SEQ ID NO: 103, VH CDR2 of SEQ ID NO: 104, VH CDR3 of SEQ ID NO: 105, VL CDR1 of SEQ ID NO: 106, VL CDR2 of SEQ ID NO: 107, and VL CDR3 of SEQ ID NO: 108,
(19) VH CDR1 of SEQ ID NO: 109, VH CDR2 of SEQ ID NO: 110, VH CDR3 of SEQ ID NO: 111, VLCDR1 of SEQ ID NO: 112, VL CDR2 of SEQ ID NO: 113, and VL CDR3 of SEQ ID NO: 114,
(20) VH CDR1 of SEQ ID NO: 115, VH CDR2 of SEQ ID NO: 116, VH CDR3 of SEQ ID NO: 117, VL CDR1 of SEQ ID NO:118, VL CDR2 of SEQ ID NO: 119, and VL CDR3 of SEQ ID NO: 120,
(21) VH CDR1 of SEQ ID NO: 121, VH CDR2 of SEQ ID NO: 122, VH CDR3 of SEQ ID NO: 123, VL CDR1 of SEQ ID NO: 124, VL CDR2 of SEQ ID NO: 125, and VL CDR3 of SEQ ID NO: 126,
(22) VH CDR1 of SEQ ID NO: 127, VH CDR2 of SEQ ID NO: 128, VH CDR3 of SEQ ID NO: 129, VL CDR1 of SEQ ID NO: 130, VL CDR2 of SEQ ID NO: 131, and VL CDR3 of SEQ ID NO: 132,
(23) VH CDR1 of SEQ ID NO: 133, VH CDR2 of SEQ ID NO: 134, VH CDR3 of SEQ NO: 135, VL CDR1 of SEQ ID NO: 136, VL CDR2 of SEQ ID NO: 137, and VL CDR3 of SEQ ID NO: 138,
(24) VH CDR1 of SEQ ID NO: 139, VH CDR2 of SEQ ID NO: 140, VH CDR3 of SEQ ID NO: 141, VL CDR1 of SEQ ID NO: 142, VL CDR2 of SEQ ID NO: 143, and VL CDR3 of SEQ ID NO: 144,
(25) VH CDR1 of SEQ ID NO: 145, VH CDR2 of SEQ ID NO: 146, VH CDR3 of SEQ ID NO: 147, VL CDR1 of SEQ ID NO:148, VL CDR2 of SEQ ID NO: 149, and VL CDR3 of SEQ ID NO: 150,
(26) VH CDR1 of SEQ ID NO: 151, VH CDR2 of SEQ ID NO: 152, VH CDR3 of SEQ ID NO: 153, VL CDR1 of SEQ ID NO: 154, VL CDR2 of SEQ ID NO: 155, and VL CDR3 of SEQ ID NO: 156,
(27) VH CDR1 of SEQ ID NO: 157, VH CDR2 of SEQ ID NO: 158, VH CDR3 of SEQ ID NO: 159, VL CDR1 of SEQ ID NO: 160, VL CDR2 of SEQ ID NO: 161, and VL CDR3 of SEQ ID NO: 162,
(28) VH CDR1 of SEQ ID NO: 163, VH CDR2 of SEQ ID NO: 164, VH CDR3 of SEQ ID NO: 165, VL CDR1 of SEQ ID NO:166, VL CDR2 of SEQ ID NO:167, and VL CDR3 of SEQ ID NO: 168, and
(29) VH CDR1 of SEQ ID NO: 169, VH CDR2 of SEQ ID NO: 170, VH CDR3 of SEQ ID NO: 171, VL CDR1 of SEQ ID NO: 172, VL CDR2 of SEQ ID NO: 173, and VL CDR3 of SEQ ID NO: 174.

2. The antibody according to claim 1, wherein the heavy chain variable region and the light chain variable region, respectively, consist of amino acid sequences having the amino acid sequence numbers described in any one of the following (1) to (29):
(1) SEQ ID NO: 176 and SEQ ID NO: 178,
(2) SEQ ID NO: 180 and SEQ ID NO: 182,
(3) SEQ ID NO: 184 and SEQ ID NO: 186,
(4) SEQ ID NO:188 and SEQ ID NO: 190,
(5) SEQ ID NO: 192 and SEQ ID NO: 194,
(6) SEQ ID NO: 196 and SEQ ID NO: 198,
(7) SEQ ID NO: 200 and SEQ ID NO: 202,
(8) SEQ ID NO: 204 and SEQ ID NO: 206,
(9) SEQ ID NO: 208 and SEQ ID NO: 210,
(10) SEQ ID NO: 212 and SEQ ID NO: 214,
(11) SEQ ID NO: 216 and SEQ ID NO: 218,
(12) SEQ ID NO: 220 and SEQ ID NO: 222,
(13) SEQ ID NO: 224 and SEQ ID NO: 226,
(14) SEQ ID NO: 228 and SEQ ID NO: 230,
(15) SEQ ID NO: 232 and SEQ ID NO: 234,
(16) SEQ ID NO: 236 and SEQ ID NO: 238,
(17) SEQ ID NO: 240 and SEQ ID NO: 242,
(18) SEQ ID NO: 244 and SEQ ID NO: 246,
(19) SEQ ID NO: 248 and SEQ ID NO: 250,
(20) SEQ ID NO: 252 and SEQ ID NO: 254,
(21) SEQ ID NO: 256 and SEQ ID NO: 258,
(22) SEQ ID NO: 260 and SEQ ID NO: 262,
(23) SEQ ID NO: 264 and SEQ ID NO: 266,
(24) SEQ ID NO: 268 and SEQ ID NO: 270,
(25) SEQ ID NO: 272 and SEQ ID NO: 274,
(26) SEQ ID NO: 276 and SEQ ID NO: 278,
(27) SEQ ID NO: 280 and SEQ ID NO: 282,
(28) SEQ ID NO: 284 and SEQ ID NO: 286, and
(29) SEQ ID NO: 288 and SEQ ID NO: 290.

3. The antibody according to claim 1 or 2, which is an antibody fragment selected from the group consisting of Fab, Fab', F(ab')₂, a single chain antibody (scFv), a dimerized V region (Diabody), a disulfide stabilized V region (dsFv), and a peptide comprising CDR.

4. DNA encoding the antibody according to any one of claims 1 to 3.

5. A recombinant vector comprising the DNA according to claim 4.

6. A transformed cell line obtained by introducing the recombinant vector according to claim 5 into a host cell.

7. A method for producing the antibody according to any one of claims 1 to 3, which comprises culturing the transformed cell line according to claim 6 in a medium, then allowing the transformed cell line to generate and accumulate the antibody according to any one of claims 1 to 3 in a culture, and then collecting the antibody from the culture.

8. A pharmaceutical composition comprising the antibody according to any one of claims 1 to 3.

9. A passive immunotherapeutic agent against influenza, which comprises the antibody according to any one of claims 1 to 3.


SEQUENCE LISTING
<110>  Fujita Health University
<110>  The Research Foundation For Microbial Disease of Osaka University
<110>  Perseus Proteomics Inc.
<120>  Anti-influenza virus neutralizing antibody
<130>  141621A
<160>  291
<170>  PatentIn version 3.5
<210>  1
<211>  5
<212>  PRT
<213>  human
<400>  1
Arg Asn Ala Ile Ser
1               5
<210>  2
<211>  17
<212>  PRT
<213>  human
<400>  2
Gly Ile Ile Ala Ile Phe Gly Thr Ala Asn Tyr Ala Gln Lys Phe Gln
1               5                   10                  15
Gly

<210>  3
<211>  11
<212>  PRT
<213>  human
<400>  3
Gly Pro Asn Tyr Tyr Glu Asn Phe Phe Asp Tyr
1               5                   10
<210>  4
<211>  11
<212>  PRT
<213>  human
<400>  4
Arg Ala Ser Gln Asp Ile Ala Asn Ser Leu Ala
1               5                   10
<210>  5
<211>  7
<212>  PRT
<213>  human
<400>  5
Gly Ala Ser Arg Leu Glu Gly
1               5
<210>  6
<211>  9
<212>  PRT
<213>  human
<400>  6
Gln Gln Tyr Phe Ser Phe Pro Arg Thr
1               5
<210>  7
<211>  5
<212>  PRT
<213>  human
<400>  7
Lys Asn Ala Ile Ser
1               5
<210>  8
<211>  17
<212>  PRT
<213>  human

<400>  8
Gly Ile Ser Ala Ile Phe Gly Thr Ala Asn Tyr Ala Gln Lys Phe Gln
1               5                   10                  15
Gly

<210>  9
<211>  15
<212>  PRT
<213>  human
<400>  9
His Pro Thr Tyr His Phe Asp Lys Ser Gly Tyr Arg Phe Asp Ser
1               5                   10                  15
<210>  10
<211>  13
<212>  PRT
<213>  human
<400>  10
Ser Gly Ser Ser Ser Asn Ile Gly Asn Asn Tyr Val Ser
1               5                   10
<210>  11
<211>  7
<212>  PRT
<213>  human
<400>  11
Asp Asn Asn Lys Arg Pro Ser
1               5
<210>  12
<211>  11
<212>  PRT
<213>  human
<400>  12
Gly Thr Trp Asp Ser Ser Leu Ser Ala Gly Val
1               5                   10
<210>  13
<211>  8
<212>  PRT
<213>  human
<400>  13
Ser Phe Ser Thr Tyr Thr Ile Ser
1               5
<210>  14
<211>  17
<212>  PRT
<213>  human
<400>  14
Arg Ser Ile Pro Leu Leu Gly Ile Thr Asn Tyr Ala Gln Lys Phe Gln
1               5                   10                  15
Gly

<210>  15
<211>  14
<212>  PRT
<213>  human
<400>  15
Tyr Gln Ser Ser Asp Tyr Tyr Asn Ser Glu Tyr Phe Gln His
1               5                   10
<210>  16
<211>  12
<212>  PRT
<213>  human
<400>  16
Arg Ala Ser Gln Asn Val Gly Thr Asn Tyr Leu Ala
1               5                   10

<210>  17
<211>  7
<212>  PRT
<213>  human
<400>  17
Gly Ala Ser Ser Arg Ala Thr
1               5
<210>  18
<211>  9
<212>  PRT
<213>  human
<400>  18
Gln Gln Tyr Ser Ser Ser Pro Leu Thr
1               5
<210>  19
<211>  5
<212>  PRT
<213>  human
<400>  19
Lys Asn Ala Ile Ser
1               5
<210>  20
<211>  17
<212>  PRT
<213>  human
<400>  20
Gly Ile Thr Ala Ile Phe Gly Thr Pro Asn Tyr Ala Gln Lys Phe Gln
1               5                   10                  15
Gly

<210>  21
<211>  11
<212>  PRT
<213>  human
<400>  21
Ser Gly Thr Tyr Tyr Val Ser Tyr Leu Asp Ser
1               5                   10
<210>  22
<211>  12
<212>  PRT
<213>  human
<400>  22
Arg Ala Ser Gln Ser Val Ser Ser Ser Tyr Leu Ala
1               5                   10
<210>  23
<211>  7
<212>  PRT
<213>  human
<400>  23
Gly Ala Ser Ser Arg Ala Thr
1               5
<210>  24
<211>  9
<212>  PRT
<213>  human
<400>  24
Gln Gln Tyr Gly Ser Ser Pro Leu Thr
1               5
<210>  25
<211>  5
<212>  PRT
<213>  human
<400>  25

Ala Tyr Gly Ile Ser
1               5
<210>  26
<211>  17
<212>  PRT
<213>  human
<400>  26
Gly Ile Val Ala Leu Phe Gly Thr Thr Asn Tyr Ala Gln Lys Leu Gln
1               5                   10                  15
Gly

<210>  27
<211>  11
<212>  PRT
<213>  human
<400>  27
Ser Gly Ser Tyr Tyr Pro Asp Tyr Phe Gln Tyr
1               5                   10
<210>  28
<211>  12
<212>  PRT
<213>  human
<400>  28
Arg Ala Ser Gln Ser Val Ser Ser Ser Tyr Leu Ala
1               5                   10
<210>  29
<211>  7
<212>  PRT
<213>  human
<400>  29
Gly Ala Ser Ser Arg Ala Thr
1               5
<210>  30
<211>  9
<212>  PRT
<213>  human
<400>  30
Gln Gln Tyr Gly Ser Ser Pro Arg Thr
1               5
<210>  31
<211>  5
<212>  PRT
<213>  human
<400>  31
Lys Tyr Ala Ile Thr
1               5
<210>  32
<211>  17
<212>  PRT
<213>  human
<400>  32
Gly Ile Ile Ala Ile Phe Gly Ser Thr Asn Tyr Ala Gln Lys Phe Gln
1               5                   10                  15
Gly

<210>  33
<211>  11
<212>  PRT
<213>  human
<400>  33
Gly Pro His Tyr Tyr Glu Ser His Leu Asp Tyr
1               5                   10
<210>  34

<211>  12
<212>  PRT
<213>  human
<400>  34
Arg Ala Ser Gln Gly Met Ser Ser Ser Phe Leu Ala
1               5                   10
<210>  35
<211>  7
<212>  PRT
<213>  human
<400>  35
Gly Ala Ser Thr Arg Ala Thr
1               5
<210>  36
<211>  9
<212>  PRT
<213>  human
<400>  36
Gln Gln Tyr Gly Ser Ser Pro Arg Thr
1               5
<210>  37
<211>  5
<212>  PRT
<213>  human
<400>  37
Arg Ser Ala Ile Ser
1               5
<210>  38
<211>  17
<212>  PRT
<213>  human
<400>  38
Gly Ile Leu Ala Ile Phe Gly Thr Thr Lys Tyr Ala Gln Lys Phe Gln
1               5                   10                  15
Gly

<210>  39
<211>  11
<212>  PRT
<213>  human
<400>  39
Gly Pro His Tyr Tyr Val Ser Tyr Phe Asp Ser
1               5                   10
<210>  40
<211>  11
<212>  PRT
<213>  human
<400>  40
Arg Ala Ser Gln Ser Val Arg Ser Tyr Leu Ala
1               5                   10
<210>  41
<211>  7
<212>  PRT
<213>  human
<400>  41
Asp Ala Ser Asn Arg Ala Thr
1               5
<210>  42
<211>  9
<212>  PRT
<213>  human
<400>  42
Gln Gln Arg Ser Asn Trp Leu Phe Thr

1                   5
<210>  43
<211>  5
<212>  PRT
<213>  human
<400>  43
Ser Asn Ala Val Ser
1                   5
<210>  44
<211>  17
<212>  PRT
<213>  human
<400>  44
Gly Ile Ile Ala Ile Phe Gly Thr Pro Lys Tyr Ala Gln Lys Phe Gln
1                   5                   10                  15
Gly

<210>  45
<211>  11
<212>  PRT
<213>  human
<400>  45
Gly Pro Asn Tyr Tyr Glu Ser Tyr Phe Asp Tyr
1                   5                   10
<210>  46
<211>  11
<212>  PRT
<213>  human
<400>  46
Arg Ala Ser Gln Gly Ile Ser Asn Tyr Leu Ala
1                   5                   10
<210>  47
<211>  7
<212>  PRT
<213>  human
<400>  47
Gly Ala Ser Thr Leu His Ser
1                   5
<210>  48
<211>  9
<212>  PRT
<213>  human
<400>  48
Gln Lys Tyr His Ser Ala Pro Leu Thr
1                   5
<210>  49
<211>  5
<212>  PRT
<213>  human
<400>  49
Lys Tyr Gly Ile Ser
1                   5
<210>  50
<211>  17
<212>  PRT
<213>  human
<400>  50
Gly Ile Leu Ala Ile Phe Gly Thr Thr Asn Tyr Ala Gln Lys Phe Gln
1                   5                   10                  15
Gly

<210>  51
<211>  11

<212> PRT
<213> human
<400> 51
Gly Asn Thr Tyr Tyr Ser Ser Tyr Phe Asp Gln
1               5                   10
<210> 52
<211> 11
<212> PRT
<213> human
<400> 52
Gly Gly Asp Lys Ile Gly Thr Gly Pro Val His
1               5                   10
<210> 53
<211> 7
<212> PRT
<213> human
<400> 53
Ala Ser Thr His Arg Pro Ser
1               5
<210> 54
<211> 12
<212> PRT
<213> human
<400> 54
Gln Met Trp Asp Ser Ser Ser Val His Pro Ile Val
1               5                   10
<210> 55
<211> 5
<212> PRT
<213> human
<400> 55
Lys Asn Ala Ile Ser
1               5
<210> 56
<211> 17
<212> PRT
<213> human
<400> 56
Gly Ile Ile Ala Ile Phe Gly Thr Ala Asn Tyr Ala Gln Lys Phe Gln
1               5                   10                  15
Gly

<210> 57
<211> 11
<212> PRT
<213> human
<400> 57
Gly Pro Asn Tyr Tyr Glu Asn Tyr Phe Asp Phe
1               5                   10
<210> 58
<211> 11
<212> PRT
<213> human
<400> 58
Arg Thr Ser Gln Ala Ile Thr Asn Tyr Leu Ala
1               5                   10
<210> 59
<211> 7
<212> PRT
<213> human
<400> 59
Ala Ala Ser Val Leu Gln Ser
1               5

<210>  60
<211>  9
<212>  PRT
<213>  human
<400>  60
Gln Lys Tyr Asp Ser Ala Pro Tyr Thr
1               5
<210>  61
<211>  5
<212>  PRT
<213>  human
<400>  61
Ser His Ala Ile Ser
1               5
<210>  62
<211>  17
<212>  PRT
<213>  human
<400>  62
Gly Ile Thr Ala Val Phe Gly Thr Ala Asn Tyr Ala Gln Lys Phe Gln
1               5                   10                  15
Gly

<210>  63
<211>  13
<212>  PRT
<213>  human
<400>  63
Ser Leu Gly Tyr His Thr Gln Tyr Asn Gly Met Asp Val
1               5                   10
<210>  64
<211>  11
<212>  PRT
<213>  human
<400>  64
Arg Ala Ser Gln Gly Ile Ser Ser Trp Leu Ala
1               5                   10
<210>  65
<211>  7
<212>  PRT
<213>  human
<400>  65
Ala Ala Ser Ser Leu Gln Ser
1               5
<210>  66
<211>  9
<212>  PRT
<213>  human
<400>  66
Gln Gln Ala Asn Ser Phe Pro Ile Thr
1               5
<210>  67
<211>  5
<212>  PRT
<213>  human
<400>  67
Asn Tyr Ala Ile Ser
1               5
<210>  68
<211>  17
<212>  PRT
<213>  human
<400>  68

Gly Ile Thr Pro Ile Phe Gly Ala Ala Asn Tyr Ala Gln Lys Phe Gln
1               5                   10                  15
Gly

<210>  69
<211>  12
<212>  PRT
<213>  human
<400>  69
His Pro Thr Tyr His Tyr Gly Ser Ala Met Asp Tyr
1               5                   10
<210>  70
<211>  12
<212>  PRT
<213>  human
<400>  70
Gly Ser Ser Ser Asn Ile Gly Asn Asn Tyr Val Ser
1               5                   10
<210>  71
<211>  7
<212>  PRT
<213>  human
<400>  71
Glu Gly Ser Asn Arg Pro Ser
1               5
<210>  72
<211>  11
<212>  PRT
<213>  human
<400>  72
Ala Thr Trp Asp Asp Ser Leu Ser Val Val Leu
1               5                   10
<210>  73
<211>  5
<212>  PRT
<213>  human
<400>  73
Lys Ser Ala Ile Thr
1               5
<210>  74
<211>  17
<212>  PRT
<213>  human
<400>  74
Gly Ile Ile Ala Ile Phe Gly Thr Thr Asn Tyr Ala Gln Lys Phe Gln
1               5                   10                  15
Asp

<210>  75
<211>  11
<212>  PRT
<213>  human
<400>  75
Gly Pro Asn Tyr Tyr Glu Ser Tyr Leu Asp Phe
1               5                   10
<210>  76
<211>  11
<212>  PRT
<213>  human
<400>  76
Arg Ala Ser Gln Ser Ile Ser Ser Trp Leu Ala
1               5                   10
<210>  77

<211> 7
<212> PRT
<213> human
<400> 77
Lys Ala Ser Ser Leu Glu Ser
1               5
<210> 78
<211> 9
<212> PRT
<213> human
<400> 78
Gln Gln Tyr Asn Ser Tyr Pro Arg Thr
1               5
<210> 79
<211> 5
<212> PRT
<213> human
<400> 79
Ser Tyr Ala Ile Ser
1               5
<210> 80
<211> 17
<212> PRT
<213> human
<400> 80
Gly Ile Ser Pro Met Phe Gly Thr Thr Arg Tyr Ala Gln Lys Phe Gln
1               5                   10                  15
Gly

<210> 81
<211> 11
<212> PRT
<213> human
<400> 81
Ser Pro Thr Tyr Tyr Pro Gly Ala Leu Asp Met
1               5                   10
<210> 82
<211> 11
<212> PRT
<213> human
<400> 82
Arg Ala Ser Gln Ala Ile Ala Asn Phe Val Ser
1               5                   10
<210> 83
<211> 7
<212> PRT
<213> human
<400> 83
Ala Ala Ser Thr Leu Gln Gly
1               5
<210> 84
<211> 9
<212> PRT
<213> human
<400> 84
Gln Gln Tyr Asn Ser Leu Pro Phe Thr
1               5
<210> 85
<211> 5
<212> PRT
<213> human
<400> 85
Ser Asn Ala Ile Ser

1                   5
<210>  86
<211>  17
<212>  PRT
<213>  human
<400>  86
Gly Ile Ile Gly Ile Phe Gly Thr Ala Asn Tyr Ala Gln Lys Phe Gln
1                   5                   10                  15
Gly

<210>  87
<211>  15
<212>  PRT
<213>  human
<400>  87
Ser Arg Gly Tyr Ser Phe Gly Tyr Gly Thr Asp Tyr Phe Asp Tyr
1                   5                   10                  15
<210>  88
<211>  11
<212>  PRT
<213>  human
<400>  88
Arg Ala Ser Gln Ser Ile Ser Ser Trp Leu Ala
1                   5                   10
<210>  89
<211>  7
<212>  PRT
<213>  human
<400>  89
Glu Ala Ser Lys Leu Gln Ser
1                   5
<210>  90
<211>  9
<212>  PRT
<213>  human
<400>  90
Gln His Tyr Glu Ala Tyr Pro Phe Ser
1                   5
<210>  91
<211>  5
<212>  PRT
<213>  human
<400>  91
Ser Asn Ala Phe Ser
1                   5
<210>  92
<211>  17
<212>  PRT
<213>  human
<400>  92
Gly Ile Ile Thr Met Phe Arg Lys Ala Glu Tyr Ala Gln Lys Phe Gln
1                   5                   10                  15
Gly

<210>  93
<211>  12
<212>  PRT
<213>  human
<400>  93
His Ser Gly Tyr His Leu Ile Gly Tyr Phe Asp Ser
1                   5                   10
<210>  94
<211>  13

<212>  PRT
<213>  human
<400>  94
Ser Gly Thr Thr Ser Asn Ile Gly Asn Asn Tyr Val Ser
1               5                   10
<210>  95
<211>  7
<212>  PRT
<213>  human
<400>  95
Asp Asn Asn Lys Arg Pro Ser
1               5
<210>  96
<211>  11
<212>  PRT
<213>  human
<400>  96
Gly Thr Trp Val Ser Ser Leu Ser Val Trp Val
1               5                   10
<210>  97
<211>  5
<212>  PRT
<213>  human
<400>  97
Asn Tyr Ala Ile Ser
1               5
<210>  98
<211>  17
<212>  PRT
<213>  human
<400>  98
Gly Ile Val Pro Met Phe Gly Thr Thr Arg Phe Ala Gln Lys Phe Gln
1               5                   10                  15
Gly

<210>  99
<211>  11
<212>  PRT
<213>  human
<400>  99
Ala Pro Leu Ile Tyr Asn Trp Tyr Phe Asp Leu
1               5                   10
<210>  100
<211>  11
<212>  PRT
<213>  human
<400>  100
Arg Ala Ser Gln Gly Ile Ser Ser Trp Leu Ala
1               5                   10
<210>  101
<211>  7
<212>  PRT
<213>  human
<400>  101
Gly Ala Ser Asn Leu Gln Ser
1               5
<210>  102
<211>  9
<212>  PRT
<213>  human
<400>  102
Gln Gln Ala Arg Thr Phe Pro Val Thr
1               5

<210>  103
<211>  5
<212>  PRT
<213>  human
<400>  103
Asn Tyr Ala Ile Ser
1               5
<210>  104
<211>  17
<212>  PRT
<213>  human
<400>  104
Gly Ile Thr Pro Ile Phe Gly Pro Ala Asn Tyr Ala Gln Arg Phe Gln
1               5                   10                  15
Gly

<210>  105
<211>  12
<212>  PRT
<213>  human
<400>  105
His Pro Thr Tyr Tyr Phe Gly Ser Ala Met Glu Tyr
1               5                   10
<210>  106
<211>  11
<212>  PRT
<213>  human
<400>  106
Gly Gly Asp Arg Ile Gly Thr Gly Pro Val His
1               5                   10
<210>  107
<211>  7
<212>  PRT
<213>  human
<400>  107
Gly Ser Thr His Arg Pro Ser
1               5
<210>  108
<211>  11
<212>  PRT
<213>  human
<400>  108
Gln Val Trp Asp Thr Ser Thr Ala Gln Pro Ile
1               5                   10
<210>  109
<211>  5
<212>  PRT
<213>  human
<400>  109
Ser His Ala Ile Ser
1               5
<210>  110
<211>  17
<212>  PRT
<213>  human
<400>  110
Gly Ile Ile Ala Ile Phe Gly Thr Thr His Tyr Ala Gln Gln Phe Gln
1               5                   10                  15
Gly

<210>  111
<211>  11
<212>  PRT

<213>  human
<400>  111
Gly Pro Asn Tyr Phe Glu Ser Tyr Phe Asp Asn
1               5                   10
<210>  112
<211>  11
<212>  PRT
<213>  human
<400>  112
Arg Ala Ser Gln Thr Ile Ser Ser Tyr Leu Ala
1               5                   10
<210>  113
<211>  7
<212>  PRT
<213>  human
<400>  113
Gly Ala Ser Asn Arg Ala Thr
1               5
<210>  114
<211>  10
<212>  PRT
<213>  human
<400>  114
Gln Gln Arg Ser Ser Trp Pro Pro Ile Thr
1               5                   10
<210>  115
<211>  5
<212>  PRT
<213>  human
<400>  115
Thr Asn Ala Ile Ser
1               5
<210>  116
<211>  17
<212>  PRT
<213>  human
<400>  116
Gly Ile Thr Ala Ile Phe Gly Thr Pro Asn Tyr Ala Gln Lys Phe Gln
1               5                   10                  15
Gly

<210>  117
<211>  11
<212>  PRT
<213>  human
<400>  117
Ser Gly Thr Tyr Tyr Val Ser Tyr Phe Asp Ser
1               5                   10
<210>  118
<211>  12
<212>  PRT
<213>  human
<400>  118
Arg Ala Ser Gln Ser Leu Tyr Ser Ser His Leu Ala
1               5                   10
<210>  119
<211>  7
<212>  PRT
<213>  human
<400>  119
Gly Ala Ser Thr Arg Ala Pro
1               5
<210>  120

<211> 9
<212> PRT
<213> human
<400> 120
Gln Gln Tyr Gly Ser Ser Pro Ile Thr
1               5
<210> 121
<211> 5
<212> PRT
<213> human
<400> 121
Asn Tyr Ala Ile Asn
1               5
<210> 122
<211> 17
<212> PRT
<213> human
<400> 122
Gly Ile Val Pro Ile Phe Gly Thr Thr Arg Phe Ala Gln Lys Phe Gln
1               5                   10                  15
Gly

<210> 123
<211> 11
<212> PRT
<213> human
<400> 123
Ala Pro Leu Ile Tyr Asn Trp Tyr Tyr Asp Leu
1               5                   10
<210> 124
<211> 11
<212> PRT
<213> human
<400> 124
Arg Ala Ser Gln Gly Ile Ser Asn Tyr Leu Ala
1               5                   10
<210> 125
<211> 7
<212> PRT
<213> human
<400> 125
Ala Ala Ser Thr Leu Gln Ser
1               5
<210> 126
<211> 9
<212> PRT
<213> human
<400> 126
Gln Lys Tyr Asn Ser Ala Pro His Thr
1               5
<210> 127
<211> 5
<212> PRT
<213> human
<400> 127
Ser Tyr Ala Leu Ser
1               5
<210> 128
<211> 17
<212> PRT
<213> human
<400> 128
Gly Ile Ser Ala Ile Phe Asn Thr Ala Thr Tyr Ala Gln Asn Val Gln

1               5                   10                  15
Gly

<210>  129
<211>  11
<212>  PRT
<213>  human
<400>  129
Ser Gly Thr Tyr Tyr Val Ser Phe Phe Asp Tyr
1               5                   10
<210>  130
<211>  11
<212>  PRT
<213>  human
<400>  130
Arg Ala Ser Gln Ser Ile Gly Ser Leu Met Ala
1               5                   10
<210>  131
<211>  7
<212>  PRT
<213>  human
<400>  131
Arg Ala Ser Asn Leu Glu Thr
1               5
<210>  132
<211>  9
<212>  PRT
<213>  human
<400>  132
Gln His Phe Lys Thr Phe Ser Arg Thr
1               5
<210>  133
<211>  5
<212>  PRT
<213>  human
<400>  133
Ser Asn Ala Ile Ser
1               5
<210>  134
<211>  17
<212>  PRT
<213>  human
<400>  134
Gly Ile Val Ala Leu Phe Gly Thr Ala Asn Tyr Ala Gln Lys Phe Gln
1               5                   10                  15
Gly

<210>  135
<211>  13
<212>  PRT
<213>  human
<400>  135
Asn Ser Gly Tyr His Ile Ser Gly Phe Tyr Leu Asp Tyr
1               5                   10
<210>  136
<211>  11
<212>  PRT
<213>  human
<400>  136
Arg Ala Ser Gln Gly Ile Ser Asn Tyr Leu Ala
1               5                   10
<210>  137
<211>  7

<212>  PRT
<213>  human
<400>  137
Thr Ala Ser Thr Leu Leu Ser
1               5
<210>  138
<211>  9
<212>  PRT
<213>  human
<400>  138
Gln Glu Tyr Lys Ser Val Pro Leu Thr
1               5
<210>  139
<211>  5
<212>  PRT
<213>  human
<400>  139
Lys Phe Ala Ile Ser
1               5
<210>  140
<211>  17
<212>  PRT
<213>  human
<400>  140
Gly Ile Ile Pro Met Phe Gly Thr Thr Asn Tyr Ala Gln Lys Phe Gln
1               5                   10                  15
Gly

<210>  141
<211>  13
<212>  PRT
<213>  human
<400>  141
Glu Glu Gly Tyr Tyr Tyr Gly Ser Gly Pro Leu Asp Ser
1               5                   10
<210>  142
<211>  17
<212>  PRT
<213>  human
<400>  142
Lys Ser Ser Gln Thr Val Leu Asn Arg Ser Asn Asn Lys Asn Tyr Leu
1               5                   10                  15
Ala

<210>  143
<211>  7
<212>  PRT
<213>  human
<400>  143
Trp Ser Ser Thr Arg Glu Ser
1               5
<210>  144
<211>  9
<212>  PRT
<213>  human
<400>  144
Gln Gln Phe Tyr Asp Met Pro Val Thr
1               5
<210>  145
<211>  5
<212>  PRT
<213>  human
<400>  145

Asn Tyr Ala Ile Ser
1               5
<210>  146
<211>  17
<212>  PRT
<213>  human
<400>  146
Gly Ile Thr Pro Ile Phe Gly Ala Ala Asn Tyr Ala Gln Lys Phe Gln
1               5                   10                  15
Gly

<210>  147
<211>  12
<212>  PRT
<213>  human
<400>  147
His Pro Met Tyr His Tyr Gly Ser Ala Met Asp Tyr
1               5                   10
<210>  148
<211>  13
<212>  PRT
<213>  human
<400>  148
Phe Gly Ser Arg Ser Asn Val Gly Ser Ser Ser Val Asn
1               5                   10
<210>  149
<211>  7
<212>  PRT
<213>  human
<400>  149
Arg Asn Asp Gln Arg Pro Ser
1               5
<210>  150
<211>  11
<212>  PRT
<213>  human
<400>  150
Ala Ala Trp Asp Asp Ser Val Lys Ser Val Val
1               5                   10
<210>  151
<211>  5
<212>  PRT
<213>  human
<400>  151
Ser Tyr Gly Ile Ser
1               5
<210>  152
<211>  17
<212>  PRT
<213>  human
<400>  152
Gly Ile Leu Ala Ile Phe Gly Thr Thr Asn Tyr Ala Gln Lys Phe Gln
1               5                   10                  15
Gly

<210>  153
<211>  11
<212>  PRT
<213>  human
<400>  153
Gly Ser Thr Tyr Tyr Ser Ser Tyr Phe Asp Gln
1               5                   10
<210>  154

<211>  11
<212>  PRT
<213>  human
<400>  154
Arg Ala Ser Gln Gly Ile Arg Asn Ser Leu Ala
1               5                   10
<210>  155
<211>  7
<212>  PRT
<213>  human
<400>  155
Ser Ala Thr Thr Leu Arg Ser
1               5
<210>  156
<211>  9
<212>  PRT
<213>  human
<400>  156
His Arg Tyr Asp Ser Ala Pro Leu Thr
1               5
<210>  157
<211>  5
<212>  PRT
<213>  human
<400>  157
Ser Tyr Ala Ile Ser
1               5
<210>  158
<211>  17
<212>  PRT
<213>  human
<400>  158
Ala Val Ile Pro Met Phe Gly Thr Leu Lys Tyr Ala Glu Asn Phe Gln
1               5                   10                  15
Gly

<210>  159
<211>  15
<212>  PRT
<213>  human
<400>  159
Asn Tyr Tyr Gly Ser Gly Thr Tyr Phe Asn Asp Ala Phe Asp Ile
1               5                   10                  15
<210>  160
<211>  14
<212>  PRT
<213>  human
<400>  160
Thr Gly Gly Asn Ser Asn Ile Gly Ala Gly Tyr Asp Val Asn
1               5                   10
<210>  161
<211>  7
<212>  PRT
<213>  human
<400>  161
Gly Asn Asn Asn Arg Pro Ser
1               5
<210>  162
<211>  11
<212>  PRT
<213>  human
<400>  162
Gln Ser Tyr Asp Asn Gly Leu Ser Gly Ser Val

1                   5                   10
<210>  163
<211>  5
<212>  PRT
<213>  human
<400>  163
Asn Tyr Ala Ile Ser
1               5
<210>  164
<211>  17
<212>  PRT
<213>  human
<400>  164
Gly Ile Thr Pro Ile Phe Gly Ala Ala Asn Tyr Ala Gln Lys Phe Gln
1               5                   10                  15
Gly

<210>  165
<211>  12
<212>  PRT
<213>  human
<400>  165
His Pro Thr Tyr Tyr Tyr Gly Ser Pro Met Asp Tyr
1               5                   10
<210>  166
<211>  11
<212>  PRT
<213>  human
<400>  166
Arg Ala Ser Gln Ser Ile Ala Thr Tyr Leu Asn
1               5                   10
<210>  167
<211>  7
<212>  PRT
<213>  human
<400>  167
Ala Ala Ser Asn Leu Gln Ser
1               5
<210>  168
<211>  9
<212>  PRT
<213>  human
<400>  168
Leu Gln Ser Tyr Ser Ala Thr Leu Thr
1               5
<210>  169
<211>  5
<212>  PRT
<213>  human
<400>  169
Lys Phe Ala Ile Thr
1               5
<210>  170
<211>  17
<212>  PRT
<213>  human
<400>  170
Gly Ile Thr Ala Leu Phe Ala Thr Thr Ser Tyr Ala Gln Lys Phe Gln
1               5                   10                  15
Asp

<210>  171
<211>  11

<212>  PRT
<213>  human
<400>  171
Gly Pro Asn Tyr Tyr Glu Thr Tyr Leu Asp Asn
1               5                   10
<210>  172
<211>  11
<212>  PRT
<213>  human
<400>  172
Arg Ala Ser Gln Ser Ile Ser Lys Trp Leu Ala
1               5                   10
<210>  173
<211>  7
<212>  PRT
<213>  human
<400>  173
Met Ala Ser Thr Leu Glu Ser
1               5
<210>  174
<211>  9
<212>  PRT
<213>  human
<400>  174
Gln Gln Tyr Asn Ser Tyr Pro Arg Thr
1               5
<210>  175
<211>  360
<212>  DNA
<213>  human
<220>
<221>  CDS
<222>  (1)..(360)
<400>  175
cag gtg cag ctg cag gag tcg ggg gct gag gtg aag aag cct ggg tcc      48
Gln Val Gln Leu Gln Glu Ser Gly Ala Glu Val Lys Lys Pro Gly Ser
1               5                   10                  15
tcg gtg aag gtc tcc tgc aag gcc tct gga ggg atc ttc agg aga aat      96
Ser Val Lys Val Ser Cys Lys Ala Ser Gly Gly Ile Phe Arg Arg Asn
            20                  25                  30
gct atc agc tgg gtg cga cag gcc cct gga caa ggg ctt gag tgg atg      144
Ala Ile Ser Trp Val Arg Gln Ala Pro Gly Gln Gly Leu Glu Trp Met
        35                  40                  45
gga ggg atc atc gct atc ttt ggg aca gca aac tac gca cag aag ttt      192
Gly Gly Ile Ile Ala Ile Phe Gly Thr Ala Asn Tyr Ala Gln Lys Phe
    50                  55                  60
cag ggc aga gtc acg att act gcg gac gaa tcc acg agc aca gtc tac      240
Gln Gly Arg Val Thr Ile Thr Ala Asp Glu Ser Thr Ser Thr Val Tyr
65                  70                  75                  80
atg gaa ctg agc agc ctg aga cct gag gac acg gcc gtg tat tac tgt      288
Met Glu Leu Ser Ser Leu Arg Pro Glu Asp Thr Ala Val Tyr Tyr Cys
                85                  90                  95
gcg aga ggg ccg aat tac tat gaa aat ttc ttt gac tac tgg ggc cag      336
Ala Arg Gly Pro Asn Tyr Tyr Glu Asn Phe Phe Asp Tyr Trp Gly Gln
            100                 105                 110
gga acc ctg gtc acc gtc tcg agc                                      360
Gly Thr Leu Val Thr Val Ser Ser
        115                 120
<210>  176
<211>  120
<212>  PRT
<213>  human
<400>  176

Gln Val Gln Leu Gln Glu Ser Gly Ala Glu Val Lys Lys Pro Gly Ser
1               5                   10                  15
Ser Val Lys Val Ser Cys Lys Ala Ser Gly Gly Ile Phe Arg Arg Asn
            20                  25                  30
Ala Ile Ser Trp Val Arg Gln Ala Pro Gly Gln Gly Leu Glu Trp Met
        35                  40                  45
Gly Gly Ile Ile Ala Ile Phe Gly Thr Ala Asn Tyr Ala Gln Lys Phe
    50                  55                  60
Gln Gly Arg Val Thr Ile Thr Ala Asp Glu Ser Thr Ser Thr Val Tyr
65                  70                  75                  80
Met Glu Leu Ser Ser Leu Arg Pro Glu Asp Thr Ala Val Tyr Tyr Cys
                85                  90                  95
Ala Arg Gly Pro Asn Tyr Tyr Glu Asn Phe Phe Asp Tyr Trp Gly Gln
            100                 105                 110
Gly Thr Leu Val Thr Val Ser Ser
        115                 120
<210>  177
<211>  321
<212>  DNA
<213>  human
<220>
<221>  CDS
<222>  (1)..(321)
<400>  177
gac atc gtg atg acc cag tct cca tcc tcc ctg tct gct tct gtg gga        48
Asp Ile Val Met Thr Gln Ser Pro Ser Ser Leu Ser Ala Ser Val Gly
1               5                   10                  15
gac aga ctt gac ctc act tgc cgg gcg agt cag gac atc gcc aat tcc        96
Asp Arg Leu Asp Leu Thr Cys Arg Ala Ser Gln Asp Ile Ala Asn Ser
            20                  25                  30
tta gcg tgg tat aaa caa aga ccg ggg aag gcc cct gaa ctc ctg gtc        144
Leu Ala Trp Tyr Lys Gln Arg Pro Gly Lys Ala Pro Glu Leu Leu Val
        35                  40                  45
ttt ggt gct tcc agg ttg gag ggt gga gtc ccg tct aga ttc agt ggc        192
Phe Gly Ala Ser Arg Leu Glu Gly Gly Val Pro Ser Arg Phe Ser Gly
    50                  55                  60
act aga gcc ggg aca gat ttc act ctc acc atc agc agt ctg cag cct        240
Thr Arg Ala Gly Thr Asp Phe Thr Leu Thr Ile Ser Ser Leu Gln Pro
65                  70                  75                  80
gaa gac ctt ggt act tat ttc tgt cag cag tat ttt tcg ttt cct cgg        288
Glu Asp Leu Gly Thr Tyr Phe Cys Gln Gln Tyr Phe Ser Phe Pro Arg
                85                  90                  95
acc ttc ggc cag ggg acc acg ctg gac ctc aga                            321
Thr Phe Gly Gln Gly Thr Thr Leu Asp Leu Arg
            100                 105
<210>  178
<211>  107
<212>  PRT
<213>  human
<400>  178
Asp Ile Val Met Thr Gln Ser Pro Ser Ser Leu Ser Ala Ser Val Gly
1               5                   10                  15
Asp Arg Leu Asp Leu Thr Cys Arg Ala Ser Gln Asp Ile Ala Asn Ser
            20                  25                  30
Leu Ala Trp Tyr Lys Gln Arg Pro Gly Lys Ala Pro Glu Leu Leu Val
        35                  40                  45
Phe Gly Ala Ser Arg Leu Glu Gly Gly Val Pro Ser Arg Phe Ser Gly
    50                  55                  60
Thr Arg Ala Gly Thr Asp Phe Thr Leu Thr Ile Ser Ser Leu Gln Pro
65                  70                  75                  80
Glu Asp Leu Gly Thr Tyr Phe Cys Gln Gln Tyr Phe Ser Phe Pro Arg
                85                  90                  95
Thr Phe Gly Gln Gly Thr Thr Leu Asp Leu Arg

            100                 105
<210> 179
<211> 372
<212> DNA
<213> human
<220>
<221> CDS
<222> (1)..(372)
<400> 179
cag gta cag ctg cag cag tca ggg gct gag gtg aag aag cct ggg tcc 48
Gln Val Gln Leu Gln Gln Ser Gly Ala Glu Val Lys Lys Pro Gly Ser
1               5                   10                  15
tcg gtg aag gtg tcc tgc aag gct tct gga ggc atc ttc aga aaa aat 96
Ser Val Lys Val Ser Cys Lys Ala Ser Gly Gly Ile Phe Arg Lys Asn
            20                  25                  30
gct atc agc tgg gtg cga cag gcc cct gga caa ggg ctt gag tgg atg 144
Ala Ile Ser Trp Val Arg Gln Ala Pro Gly Gln Gly Leu Glu Trp Met
        35                  40                  45
gga ggg atc agc gct atc ttt ggt acg gca aac tac gca cag aag ttc 192
Gly Gly Ile Ser Ala Ile Phe Gly Thr Ala Asn Tyr Ala Gln Lys Phe
    50                  55                  60
cag ggc aga gtc acg att acc gcg gac gaa tcg tcg aat aca gtc tat 240
Gln Gly Arg Val Thr Ile Thr Ala Asp Glu Ser Ser Asn Thr Val Tyr
65                  70                  75                  80
atg gac ctg agc agg ctg aga tct gag gac acg gcc att tat tac tgt 288
Met Asp Leu Ser Arg Leu Arg Ser Glu Asp Thr Ala Ile Tyr Tyr Cys
                85                  90                  95
gcg agc cac cct acc tat cac ttt gat aaa agt ggt tat cgc ttt gac 336
Ala Ser His Pro Thr Tyr His Phe Asp Lys Ser Gly Tyr Arg Phe Asp
            100                 105                 110
tcc tgg ggc cag gga acc ctg gtc acc gtc tcg agc 372
Ser Trp Gly Gln Gly Thr Leu Val Thr Val Ser Ser
        115                 120
<210> 180
<211> 124
<212> PRT
<213> human
<400> 180
Gln Val Gln Leu Gln Gln Ser Gly Ala Glu Val Lys Lys Pro Gly Ser
1               5                   10                  15
Ser Val Lys Val Ser Cys Lys Ala Ser Gly Gly Ile Phe Arg Lys Asn
            20                  25                  30
Ala Ile Ser Trp Val Arg Gln Ala Pro Gly Gln Gly Leu Glu Trp Met
        35                  40                  45
Gly Gly Ile Ser Ala Ile Phe Gly Thr Ala Asn Tyr Ala Gln Lys Phe
    50                  55                  60
Gln Gly Arg Val Thr Ile Thr Ala Asp Glu Ser Ser Asn Thr Val Tyr
65                  70                  75                  80
Met Asp Leu Ser Arg Leu Arg Ser Glu Asp Thr Ala Ile Tyr Tyr Cys
                85                  90                  95
Ala Ser His Pro Thr Tyr His Phe Asp Lys Ser Gly Tyr Arg Phe Asp
            100                 105                 110
Ser Trp Gly Gln Gly Thr Leu Val Thr Val Ser Ser
        115                 120
<210> 181
<211> 333
<212> DNA
<213> human
<220>
<221> CDS
<222> (1)..(333)
<400> 181
cag tct gtg ttg acg cag ccg ccc tca gtg tct gcg gcc cca gga cag 48

Gln Ser Val Leu Thr Gln Pro Pro Ser Val Ser Ala Ala Pro Gly Gln
1               5                   10                  15
aag gtc acc atc tcc tgc tct gga agc agc tcc aac att ggg aat aat      96
Lys Val Thr Ile Ser Cys Ser Gly Ser Ser Ser Asn Ile Gly Asn Asn
            20                  25                  30
tat gta tcc tgg tac cag cag ctc cca gga aca gcc ccc aaa ctc ctc      144
Tyr Val Ser Trp Tyr Gln Gln Leu Pro Gly Thr Ala Pro Lys Leu Leu
        35                  40                  45
att tat gac aat aat aag cga ccc tca ggg att cct gac cga ttc tct      192
Ile Tyr Asp Asn Asn Lys Arg Pro Ser Gly Ile Pro Asp Arg Phe Ser
    50                  55                  60
ggc tcc aag tct ggc acg tca gcc acc ctg ggc atc acc gga ctc cag      240
Gly Ser Lys Ser Gly Thr Ser Ala Thr Leu Gly Ile Thr Gly Leu Gln
65                  70                  75                  80
act ggg gac gag gcc gat tat tac tgc gga aca tgg gat agc agc ctg      288
Thr Gly Asp Glu Ala Asp Tyr Tyr Cys Gly Thr Trp Asp Ser Ser Leu
                85                  90                  95
agt gct ggg gtg ttc ggc gga ggg acc aag ctg acc gtc cta ggt          333
Ser Ala Gly Val Phe Gly Gly Gly Thr Lys Leu Thr Val Leu Gly
            100                 105                 110
<210>  182
<211>  111
<212>  PRT
<213>  human
<400>  182
Gln Ser Val Leu Thr Gln Pro Pro Ser Val Ser Ala Ala Pro Gly Gln
1               5                   10                  15
Lys Val Thr Ile Ser Cys Ser Gly Ser Ser Ser Asn Ile Gly Asn Asn
            20                  25                  30
Tyr Val Ser Trp Tyr Gln Gln Leu Pro Gly Thr Ala Pro Lys Leu Leu
        35                  40                  45
Ile Tyr Asp Asn Asn Lys Arg Pro Ser Gly Ile Pro Asp Arg Phe Ser
    50                  55                  60
Gly Ser Lys Ser Gly Thr Ser Ala Thr Leu Gly Ile Thr Gly Leu Gln
65                  70                  75                  80
Thr Gly Asp Glu Ala Asp Tyr Tyr Cys Gly Thr Trp Asp Ser Ser Leu
                85                  90                  95
Ser Ala Gly Val Phe Gly Gly Gly Thr Lys Leu Thr Val Leu Gly
            100                 105                 110
<210>  183
<211>  378
<212>  DNA
<213>  human
<220>
<221>  CDS
<222>  (1)..(378)
<400>  183
cag gtg cag ctg gtg cag tct ggg gct gaa gtg aag aag cct ggg tcc      48
Gln Val Gln Leu Val Gln Ser Gly Ala Glu Val Lys Lys Pro Gly Ser
1               5                   10                  15
tcg gtg aag gtc tcc tgc aag gct tct gga ggc acc ttc agt agc ttc      96
Ser Val Lys Val Ser Cys Lys Ala Ser Gly Gly Thr Phe Ser Ser Phe
            20                  25                  30
agc acc tat act atc agc tgg gtg cga cag gcc cct gga caa ggg ctt      144
Ser Thr Tyr Thr Ile Ser Trp Val Arg Gln Ala Pro Gly Gln Gly Leu
        35                  40                  45
gag tgg atg gga agg tcc atc cct ctc ctt ggt ata aca aac tac gca      192
Glu Trp Met Gly Arg Ser Ile Pro Leu Leu Gly Ile Thr Asn Tyr Ala
    50                  55                  60
cag aag ttc cag ggc aga gtc acg att acc gcg gac ata tcc acg agc      240
Gln Lys Phe Gln Gly Arg Val Thr Ile Thr Ala Asp Ile Ser Thr Ser
65                  70                  75                  80
aca gcc tac atg gag ctg agc agc ctg aga tct gag gac acg gcc gtg      288

Thr Ala Tyr Met Glu Leu Ser Ser Leu Arg Ser Glu Asp Thr Ala Val
                85                  90                  95
tat tac tgt gcg aga tac cag agt agt gac tat tac aac tct gaa tac 336
Tyr Tyr Cys Ala Arg Tyr Gln Ser Ser Asp Tyr Tyr Asn Ser Glu Tyr
            100                 105                 110
ttc caa cac tgg ggc cag ggc acc ctg gtc acc gtc tcg agc 378
Phe Gln His Trp Gly Gln Gly Thr Leu Val Thr Val Ser Ser
        115                 120                 125
<210> 184
<211> 126
<212> PRT
<213> human
<400> 184
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Ser Phe
            20                  25                  30
Ser Thr Tyr Thr Ile Ser Trp Val Arg Gln Ala Pro Gly Gln Gly Leu
        35                  40                  45
Glu Trp Met Gly Arg Ser Ile Pro Leu Leu Gly Ile Thr Asn Tyr Ala
    50                  55                  60
Gln Lys Phe Gln Gly Arg Val Thr Ile Thr Ala Asp Ile Ser Thr Ser
65                  70                  75                  80
Thr Ala Tyr Met Glu Leu Ser Ser Leu Arg Ser Glu Asp Thr Ala Val
                85                  90                  95
Tyr Tyr Cys Ala Arg Tyr Gln Ser Ser Asp Tyr Tyr Asn Ser Glu Tyr
            100                 105                 110
Phe Gln His Trp Gly Gln Gly Thr Leu Val Thr Val Ser Ser
        115                 120                 125
<210> 185
<211> 324
<212> DNA
<213> human
<220>
<221> CDS
<222> (1)..(324)
<400> 185
gaa att gtg ttg acg cag tct cca ggc acc ctg tct ttg tct cta ggg 48
Glu Ile Val Leu Thr Gln Ser Pro Gly Thr Leu Ser Leu Ser Leu Gly
1               5                   10                  15
gaa aga gtc acc ctc tcc tgc agg gcc agt cag aat gtt ggc acc aat 96
Glu Arg Val Thr Leu Ser Cys Arg Ala Ser Gln Asn Val Gly Thr Asn
            20                  25                  30
tac tta gcc tgg tac cag cag aaa cct ggc cag gct ccc agg ctc ctc 144
Tyr Leu Ala Trp Tyr Gln Gln Lys Pro Gly Gln Ala Pro Arg Leu Leu
        35                  40                  45
atc tat ggt gca tcc agc agg gcc act ggc gtc cca gac agg ttc agt 192
Ile Tyr Gly Ala Ser Ser Arg Ala Thr Gly Val Pro Asp Arg Phe Ser
    50                  55                  60
ggc agt ggg tct ggg aca gac ttc act ctc acc atc agc aga ctg gcg 240
Gly Ser Gly Ser Gly Thr Asp Phe Thr Leu Thr Ile Ser Arg Leu Ala
65                  70                  75                  80
cct gaa gat ttt gca gtg tac tac tgt cag cag tat agt agt tca cct 288
Pro Glu Asp Phe Ala Val Tyr Tyr Cys Gln Gln Tyr Ser Ser Ser Pro
                85                  90                  95
ctc act ttc ggc gga ggg acc aaa ctg gag atc aag 324
Leu Thr Phe Gly Gly Gly Thr Lys Leu Glu Ile Lys
            100                 105
<210> 186
<211> 108
<212> PRT
<213> human
<400> 186

Glu Ile Val Leu Thr Gln Ser Pro Gly Thr Leu Ser Leu Ser Leu Gly
1               5                   10                  15
Glu Arg Val Thr Leu Ser Cys Arg Ala Ser Gln Asn Val Gly Thr Asn
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Val Pro Asp Arg Phe Ser
    50                  55                  60
Gly Ser Gly Ser Gly Thr Asp Phe Thr Leu Thr Ile Ser Arg Leu Ala
65                  70                  75                  80
Pro Glu Asp Phe Ala Val Tyr Tyr Cys Gln Gln Tyr Ser Ser Ser Pro
                85                  90                  95
Leu Thr Phe Gly Gly Gly Thr Lys Leu Glu Ile Lys
            100                 105
<210>  187
<211>  360
<212>  DNA
<213>  human
<220>
<221>  CDS
<222>  (1)..(360)
<400>  187
cag gtg cag ctg gtg cag tct ggg gcc gag gtg aag aag cct ggg tcc       48
Gln Val Gln Leu Val Gln Ser Gly Ala Glu Val Lys Lys Pro Gly Ser
1               5                   10                  15
tcg gtg aag gtc tcc tgc aag gcc tct gga ggc att ttc cgc aaa aat       96
Ser Val Lys Val Ser Cys Lys Ala Ser Gly Gly Ile Phe Arg Lys Asn
            20                  25                  30
gcc atc agc tac gtg cgc cag gcc cct gga caa ggg gtt gag tgg atg      144
Ala Ile Ser Tyr Val Arg Gln Ala Pro Gly Gln Gly Val Glu Trp Met
        35                  40                  45
gga ggg atc acc gct atc ttt ggc aca cca aac tac gca cag aag ttc      192
Gly Gly Ile Thr Ala Ile Phe Gly Thr Pro Asn Tyr Ala Gln Lys Phe
    50                  55                  60
cag ggc aga atc acc att agc gcg gac gaa tcc acg aac aca gtc tac      240
Gln Gly Arg Ile Thr Ile Ser Ala Asp Glu Ser Thr Asn Thr Val Tyr
65                  70                  75                  80
atg gaa ctg ggc agt ctg aca tct gag gac acg gcc gtt tat tat tgt      288
Met Glu Leu Gly Ser Leu Thr Ser Glu Asp Thr Ala Val Tyr Tyr Cys
                85                  90                  95
gcg acc agt ggg acc tac tac gtc tcc tac ctt gac tcc tgg ggc cag      336
Ala Thr Ser Gly Thr Tyr Tyr Val Ser Tyr Leu Asp Ser Trp Gly Gln
            100                 105                 110
gga acc ctg gtc acc gtc tcg agc                                      360
Gly Thr Leu Val Thr Val Ser Ser
        115                 120
<210>  188
<211>  120
<212>  PRT
<213>  human
<400>  188
Gln Val Gln Leu Val Gln Ser Gly Ala Glu Val Lys Lys Pro Gly Ser
1               5                   10                  15
Ser Val Lys Val Ser Cys Lys Ala Ser Gly Gly Ile Phe Arg Lys Asn
            20                  25                  30
Ala Ile Ser Tyr Val Arg Gln Ala Pro Gly Gln Gly Val Glu Trp Met
        35                  40                  45
Gly Gly Ile Thr Ala Ile Phe Gly Thr Pro Asn Tyr Ala Gln Lys Phe
    50                  55                  60
Gln Gly Arg Ile Thr Ile Ser Ala Asp Glu Ser Thr Asn Thr Val Tyr
65                  70                  75                  80
Met Glu Leu Gly Ser Leu Thr Ser Glu Asp Thr Ala Val Tyr Tyr Cys
                85                  90                  95

Ala Thr Ser Gly Thr Tyr Tyr Val Ser Tyr Leu Asp Ser Trp Gly Gln
            100                 105                 110
Gly Thr Leu Val Thr Val Ser Ser
        115                 120
<210> 189
<211> 324
<212> DNA
<213> human
<220>
<221> CDS
<222> (1)..(324)
<400> 189
gat gtt gtg atg act cag tct cca ggc acc ctg tct ttg tct cca ggg 48
Asp Val Val Met Thr Gln Ser Pro Gly Thr Leu Ser Leu Ser Pro Gly
1               5                   10                  15
gaa aga gcc acc ctc tcc tgc agg gcc agt cag agt gtt agc agc agc 96
Glu Arg Ala Thr Leu Ser Cys Arg Ala Ser Gln Ser Val Ser Ser Ser
            20                  25                  30
tac tta gcc tgg tac cag cag aaa cct ggc cag gct ccc agg ctc ctc 144
Tyr Leu Ala Trp Tyr Gln Gln Lys Pro Gly Gln Ala Pro Arg Leu Leu
        35                  40                  45
atc tat ggt gca tcc agc agg gcc act ggc atc cca gac agg ttc agt 192
Ile Tyr Gly Ala Ser Ser Arg Ala Thr Gly Ile Pro Asp Arg Phe Ser
    50                  55                  60
ggc agt ggg tct ggg aca gac ttc act ctc acc atc agc aga ctg gag 240
Gly Ser Gly Ser Gly Thr Asp Phe Thr Leu Thr Ile Ser Arg Leu Glu
65                  70                  75                  80
cct gaa gat ttt gca gtg tat tac tgt cag cag tat ggt agc tca cct 288
Pro Glu Asp Phe Ala Val Tyr Tyr Cys Gln Gln Tyr Gly Ser Ser Pro
                85                  90                  95
ctc act ttc ggc cct ggg acc aaa gtg gat atc aaa 324
Leu Thr Phe Gly Pro Gly Thr Lys Val Asp Ile Lys
            100                 105
<210> 190
<211> 108
<212> PRT
<213> human
<400> 190
Asp Val Val Met Thr Gln Ser Pro Gly Thr Leu Ser Leu Ser Pro Gly
1               5                   10                  15
Glu Arg Ala Thr Leu Ser Cys Arg Ala Ser Gln Ser Val Ser Ser Ser
            20                  25                  30
Tyr Leu Ala Trp Tyr Gln Gln Lys Pro Gly Gln Ala Pro Arg Leu Leu
        35                  40                  45
Ile Tyr Gly Ala Ser Ser Arg Ala Thr Gly Ile Pro Asp Arg Phe Ser
    50                  55                  60
Gly Ser Gly Ser Gly Thr Asp Phe Thr Leu Thr Ile Ser Arg Leu Glu
65                  70                  75                  80
Pro Glu Asp Phe Ala Val Tyr Tyr Cys Gln Gln Tyr Gly Ser Ser Pro
                85                  90                  95
Leu Thr Phe Gly Pro Gly Thr Lys Val Asp Ile Lys
            100                 105
<210> 191
<211> 360
<212> DNA
<213> human
<220>
<221> CDS
<222> (1)..(360)
<400> 191
gag gtg cag ctg gtg gag tct ggg gcc gag gtg aag aag cct ggg tcc 48
Glu Val Gln Leu Val Glu Ser Gly Ala Glu Val Lys Lys Pro Gly Ser
1               5                   10                  15

tcg gtg aag gtc tcc tgc aag gct tct gga ggc cac ttc aac gcc tat 96
Ser Val Lys Val Ser Cys Lys Ala Ser Gly Gly His Phe Asn Ala Tyr
            20                  25                  30
gga atc agc tgg gtg cgg cag gcc cca gga caa ggc ctt gag tgg atg 144
Gly Ile Ser Trp Val Arg Gln Ala Pro Gly Gln Gly Leu Glu Trp Met
        35                  40                  45
gga ggg att gtc gcc ctc ttt gga aca aca aac tac gca cag aag ttg 192
Gly Gly Ile Val Ala Leu Phe Gly Thr Thr Asn Tyr Ala Gln Lys Leu
    50                  55                  60
cag ggc aga gtc aca att acc gcg gac gcg tcc acg aac aca gtg tac 240
Gln Gly Arg Val Thr Ile Thr Ala Asp Ala Ser Thr Asn Thr Val Tyr
65                  70                  75                  80
atg gag ttg acc agc ctg aga tct gac gac acg gcc gta tat tac tgt 288
Met Glu Leu Thr Ser Leu Arg Ser Asp Asp Thr Ala Val Tyr Tyr Cys
                85                  90                  95
gcg aga agc ggg agt tat tac cct gat tac ttc caa tat tgg ggc cag 336
Ala Arg Ser Gly Ser Tyr Tyr Pro Asp Tyr Phe Gln Tyr Trp Gly Gln
            100                 105                 110
gga acc ctg gtc acc gtc tcg agc 360
Gly Thr Leu Val Thr Val Ser Ser
        115                 120
<210> 192
<211> 120
<212> PRT
<213> human
<400> 192
Glu Val Gln Leu Val Glu Ser Gly Ala Glu Val Lys Lys Pro Gly Ser
1               5                   10                  15
Ser Val Lys Val Ser Cys Lys Ala Ser Gly Gly His Phe Asn Ala Tyr
            20                  25                  30
Gly Ile Ser Trp Val Arg Gln Ala Pro Gly Gln Gly Leu Glu Trp Met
        35                  40                  45
Gly Gly Ile Val Ala Leu Phe Gly Thr Thr Asn Tyr Ala Gln Lys Leu
    50                  55                  60
Gln Gly Arg Val Thr Ile Thr Ala Asp Ala Ser Thr Asn Thr Val Tyr
65                  70                  75                  80
Met Glu Leu Thr Ser Leu Arg Ser Asp Asp Thr Ala Val Tyr Tyr Cys
                85                  90                  95
Ala Arg Ser Gly Ser Tyr Tyr Pro Asp Tyr Phe Gln Tyr Trp Gly Gln
            100                 105                 110
Gly Thr Leu Val Thr Val Ser Ser
        115                 120
<210> 193
<211> 324
<212> DNA
<213> human
<220>
<221> CDS
<222> (1)..(324)
<400> 193
gaa att gtg ttg acg cag tct cca ggc acc ctg tct ttg tct cca ggg 48
Glu Ile Val Leu Thr Gln Ser Pro Gly Thr Leu Ser Leu Ser Pro Gly
1               5                   10                  15
gaa aga gcc acc ctc tcc tgc agg gcc agt cag agt gtt agc agc agc 96
Glu Arg Ala Thr Leu Ser Cys Arg Ala Ser Gln Ser Val Ser Ser Ser
            20                  25                  30
tac tta gcc tgg tac cag cag aaa cct ggc cag gct ccc agg ctc ctc 144
Tyr Leu Ala Trp Tyr Gln Gln Lys Pro Gly Gln Ala Pro Arg Leu Leu
        35                  40                  45
atc cat ggt gca tcc agc agg gcc act ggc atc cca gac agg ttc agt 192
Ile His Gly Ala Ser Ser Arg Ala Thr Gly Ile Pro Asp Arg Phe Ser
    50                  55                  60
ggc agt ggg tct ggg aca gac ttc act ctc tcc atc agc aga ctg gag 240

Gly Ser Gly Ser Gly Thr Asp Phe Thr Leu Ser Ile Ser Arg Leu Glu
65                  70                  75                  80
cct gaa gat ttt gca gtg tat tac tgt cag cag tat ggt agc tca cct       288
Pro Glu Asp Phe Ala Val Tyr Tyr Cys Gln Gln Tyr Gly Ser Ser Pro
                85                  90                  95
cgg acg ttc ggc caa ggg acc aag gtg gaa atc aaa                       324
Arg Thr Phe Gly Gln Gly Thr Lys Val Glu Ile Lys
            100                 105
<210>  194
<211>  108
<212>  PRT
<213>  human
<400>  194
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Ser Val Ser Ser Ser
            20                  25                  30
Tyr Leu Ala Trp Tyr Gln Gln Lys Pro Gly Gln Ala Pro Arg Leu Leu
        35                  40                  45
Ile His Gly Ala Ser Ser Arg Ala Thr Gly Ile Pro Asp Arg Phe Ser
    50                  55                  60
Gly Ser Gly Ser Gly Thr Asp Phe Thr Leu Ser Ile Ser Arg Leu Glu
65                  70                  75                  80
Pro Glu Asp Phe Ala Val Tyr Tyr Cys Gln Gln Tyr Gly Ser Ser Pro
                85                  90                  95
Arg Thr Phe Gly Gln Gly Thr Lys Val Glu Ile Lys
            100                 105
<210>  195
<211>  366
<212>  DNA
<213>  human
<220>
<221>  CDS
<222>  (1)..(366)
<400>  195
cag gtg cag ctg gtg caa tct ggg tct gag gtg aag aag cct ggg tcc        48
Gln Val Gln Leu Val Gln Ser Gly Ser Glu Val Lys Lys Pro Gly Ser
1               5                   10                  15
tcg gtg aag gtc tcc tgc tcc ttc aag gca tct gga ggc atc atc agc        96
Ser Val Lys Val Ser Cys Ser Phe Lys Ala Ser Gly Gly Ile Ile Ser
            20                  25                  30
aag tat gcc atc acc tgg gtg cga cag gcc cct gga caa ggg ctt gag       144
Lys Tyr Ala Ile Thr Trp Val Arg Gln Ala Pro Gly Gln Gly Leu Glu
        35                  40                  45
tgg atg gga ggg atc atc gct atc ttt ggt tca aca aac tac gca cag       192
Trp Met Gly Gly Ile Ile Ala Ile Phe Gly Ser Thr Asn Tyr Ala Gln
    50                  55                  60
aag ttc cag ggc aga ctc acg att acc tcg gac gaa tcc acg agc aca       240
Lys Phe Gln Gly Arg Leu Thr Ile Thr Ser Asp Glu Ser Thr Ser Thr
65                  70                  75                  80
gcc tat atg gaa ctg agc agc ctg aca tct gaa gac acg gcc gtg tat       288
Ala Tyr Met Glu Leu Ser Ser Leu Thr Ser Glu Asp Thr Ala Val Tyr
                85                  90                  95
tat tgt ggg aga gga cca cat tac tat gag agc cac ctt gac tac tgg       336
Tyr Cys Gly Arg Gly Pro His Tyr Tyr Glu Ser His Leu Asp Tyr Trp
            100                 105                 110
ggc cag gga acc ctg gtc acc gtc tcg agc                               366
Gly Gln Gly Thr Leu Val Thr Val Ser Ser
        115                 120
<210>  196
<211>  122
<212>  PRT
<213>  human

<400>  196
Gln Val Gln Leu Val Gln Ser Gly Ser Glu Val Lys Lys Pro Gly Ser
1               5                   10                  15
Ser Val Lys Val Ser Cys Ser Phe Lys Ala Ser Gly Gly Ile Ile Ser
            20                  25                  30
Lys Tyr Ala Ile Thr Trp Val Arg Gln Ala Pro Gly Gln Gly Leu Glu
        35                  40                  45
Trp Met Gly Gly Ile Ile Ala Ile Phe Gly Ser Thr Asn Tyr Ala Gln
    50                  55                  60
Lys Phe Gln Gly Arg Leu Thr Ile Thr Ser Asp Glu Ser Thr Ser Thr
65                  70                  75                  80
Ala Tyr Met Glu Leu Ser Ser Leu Thr Ser Glu Asp Thr Ala Val Tyr
                85                  90                  95
Tyr Cys Gly Arg Gly Pro His Tyr Tyr Glu Ser His Leu Asp Tyr Trp
            100                 105                 110
Gly Gln Gly Thr Leu Val Thr Val Ser Ser
        115                 120
<210>  197
<211>  324
<212>  DNA
<213>  human
<220>
<221>  CDS
<222>  (1)..(324)
<400>  197
gaa att gtg ttg acg cag tct cca ggc acc ctg tct ttg tct cca ggg      48
Glu Ile Val Leu Thr Gln Ser Pro Gly Thr Leu Ser Leu Ser Pro Gly
1               5                   10                  15
gag aga gcc acc ctc tcc tgc agg gcc agt cag ggt atg agc agc agc      96
Glu Arg Ala Thr Leu Ser Cys Arg Ala Ser Gln Gly Met Ser Ser Ser
            20                  25                  30
ttc tta gcc tgg tac caa cag aag cct ggc cag cct ccc agg ctc ctc      144
Phe Leu Ala Trp Tyr Gln Gln Lys Pro Gly Gln Pro Pro Arg Leu Leu
        35                  40                  45
atc tct ggt gca tcc acc agg gcc act ggc atc cca gac agg ttc agt      192
Ile Ser Gly Ala Ser Thr Arg Ala Thr Gly Ile Pro Asp Arg Phe Ser
    50                  55                  60
ggc agt ggg tct ggg aca gac ttc act ctc acc atc aac aga ctg gag      240
Gly Ser Gly Ser Gly Thr Asp Phe Thr Leu Thr Ile Asn Arg Leu Glu
65                  70                  75                  80
cct gaa gat ttt gca gtg tat tac tgt cag cag tat ggt agc tca cct      288
Pro Glu Asp Phe Ala Val Tyr Tyr Cys Gln Gln Tyr Gly Ser Ser Pro
                85                  90                  95
cgg acg ttc ggc caa ggg acc aag gtg gaa atc aaa                      324
Arg Thr Phe Gly Gln Gly Thr Lys Val Glu Ile Lys
            100                 105
<210>  198
<211>  108
<212>  PRT
<213>  human
<400>  198
Glu Ile Val Leu Thr Gln Ser Pro Gly Thr Leu Ser Leu Ser Pro Gly
1               5                   10                  15
Glu Arg Ala Thr Leu Ser Cys Arg Ala Ser Gln Gly Met Ser Ser Ser
            20                  25                  30
Phe Leu Ala Trp Tyr Gln Gln Lys Pro Gly Gln Pro Pro Arg Leu Leu
        35                  40                  45
Ile Ser Gly Ala Ser Thr Arg Ala Thr Gly Ile Pro Asp Arg Phe Ser
    50                  55                  60
Gly Ser Gly Ser Gly Thr Asp Phe Thr Leu Thr Ile Asn Arg Leu Glu
65                  70                  75                  80
Pro Glu Asp Phe Ala Val Tyr Tyr Cys Gln Gln Tyr Gly Ser Ser Pro
                85                  90                  95

Arg Thr Phe Gly Gln Gly Thr Lys Val Glu Ile Lys
            100                 105
<210>  199
<211>  360
<212>  DNA
<213>  human
<220>
<221>  CDS
<222>  (1)..(360)
<400>  199
cag gtg cag ctg gtg caa tct ggg tct gag gtg aag aag cct ggg tcc        48
Gln Val Gln Leu Val Gln Ser Gly Ser Glu Val Lys Lys Pro Gly Ser
1               5                   10                  15
tcg gtg aag gtc tcc tgc aag tct tct gga ggc atc ctc agg agg tct        96
Ser Val Lys Val Ser Cys Lys Ser Ser Gly Gly Ile Leu Arg Arg Ser
            20                  25                  30
gcc atc agc tgg gtg cga cag gcc cct gga caa ggg ctt gag tgg atg       144
Ala Ile Ser Trp Val Arg Gln Ala Pro Gly Gln Gly Leu Glu Trp Met
        35                  40                  45
gga ggg atc ctc gct atc ttt ggt aca aca aag tac gca cag aag ttc       192
Gly Gly Ile Leu Ala Ile Phe Gly Thr Thr Lys Tyr Ala Gln Lys Phe
    50                  55                  60
cag ggc aga atc acc att acc gcg gac gaa tcc acg aat aca gtc cac       240
Gln Gly Arg Ile Thr Ile Thr Ala Asp Glu Ser Thr Asn Thr Val His
65                  70                  75                  80
atg gag ctg agc agc ctg aga cct gac gac acg gcc gtg tat tac tgt       288
Met Glu Leu Ser Ser Leu Arg Pro Asp Asp Thr Ala Val Tyr Tyr Cys
                85                  90                  95
gcg gga ggc cca cac tat tat gtt tcc tac ttt gac tcc tgg ggc cag       336
Ala Gly Gly Pro His Tyr Tyr Val Ser Tyr Phe Asp Ser Trp Gly Gln
            100                 105                 110
gga acc ctg gtc acc gtc tcg agc                                       360
Gly Thr Leu Val Thr Val Ser Ser
        115                 120
<210>  200
<211>  120
<212>  PRT
<213>  human
<400>  200
Gln Val Gln Leu Val Gln Ser Gly Ser Glu Val Lys Lys Pro Gly Ser
1               5                   10                  15
Ser Val Lys Val Ser Cys Lys Ser Ser Gly Gly Ile Leu Arg Arg Ser
            20                  25                  30
Ala Ile Ser Trp Val Arg Gln Ala Pro Gly Gln Gly Leu Glu Trp Met
        35                  40                  45
Gly Gly Ile Leu Ala Ile Phe Gly Thr Thr Lys Tyr Ala Gln Lys Phe
    50                  55                  60
Gln Gly Arg Ile Thr Ile Thr Ala Asp Glu Ser Thr Asn Thr Val His
65                  70                  75                  80
Met Glu Leu Ser Ser Leu Arg Pro Asp Asp Thr Ala Val Tyr Tyr Cys
                85                  90                  95
Ala Gly Gly Pro His Tyr Tyr Val Ser Tyr Phe Asp Ser Trp Gly Gln
            100                 105                 110
Gly Thr Leu Val Thr Val Ser Ser
        115                 120
<210>  201
<211>  321
<212>  DNA
<213>  human
<220>
<221>  CDS
<222>  (1)..(321)
<400>  201

gac atc cag atg acc cag tct cca gcc acc ctg tct ttg tct cca ggg 48
Asp Ile Gln Met Thr Gln Ser Pro Ala Thr Leu Ser Leu Ser Pro Gly
1               5                   10                  15
gag aga gcc acc ctc tcc tgc agg gcc agt cag agt gtt agg agc tac 96
Glu Arg Ala Thr Leu Ser Cys Arg Ala Ser Gln Ser Val Arg Ser Tyr
            20                  25                  30
tta gcc tgg tac caa cag aaa cct ggc cag gct ccc agg ctc ctc atc 144
Leu Ala Trp Tyr Gln Gln Lys Pro Gly Gln Ala Pro Arg Leu Leu Ile
        35                  40                  45
tat gat gca tcc aac agg gcc act ggc atc cca gcc agg ttc agc ggc 192
Tyr Asp Ala Ser Asn Arg Ala Thr Gly Ile Pro Ala Arg Phe Ser Gly
    50                  55                  60
agt ggg tct ggg aca gag ttc act ctc acc atc agc cgc cta gag cct 240
Ser Gly Ser Gly Thr Glu Phe Thr Leu Thr Ile Ser Arg Leu Glu Pro
65                  70                  75                  80
gaa gat ttt gca gtt tat tac tgt cag caa cgt agt aac tgg tta ttc 288
Glu Asp Phe Ala Val Tyr Tyr Cys Gln Gln Arg Ser Asn Trp Leu Phe
                85                  90                  95
act ttc ggc cct ggg acc aaa gtg gat ttc aaa 321
Thr Phe Gly Pro Gly Thr Lys Val Asp Phe Lys
            100                 105
<210> 202
<211> 107
<212> PRT
<213> human
<400> 202
Asp Ile Gln Met Thr Gln Ser Pro Ala Thr Leu Ser Leu Ser Pro Gly
1               5                   10                  15
Glu Arg Ala Thr Leu Ser Cys Arg Ala Ser Gln Ser Val Arg Ser Tyr
            20                  25                  30
Leu Ala Trp Tyr Gln Gln Lys Pro Gly Gln Ala Pro Arg Leu Leu Ile
        35                  40                  45
Tyr Asp Ala Ser Asn Arg Ala Thr Gly Ile Pro Ala Arg Phe Ser Gly
    50                  55                  60
Ser Gly Ser Gly Thr Glu Phe Thr Leu Thr Ile Ser Arg Leu Glu Pro
65                  70                  75                  80
Glu Asp Phe Ala Val Tyr Tyr Cys Gln Gln Arg Ser Asn Trp Leu Phe
                85                  90                  95
Thr Phe Gly Pro Gly Thr Lys Val Asp Phe Lys
            100                 105
<210> 203
<211> 360
<212> DNA
<213> human
<220>
<221> CDS
<222> (1)..(360)
<400> 203
cag gtg cag ctg gtg cag tct ggg gct gag gtg aag aag cct ggg tcc 48
Gln Val Gln Leu Val Gln Ser Gly Ala Glu Val Lys Lys Pro Gly Ser
1               5                   10                  15
tcg gtg aag gtc tcc tgc aag gct tct gga ggc atc ttc agg agc aat 96
Ser Val Lys Val Ser Cys Lys Ala Ser Gly Gly Ile Phe Arg Ser Asn
            20                  25                  30
gct gtc agc tgg gtg cga cag gcc cct ggc caa ggg ctt gag tgg atg 144
Ala Val Ser Trp Val Arg Gln Ala Pro Gly Gln Gly Leu Glu Trp Met
        35                  40                  45
gga ggg atc atc gct atc ttt ggt aca cca aag tat gca cag aag ttc 192
Gly Gly Ile Ile Ala Ile Phe Gly Thr Pro Lys Tyr Ala Gln Lys Phe
    50                  55                  60
cag ggc aga gtc acc att acc gcg gac gaa tcc acg agc aca gtt tac 240
Gln Gly Arg Val Thr Ile Thr Ala Asp Glu Ser Thr Ser Thr Val Tyr
65                  70                  75                  80

atg gag ttg agc ggt ctg aga tat gag gac acg gcc gtc tat tac tgt 288
Met Glu Leu Ser Gly Leu Arg Tyr Glu Asp Thr Ala Val Tyr Tyr Cys
                85                  90                  95
gcg aga ggg ccg aat tac tat gaa agt tac ttc gac tat tgg ggc cag 336
Ala Arg Gly Pro Asn Tyr Tyr Glu Ser Tyr Phe Asp Tyr Trp Gly Gln
            100                 105                 110
gga acc ctg gtc acc gtc tcg agc 360
Gly Thr Leu Val Thr Val Ser Ser
        115                 120
<210> 204
<211> 120
<212> PRT
<213> human
<400> 204
Gln Val Gln Leu Val Gln Ser Gly Ala Glu Val Lys Lys Pro Gly Ser
1               5                   10                  15
Ser Val Lys Val Ser Cys Lys Ala Ser Gly Gly Ile Phe Arg Ser Asn
            20                  25                  30
Ala Val Ser Trp Val Arg Gln Ala Pro Gly Gln Gly Leu Glu Trp Met
        35                  40                  45
Gly Gly Ile Ile Ala Ile Phe Gly Thr Pro Lys Tyr Ala Gln Lys Phe
    50                  55                  60
Gln Gly Arg Val Thr Ile Thr Ala Asp Glu Ser Thr Ser Thr Val Tyr
65                  70                  75                  80
Met Glu Leu Ser Gly Leu Arg Tyr Glu Asp Thr Ala Val Tyr Tyr Cys
                85                  90                  95
Ala Arg Gly Pro Asn Tyr Tyr Glu Ser Tyr Phe Asp Tyr Trp Gly Gln
            100                 105                 110
Gly Thr Leu Val Thr Val Ser Ser
        115                 120
<210> 205
<211> 321
<212> DNA
<213> human
<220>
<221> CDS
<222> (1)..(321)
<400> 205
gac atc gtg atg acc cag tct cca ccc tcc ctg tct gca tct gta gga 48
Asp Ile Val Met Thr Gln Ser Pro Pro Ser Leu Ser Ala Ser Val Gly
1               5                   10                  15
gac aga gtc acc atc act tgc cgg gcg agt cag ggc atc agt aat tat 96
Asp Arg Val Thr Ile Thr Cys Arg Ala Ser Gln Gly Ile Ser Asn Tyr
            20                  25                  30
tta gcc tgg tat cag cag aaa cca ggg aaa gct ccc aag ctc ctg atc 144
Leu Ala Trp Tyr Gln Gln Lys Pro Gly Lys Ala Pro Lys Leu Leu Ile
        35                  40                  45
ttt ggt gca tct act ttg cac tca ggg gtc tcg tct cgg ttc agt ggc 192
Phe Gly Ala Ser Thr Leu His Ser Gly Val Ser Ser Arg Phe Ser Gly
    50                  55                  60
agc gga tct ggg cca gat ttc act ctc acc atc agc agt ctg cag cct 240
Ser Gly Ser Gly Pro Asp Phe Thr Leu Thr Ile Ser Ser Leu Gln Pro
65                  70                  75                  80
gaa gat gct gca act tat tac tgt caa aag tat cac agt gcc cct ctc 288
Glu Asp Ala Ala Thr Tyr Tyr Cys Gln Lys Tyr His Ser Ala Pro Leu
                85                  90                  95
act ttc ggc gga ggg acc aag gtg gag atg aaa 321
Thr Phe Gly Gly Gly Thr Lys Val Glu Met Lys
            100                 105
<210> 206
<211> 107
<212> PRT
<213> human

<400>  206
Asp Ile Val Met Thr Gln Ser Pro Pro Ser Leu Ser Ala Ser Val Gly
1               5                   10                  15
Asp Arg Val Thr Ile Thr Cys Arg Ala Ser Gln Gly Ile Ser Asn Tyr
            20                  25                  30
Leu Ala Trp Tyr Gln Gln Lys Pro Gly Lys Ala Pro Lys Leu Leu Ile
        35                  40                  45
Phe Gly Ala Ser Thr Leu His Ser Gly Val Ser Ser Arg Phe Ser Gly
    50                  55                  60
Ser Gly Ser Gly Pro Asp Phe Thr Leu Thr Ile Ser Ser Leu Gln Pro
65                  70                  75                  80
Glu Asp Ala Ala Thr Tyr Tyr Cys Gln Lys Tyr His Ser Ala Pro Leu
                85                  90                  95
Thr Phe Gly Gly Gly Thr Lys Val Glu Met Lys
            100                 105
<210>  207
<211>  360
<212>  DNA
<213>  human
<220>
<221>  CDS
<222>  (1)..(360)
<400>  207
cag gtg cag ctg gtg cag tct ggg gct gag gtg aag aag cct ggg tcc      48
Gln Val Gln Leu Val Gln Ser Gly Ala Glu Val Lys Lys Pro Gly Ser
1               5                   10                  15
tcg gtg aag gtc tcc tgc aag gct tct gga ggc atc ttc aac aag tat      96
Ser Val Lys Val Ser Cys Lys Ala Ser Gly Gly Ile Phe Asn Lys Tyr
            20                  25                  30
ggt atc agc tgg gtg cga cag gcc cct gga caa ggg ctt gag tgg atg      144
Gly Ile Ser Trp Val Arg Gln Ala Pro Gly Gln Gly Leu Glu Trp Met
        35                  40                  45
ggg ggg atc ctc gca att ttc gga aca aca aac tac gca cag aag ttc      192
Gly Gly Ile Leu Ala Ile Phe Gly Thr Thr Asn Tyr Ala Gln Lys Phe
    50                  55                  60
cag ggc aga gtc acg att agc gcg gac gag tcc tcg act aca gtg tac      240
Gln Gly Arg Val Thr Ile Ser Ala Asp Glu Ser Ser Thr Thr Val Tyr
65                  70                  75                  80
atg gag ctg agc agc ctg acg tct gag gac acg gcc gtg tat tat tgt      288
Met Glu Leu Ser Ser Leu Thr Ser Glu Asp Thr Ala Val Tyr Tyr Cys
                85                  90                  95
gcg agg ggg aat act tat tac tcc agt tac ttt gac cag tgg ggc cag      336
Ala Arg Gly Asn Thr Tyr Tyr Ser Ser Tyr Phe Asp Gln Trp Gly Gln
            100                 105                 110
ggc acc ctg gtc acc gtc tcg agc                                      360
Gly Thr Leu Val Thr Val Ser Ser
        115                 120
<210>  208
<211>  120
<212>  PRT
<213>  human
<400>  208
Gln Val Gln Leu Val Gln Ser Gly Ala Glu Val Lys Lys Pro Gly Ser
1               5                   10                  15
Ser Val Lys Val Ser Cys Lys Ala Ser Gly Gly Ile Phe Asn Lys Tyr
            20                  25                  30
Gly Ile Ser Trp Val Arg Gln Ala Pro Gly Gln Gly Leu Glu Trp Met
        35                  40                  45
Gly Gly Ile Leu Ala Ile Phe Gly Thr Thr Asn Tyr Ala Gln Lys Phe
    50                  55                  60
Gln Gly Arg Val Thr Ile Ser Ala Asp Glu Ser Ser Thr Thr Val Tyr
65                  70                  75                  80
Met Glu Leu Ser Ser Leu Thr Ser Glu Asp Thr Ala Val Tyr Tyr Cys

85                  90                  95
Ala Arg Gly Asn Thr Tyr Tyr Ser Ser Tyr Phe Asp Gln Trp Gly Gln
            100                 105                 110
Gly Thr Leu Val Thr Val Ser Ser
        115                 120
<210>  209
<211>  330
<212>  DNA
<213>  human
<220>
<221>  CDS
<222>  (1)..(330)
<400>  209
cag tct gtg ttg acg cag ccg ccc tcg gtg tca gtg gcc cca ggt caa      48
Gln Ser Val Leu Thr Gln Pro Pro Ser Val Ser Val Ala Pro Gly Gln
1               5                   10                  15
acg gcc acc atc acc tgt ggg gga gac aag att ggg act gga cct gtg      96
Thr Ala Thr Ile Thr Cys Gly Gly Asp Lys Ile Gly Thr Gly Pro Val
            20                  25                  30
cat tgg tat cag cag atg cca ggc cgg gcc cct gtc ctg ctc att tat      144
His Trp Tyr Gln Gln Met Pro Gly Arg Ala Pro Val Leu Leu Ile Tyr
        35                  40                  45
gcc agc act cat cgg ccc tca agg atc cct gag cgg ttc tct ggc tcc      192
Ala Ser Thr His Arg Pro Ser Arg Ile Pro Glu Arg Phe Ser Gly Ser
    50                  55                  60
aag tct gac aat acg gcc acc ctc tcc atc agc ggg gtc gga gtc ggg      240
Lys Ser Asp Asn Thr Ala Thr Leu Ser Ile Ser Gly Val Gly Val Gly
65                  70                  75                  80
gat gag gcc gac tat tac tgt cag atg tgg gac agt tcc tct gtc cat      288
Asp Glu Ala Asp Tyr Tyr Cys Gln Met Trp Asp Ser Ser Ser Val His
                85                  90                  95
ccg att gtt ttc ggc ggg ggg acc aag ctg acc gtc ctc agt              330
Pro Ile Val Phe Gly Gly Gly Thr Lys Leu Thr Val Leu Ser
            100                 105                 110
<210>  210
<211>  110
<212>  PRT
<213>  human
<400>  210
Gln Ser Val Leu Thr Gln Pro Pro Ser Val Ser Val Ala Pro Gly Gln
1               5                   10                  15
Thr Ala Thr Ile Thr Cys Gly Gly Asp Lys Ile Gly Thr Gly Pro Val
            20                  25                  30
His Trp Tyr Gln Gln Met Pro Gly Arg Ala Pro Val Leu Leu Ile Tyr
        35                  40                  45
Ala Ser Thr His Arg Pro Ser Arg Ile Pro Glu Arg Phe Ser Gly Ser
    50                  55                  60
Lys Ser Asp Asn Thr Ala Thr Leu Ser Ile Ser Gly Val Gly Val Gly
65                  70                  75                  80
Asp Glu Ala Asp Tyr Tyr Cys Gln Met Trp Asp Ser Ser Ser Val His
                85                  90                  95
Pro Ile Val Phe Gly Gly Gly Thr Lys Leu Thr Val Leu Ser
            100                 105                 110
<210>  211
<211>  360
<212>  DNA
<213>  human
<220>
<221>  CDS
<222>  (1)..(360)
<400>  211
cag gta cag ctg cag cag tca ggg gct gag gtg aag aag cct ggg tcc      48
Gln Val Gln Leu Gln Gln Ser Gly Ala Glu Val Lys Lys Pro Gly Ser

1               5                   10                  15
tcg gtg aag gtc tcc tgc aag gct tct gga ggc atc ttc agg aag aat          96
Ser Val Lys Val Ser Cys Lys Ala Ser Gly Gly Ile Phe Arg Lys Asn
            20                  25                  30
gct atc agc tgg gtg cga cag gcc ccc gga caa ggg ctt gag tgg atg          144
Ala Ile Ser Trp Val Arg Gln Ala Pro Gly Gln Gly Leu Glu Trp Met
        35                  40                  45
gga ggg atc atc gct atc ttt ggt aca gca aac tac gca cag aag ttc          192
Gly Gly Ile Ile Ala Ile Phe Gly Thr Ala Asn Tyr Ala Gln Lys Phe
    50                  55                  60
cag ggc aga gtc acg att acc gcg gac gat tca acg aac aca gtc tac          240
Gln Gly Arg Val Thr Ile Thr Ala Asp Asp Ser Thr Asn Thr Val Tyr
65                  70                  75                  80
atg gag ctg acc agc ctg aga tat gag gac acg gcc gtg tat tac tgt          288
Met Glu Leu Thr Ser Leu Arg Tyr Glu Asp Thr Ala Val Tyr Tyr Cys
                85                  90                  95
gcg aga ggg ccg aat tac tat gaa aat tac ttt gac ttc tgg ggc cag          336
Ala Arg Gly Pro Asn Tyr Tyr Glu Asn Tyr Phe Asp Phe Trp Gly Gln
            100                 105                 110
ggc acc ctg gtc acc gtc tcg agc                                          360
Gly Thr Leu Val Thr Val Ser Ser
        115                 120
<210>  212
<211>  120
<212>  PRT
<213>  human
<400>  212
Gln Val Gln Leu Gln Gln Ser Gly Ala Glu Val Lys Lys Pro Gly Ser
1               5                   10                  15
Ser Val Lys Val Ser Cys Lys Ala Ser Gly Gly Ile Phe Arg Lys Asn
            20                  25                  30
Ala Ile Ser Trp Val Arg Gln Ala Pro Gly Gln Gly Leu Glu Trp Met
        35                  40                  45
Gly Gly Ile Ile Ala Ile Phe Gly Thr Ala Asn Tyr Ala Gln Lys Phe
    50                  55                  60
Gln Gly Arg Val Thr Ile Thr Ala Asp Asp Ser Thr Asn Thr Val Tyr
65                  70                  75                  80
Met Glu Leu Thr Ser Leu Arg Tyr Glu Asp Thr Ala Val Tyr Tyr Cys
                85                  90                  95
Ala Arg Gly Pro Asn Tyr Tyr Glu Asn Tyr Phe Asp Phe Trp Gly Gln
            100                 105                 110
Gly Thr Leu Val Thr Val Ser Ser
        115                 120
<210>  213
<211>  321
<212>  DNA
<213>  human
<220>
<221>  CDS
<222>  (1)..(321)
<400>  213
gaa att gtg ttg acg cag tct cca tcc tcc ctg tct gca tct ttg aga          48
Glu Ile Val Leu Thr Gln Ser Pro Ser Ser Leu Ser Ala Ser Leu Arg
1               5                   10                  15
gac act gtc acc atc act tgc cgg acg agc cag gcc att acc aat tat          96
Asp Thr Val Thr Ile Thr Cys Arg Thr Ser Gln Ala Ile Thr Asn Tyr
            20                  25                  30
tta gcc tgg tat caa cag agg cca ggt aaa gct cct aaa ctc ctg atc          144
Leu Ala Trp Tyr Gln Gln Arg Pro Gly Lys Ala Pro Lys Leu Leu Ile
        35                  40                  45
tat gct gca tcc gtt ttg caa tca ggg gtc cca tct cgt ttc agt ggc          192
Tyr Ala Ala Ser Val Leu Gln Ser Gly Val Pro Ser Arg Phe Ser Gly
    50                  55                  60

agt gga tct ggg aca gat ttc act ctc acc atc agc agc ctg cag cct     240
Ser Gly Ser Gly Thr Asp Phe Thr Leu Thr Ile Ser Ser Leu Gln Pro
65                  70                  75                  80
gaa gat ttt gca act tat tac tgt caa aag tat gac agt gcc ccg tac     288
Glu Asp Phe Ala Thr Tyr Tyr Cys Gln Lys Tyr Asp Ser Ala Pro Tyr
                85                  90                  95
act ttt ggc cag ggg acc aag ctg gag atc aaa                         321
Thr Phe Gly Gln Gly Thr Lys Leu Glu Ile Lys
            100                 105
<210>  214
<211>  107
<212>  PRT
<213>  human
<400>  214
Glu Ile Val Leu Thr Gln Ser Pro Ser Ser Leu Ser Ala Ser Leu Arg
1               5                   10                  15
Asp Thr Val Thr Ile Thr Cys Arg Thr Ser Gln Ala Ile Thr Asn Tyr
            20                  25                  30
Leu Ala Trp Tyr Gln Gln Arg Pro Gly Lys Ala Pro Lys Leu Leu Ile
        35                  40                  45
Tyr Ala Ala Ser Val Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Lys Tyr Asp Ser Ala Pro Tyr
                85                  90                  95
Thr Phe Gly Gln Gly Thr Lys Leu Glu Ile Lys
            100                 105
<210>  215
<211>  366
<212>  DNA
<213>  human
<220>
<221>  CDS
<222>  (1)..(366)
<400>  215
cag gtg cag ctg cag gag tcg ggg gct gag gtg aag aag cct ggg tcc      48
Gln Val Gln Leu Gln Glu Ser Gly Ala Glu Val Lys Lys Pro Gly Ser
1               5                   10                  15
tcg gtg aag gtc tcc tgc aag gct tct gga gga ccc ttc agg agc cat      96
Ser Val Lys Val Ser Cys Lys Ala Ser Gly Gly Pro Phe Arg Ser His
            20                  25                  30
gct att agc tgg gtg cga cag gcc gct gga caa ggg ctt gag tgg atg     144
Ala Ile Ser Trp Val Arg Gln Ala Ala Gly Gln Gly Leu Glu Trp Met
        35                  40                  45
gga ggg atc acc gct gtc ttt ggt aca gca aac tac gca cag aag ttc     192
Gly Gly Ile Thr Ala Val Phe Gly Thr Ala Asn Tyr Ala Gln Lys Phe
    50                  55                  60
cag ggc aga gtc acg ata acc gcg gac gaa tcc acg aac ata gtc tcc     240
Gln Gly Arg Val Thr Ile Thr Ala Asp Glu Ser Thr Asn Ile Val Ser
65                  70                  75                  80
atg gag ctg agc agc ctg aga tct gag gac acg gcc atc tat tac tgt     288
Met Glu Leu Ser Ser Leu Arg Ser Glu Asp Thr Ala Ile Tyr Tyr Cys
                85                  90                  95
gcg aga agc ctt ggc tac cac act cag tac aac ggt atg gac gtc tgg     336
Ala Arg Ser Leu Gly Tyr His Thr Gln Tyr Asn Gly Met Asp Val Trp
            100                 105                 110
ggc caa ggg acc acg gtc acc gtc tcg agc                             366
Gly Gln Gly Thr Thr Val Thr Val Ser Ser
        115                 120
<210>  216
<211>  122
<212>  PRT

<213>  human
<400>  216
Gln Val Gln Leu Gln Glu Ser Gly Ala Glu Val Lys Lys Pro Gly Ser
1               5                   10                  15
Ser Val Lys Val Ser Cys Lys Ala Ser Gly Gly Pro Phe Arg Ser His
            20                  25                  30
Ala Ile Ser Trp Val Arg Gln Ala Ala Gly Gln Gly Leu Glu Trp Met
        35                  40                  45
Gly Gly Ile Thr Ala Val Phe Gly Thr Ala Asn Tyr Ala Gln Lys Phe
    50                  55                  60
Gln Gly Arg Val Thr Ile Thr Ala Asp Glu Ser Thr Asn Ile Val Ser
65                  70                  75                  80
Met Glu Leu Ser Ser Leu Arg Ser Glu Asp Thr Ala Ile Tyr Tyr Cys
                85                  90                  95
Ala Arg Ser Leu Gly Tyr His Thr Gln Tyr Asn Gly Met Asp Val Trp
            100                 105                 110
Gly Gln Gly Thr Thr Val Thr Val Ser Ser
        115                 120
<210>  217
<211>  321
<212>  DNA
<213>  human
<220>
<221>  CDS
<222>  (1)..(321)
<400>  217
gat gtt gtg atg act cag tct cca tct tct gtg tct gca tct gta gga        48
Asp Val Val Met Thr Gln Ser Pro Ser Ser Val Ser Ala Ser Val Gly
1               5                   10                  15
gac aga gtc acc atc act tgt cgg gcg agt cag ggt att agc agc tgg        96
Asp Arg Val Thr Ile Thr Cys Arg Ala Ser Gln Gly Ile Ser Ser Trp
            20                  25                  30
tta gcc tgg tat cag cag aaa cca ggg aaa gcc cct aag ctc ctg atc       144
Leu Ala Trp Tyr Gln Gln Lys Pro Gly Lys Ala Pro Lys Leu Leu Ile
        35                  40                  45
tat gct gca tcc agt ttg caa agt ggg gtc cca tca agg ttc agc ggc       192
Tyr Ala Ala Ser Ser Leu Gln Ser Gly Val Pro Ser Arg Phe Ser Gly
    50                  55                  60
agt gga tct ggg aca gat ttc act ctc act atc agc agc ctg cag cct       240
Ser Gly Ser Gly Thr Asp Phe Thr Leu Thr Ile Ser Ser Leu Gln Pro
65                  70                  75                  80
gaa gat ttt gca act tac tat tgt caa cag gct aac agt ttc ccg atc       288
Glu Asp Phe Ala Thr Tyr Tyr Cys Gln Gln Ala Asn Ser Phe Pro Ile
                85                  90                  95
acc ttc ggc caa ggg aca cga ctg gag att aaa                           321
Thr Phe Gly Gln Gly Thr Arg Leu Glu Ile Lys
            100                 105
<210>  218
<211>  107
<212>  PRT
<213>  human
<400>  218
Asp Val Val Met Thr Gln Ser Pro Ser Ser Val Ser Ala Ser Val Gly
1               5                   10                  15
Asp Arg Val Thr Ile Thr Cys Arg Ala Ser Gln Gly Ile Ser Ser Trp
            20                  25                  30
Leu Ala Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ala Asn Ser Phe Pro Ile

85                  90                  95
Thr Phe Gly Gln Gly Thr Arg Leu Glu Ile Lys
            100                 105
<210>  219
<211>  363
<212>  DNA
<213>  human
<220>
<221>  CDS
<222>  (1)..(363)
<400>  219
cag gta cag ctg cag cag tca ggg gct gac gtg aag aag gct ggg tcc      48
Gln Val Gln Leu Gln Gln Ser Gly Ala Asp Val Lys Lys Ala Gly Ser
1               5                   10                  15
tcg gtg aag gtc tcc tgc aag gct tct gga cgc acc ttc ggc aat tat      96
Ser Val Lys Val Ser Cys Lys Ala Ser Gly Arg Thr Phe Gly Asn Tyr
            20                  25                  30
gct atc agc tgg gtg cga cag gcc cct gga caa gga ctt gag tgg atg      144
Ala Ile Ser Trp Val Arg Gln Ala Pro Gly Gln Gly Leu Glu Trp Met
        35                  40                  45
gga ggg atc acc cct atc ttt gga gca gca aac tac gca cag aag ttc      192
Gly Gly Ile Thr Pro Ile Phe Gly Ala Ala Asn Tyr Ala Gln Lys Phe
    50                  55                  60
cag ggc aga gtc act att tcc gcg gac tta tcc acg aga atg gtc tat      240
Gln Gly Arg Val Thr Ile Ser Ala Asp Leu Ser Thr Arg Met Val Tyr
65                  70                  75                  80
atg gac ttg agc agc ctg aga tct gac gac acg gcc ttc tat tac tgt      288
Met Asp Leu Ser Ser Leu Arg Ser Asp Asp Thr Ala Phe Tyr Tyr Cys
                85                  90                  95
gcg gga cat ccc acg tat cat tat ggt tcg gcg atg gac tac tgg ggc      336
Ala Gly His Pro Thr Tyr His Tyr Gly Ser Ala Met Asp Tyr Trp Gly
            100                 105                 110
cag gga acc ctg gtc acc gtc tcg agc                                  363
Gln Gly Thr Leu Val Thr Val Ser Ser
        115                 120
<210>  220
<211>  121
<212>  PRT
<213>  human
<400>  220
Gln Val Gln Leu Gln Gln Ser Gly Ala Asp Val Lys Lys Ala Gly Ser
1               5                   10                  15
Ser Val Lys Val Ser Cys Lys Ala Ser Gly Arg Thr Phe Gly Asn Tyr
            20                  25                  30
Ala Ile Ser Trp Val Arg Gln Ala Pro Gly Gln Gly Leu Glu Trp Met
        35                  40                  45
Gly Gly Ile Thr Pro Ile Phe Gly Ala Ala Asn Tyr Ala Gln Lys Phe
    50                  55                  60
Gln Gly Arg Val Thr Ile Ser Ala Asp Leu Ser Thr Arg Met Val Tyr
65                  70                  75                  80
Met Asp Leu Ser Ser Leu Arg Ser Asp Asp Thr Ala Phe Tyr Tyr Cys
                85                  90                  95
Ala Gly His Pro Thr Tyr His Tyr Gly Ser Ala Met Asp Tyr Trp Gly
            100                 105                 110
Gln Gly Thr Leu Val Thr Val Ser Ser
        115                 120
<210>  221
<211>  333
<212>  DNA
<213>  human
<220>
<221>  CDS
<222>  (1)..(333)

<400>  221
cag tct gtg ttg acg cag ccg ccc tca gtg tct gcg gcc cca ggc cag      48
Gln Ser Val Leu Thr Gln Pro Pro Ser Val Ser Ala Ala Pro Gly Gln
1               5                   10                  15
aag gtc acc atc acc tgt tct gga agc agc tcc aac att ggc aat aat      96
Lys Val Thr Ile Thr Cys Ser Gly Ser Ser Ser Asn Ile Gly Asn Asn
            20                  25                  30
tat gtg tcc tgg tac cag caa ctc cca ggg aca gcc ccc aaa ctc ctc      144
Tyr Val Ser Trp Tyr Gln Gln Leu Pro Gly Thr Ala Pro Lys Leu Leu
        35                  40                  45
att tct gaa gga agt aat cga ccc tcc ggg att cct gac cgc ttc tct      192
Ile Ser Glu Gly Ser Asn Arg Pro Ser Gly Ile Pro Asp Arg Phe Ser
    50                  55                  60
ggc tcc aag tct ggc acg tca gcc acc ctg ggc atc acc gga ctc cag      240
Gly Ser Lys Ser Gly Thr Ser Ala Thr Leu Gly Ile Thr Gly Leu Gln
65                  70                  75                  80
act ggg gac gag gcc act tat tac tgc gca aca tgg gat gac agc ctg      288
Thr Gly Asp Glu Ala Thr Tyr Tyr Cys Ala Thr Trp Asp Asp Ser Leu
                85                  90                  95
agt gtc gtg ctt ttc ggc gga ggg acc cga ctg acc gtc ctt agt      333
Ser Val Val Leu Phe Gly Gly Gly Thr Arg Leu Thr Val Leu Ser
            100                 105                 110
<210>  222
<211>  111
<212>  PRT
<213>  human
<400>  222
Gln Ser Val Leu Thr Gln Pro Pro Ser Val Ser Ala Ala Pro Gly Gln
1               5                   10                  15
Lys Val Thr Ile Thr Cys Ser Gly Ser Ser Ser Asn Ile Gly Asn Asn
            20                  25                  30
Tyr Val Ser Trp Tyr Gln Gln Leu Pro Gly Thr Ala Pro Lys Leu Leu
        35                  40                  45
Ile Ser Glu Gly Ser Asn Arg Pro Ser Gly Ile Pro Asp Arg Phe Ser
    50                  55                  60
Gly Ser Lys Ser Gly Thr Ser Ala Thr Leu Gly Ile Thr Gly Leu Gln
65                  70                  75                  80
Thr Gly Asp Glu Ala Thr Tyr Tyr Cys Ala Thr Trp Asp Asp Ser Leu
                85                  90                  95
Ser Val Val Leu Phe Gly Gly Gly Thr Arg Leu Thr Val Leu Ser
            100                 105                 110
<210>  223
<211>  360
<212>  DNA
<213>  human
<220>
<221>  CDS
<222>  (1)..(360)
<400>  223
cag gtg cag ctg gtg cag tct ggg gct gag gtg aag aag cct ggg tcc      48
Gln Val Gln Leu Val Gln Ser Gly Ala Glu Val Lys Lys Pro Gly Ser
1               5                   10                  15
tcg gtg aag gtc tcc tgc aag gct tct gga ggc atc ttc agg aag agt      96
Ser Val Lys Val Ser Cys Lys Ala Ser Gly Gly Ile Phe Arg Lys Ser
            20                  25                  30
gct atc acc tgg gtg cga cag gcc ccc gga caa ggg ctt gag tgg atg      144
Ala Ile Thr Trp Val Arg Gln Ala Pro Gly Gln Gly Leu Glu Trp Met
        35                  40                  45
gga ggg atc atc gct atc ttt ggt aca aca aac tac gct cag aag ttc      192
Gly Gly Ile Ile Ala Ile Phe Gly Thr Thr Asn Tyr Ala Gln Lys Phe
    50                  55                  60
cag gac aga gtc acg att acc gcg gac gat tcc acg aac aca gtc tac      240
Gln Asp Arg Val Thr Ile Thr Ala Asp Asp Ser Thr Asn Thr Val Tyr

65                  70                  75                  80
atg gaa ctg acc agc ctg aga tat gag gac acg gcc gtc tat tac tgt      288
Met Glu Leu Thr Ser Leu Arg Tyr Glu Asp Thr Ala Val Tyr Tyr Cys
                85                  90                  95
gcg aga ggg ccc aat tac tat gaa agt tac ctt gac ttc tgg ggc cag      336
Ala Arg Gly Pro Asn Tyr Tyr Glu Ser Tyr Leu Asp Phe Trp Gly Gln
            100                 105                 110
ggg acc acg gtc acc gtc tcg agc                                      360
Gly Thr Thr Val Thr Val Ser Ser
        115                 120
<210>  224
<211>  120
<212>  PRT
<213>  human
<400>  224
Gln Val Gln Leu Val Gln Ser Gly Ala Glu Val Lys Lys Pro Gly Ser
1               5                   10                  15
Ser Val Lys Val Ser Cys Lys Ala Ser Gly Gly Ile Phe Arg Lys Ser
            20                  25                  30
Ala Ile Thr Trp Val Arg Gln Ala Pro Gly Gln Gly Leu Glu Trp Met
        35                  40                  45
Gly Gly Ile Ile Ala Ile Phe Gly Thr Thr Asn Tyr Ala Gln Lys Phe
    50                  55                  60
Gln Asp Arg Val Thr Ile Thr Ala Asp Asp Ser Thr Asn Thr Val Tyr
65                  70                  75                  80
Met Glu Leu Thr Ser Leu Arg Tyr Glu Asp Thr Ala Val Tyr Tyr Cys
                85                  90                  95
Ala Arg Gly Pro Asn Tyr Tyr Glu Ser Tyr Leu Asp Phe Trp Gly Gln
            100                 105                 110
Gly Thr Thr Val Thr Val Ser Ser
        115                 120
<210>  225
<211>  321
<212>  DNA
<213>  human
<220>
<221>  CDS
<222>  (1)..(321)
<400>  225
gaa att gtg ttg acg cag tct cct tcc acc ctg tct gca tct gta gga       48
Glu Ile Val Leu Thr Gln Ser Pro Ser Thr Leu Ser Ala Ser Val Gly
1               5                   10                  15
gac aga gtc acc atc act tgc cgg gcc agt cag agt att agt agc tgg       96
Asp Arg Val Thr Ile Thr Cys Arg Ala Ser Gln Ser Ile Ser Ser Trp
            20                  25                  30
ttg gcc tgg tat cag cag aaa cca ggg aaa gcc cct aag ctc ctg atc      144
Leu Ala Trp Tyr Gln Gln Lys Pro Gly Lys Ala Pro Lys Leu Leu Ile
        35                  40                  45
tat aag gcg tct agt tta gaa agt ggg gtc cca tca agg ttc agc ggc      192
Tyr Lys Ala Ser Ser Leu Glu Ser Gly Val Pro Ser Arg Phe Ser Gly
    50                  55                  60
agt gga tct ggg aca gaa ttc act ctc acc atc agc agc ctg cag cct      240
Ser Gly Ser Gly Thr Glu Phe Thr Leu Thr Ile Ser Ser Leu Gln Pro
65                  70                  75                  80
gat gat ttt gca act tat tac tgc caa cag tat aat agt tat ccc cgg      288
Asp Asp Phe Ala Thr Tyr Tyr Cys Gln Gln Tyr Asn Ser Tyr Pro Arg
                85                  90                  95
acg ttc ggc caa ggg acc aag gtg gaa atc aaa                          321
Thr Phe Gly Gln Gly Thr Lys Val Glu Ile Lys
            100                 105
<210>  226
<211>  107
<212>  PRT

<213>  human
<400>  226
Glu Ile Val Leu Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Lys Pro Gly Lys Ala Pro Lys Leu Leu Ile
        35                  40                  45
Tyr Lys Ala Ser Ser Leu Glu Ser Gly Val Pro Ser Arg Phe Ser Gly
    50                  55                  60
Ser Gly Ser Gly Thr Glu Phe Thr Leu Thr Ile Ser Ser Leu Gln Pro
65                  70                  75                  80
Asp Asp Phe Ala Thr Tyr Tyr Cys Gln Gln Tyr Asn Ser Tyr Pro Arg
                85                  90                  95
Thr Phe Gly Gln Gly Thr Lys Val Glu Ile Lys
            100                 105
<210>  227
<211>  360
<212>  DNA
<213>  human
<220>
<221>  CDS
<222>  (1)..(360)
<400>  227
cag gtg cag ctg gtg caa tct ggg tct gag gtg aag aag cct ggg tcc      48
Gln Val Gln Leu Val Gln Ser Gly Ser Glu Val Lys Lys Pro Gly Ser
1               5                   10                  15
tcg gtg aag gtc tcc tgc aag gct tct gga gtc acc ttc agc agt tat      96
Ser Val Lys Val Ser Cys Lys Ala Ser Gly Val Thr Phe Ser Ser Tyr
            20                  25                  30
gct atc agc tgg gtg cga cag gcc ccc gga caa ggg ctt gag tgg atg      144
Ala Ile Ser Trp Val Arg Gln Ala Pro Gly Gln Gly Leu Glu Trp Met
        35                  40                  45
gga ggg atc agc cct atg ttt gga acg aca agg tat gca cag aag ttc      192
Gly Gly Ile Ser Pro Met Phe Gly Thr Thr Arg Tyr Ala Gln Lys Phe
    50                  55                  60
cag ggg aga gtc acg att acc gcg gac gaa tcc acg agg aca ggc ttc      240
Gln Gly Arg Val Thr Ile Thr Ala Asp Glu Ser Thr Arg Thr Gly Phe
65                  70                  75                  80
atg gag ctg agc agc ctg aga tct ggg gac acg gcc gtg tat tac tgt      288
Met Glu Leu Ser Ser Leu Arg Ser Gly Asp Thr Ala Val Tyr Tyr Cys
                85                  90                  95
gcg aga tcc cca act tat tat cct ggc gct ctt gat atg tgg ggc caa      336
Ala Arg Ser Pro Thr Tyr Tyr Pro Gly Ala Leu Asp Met Trp Gly Gln
            100                 105                 110
ggg acc acg gtc acc gtc tcg agc                                      360
Gly Thr Thr Val Thr Val Ser Ser
        115                 120
<210>  228
<211>  120
<212>  PRT
<213>  human
<400>  228
Gln Val Gln Leu Val Gln Ser Gly Ser Glu Val Lys Lys Pro Gly Ser
1               5                   10                  15
Ser Val Lys Val Ser Cys Lys Ala Ser Gly Val Thr Phe Ser Ser Tyr
            20                  25                  30
Ala Ile Ser Trp Val Arg Gln Ala Pro Gly Gln Gly Leu Glu Trp Met
        35                  40                  45
Gly Gly Ile Ser Pro Met Phe Gly Thr Thr Arg Tyr Ala Gln Lys Phe
    50                  55                  60
Gln Gly Arg Val Thr Ile Thr Ala Asp Glu Ser Thr Arg Thr Gly Phe
65                  70                  75                  80

Met Glu Leu Ser Ser Leu Arg Ser Gly Asp Thr Ala Val Tyr Tyr Cys
                85                  90                  95
Ala Arg Ser Pro Thr Tyr Tyr Pro Gly Ala Leu Asp Met Trp Gly Gln
            100                 105                 110
Gly Thr Thr Val Thr Val Ser Ser
        115                 120
<210>  229
<211>  321
<212>  DNA
<213>  human
<220>
<221>  CDS
<222>  (1)..(321)
<400>  229
gat gtt gtg atg act cag tct cca tct ttg ctg tct gca tct gtg gga      48
Asp Val Val Met Thr Gln Ser Pro Ser Leu Leu Ser Ala Ser Val Gly
1               5                   10                  15
gac aga gtc acc atc act tgt cgg gcg agt cag gcc atc gcc aat ttc      96
Asp Arg Val Thr Ile Thr Cys Arg Ala Ser Gln Ala Ile Ala Asn Phe
            20                  25                  30
gta tcc tgg ttt cag cag aaa cca ggg gaa gcc cct aag tcc ctg atc      144
Val Ser Trp Phe Gln Gln Lys Pro Gly Glu Ala Pro Lys Ser Leu Ile
        35                  40                  45
cat gct gca tcc act ttg caa ggt gga gtc cca ccg aga ttc agc ggc      192
His Ala Ala Ser Thr Leu Gln Gly Gly Val Pro Pro Arg Phe Ser Gly
    50                  55                  60
agt gga tat ggg gcg gtt ttc act ctc acc atc acc aac ctg cag ccg      240
Ser Gly Tyr Gly Ala Val Phe Thr Leu Thr Ile Thr Asn Leu Gln Pro
65                  70                  75                  80
gaa gat ttt gca act tat ttc tgc caa caa tat aac agt ctc ccc ttc      288
Glu Asp Phe Ala Thr Tyr Phe Cys Gln Gln Tyr Asn Ser Leu Pro Phe
                85                  90                  95
act ttc ggc cct ggg acc gat gtg gaa gtg cga                          321
Thr Phe Gly Pro Gly Thr Asp Val Glu Val Arg
            100                 105
<210>  230
<211>  107
<212>  PRT
<213>  human
<400>  230
Asp Val Val Met Thr Gln Ser Pro Ser Leu Leu Ser Ala Ser Val Gly
1               5                   10                  15
Asp Arg Val Thr Ile Thr Cys Arg Ala Ser Gln Ala Ile Ala Asn Phe
            20                  25                  30
Val Ser Trp Phe Gln Gln Lys Pro Gly Glu Ala Pro Lys Ser Leu Ile
        35                  40                  45
His Ala Ala Ser Thr Leu Gln Gly Gly Val Pro Pro Arg Phe Ser Gly
    50                  55                  60
Ser Gly Tyr Gly Ala Val Phe Thr Leu Thr Ile Thr Asn Leu Gln Pro
65                  70                  75                  80
Glu Asp Phe Ala Thr Tyr Phe Cys Gln Gln Tyr Asn Ser Leu Pro Phe
                85                  90                  95
Thr Phe Gly Pro Gly Thr Asp Val Glu Val Arg
            100                 105
<210>  231
<211>  372
<212>  DNA
<213>  human
<220>
<221>  CDS
<222>  (1)..(372)
<400>  231
cag gtg cag ctg gtg cag tct ggg gct gag gtg aag aag cct ggg tcc      48

Gln Val Gln Leu Val Gln Ser Gly Ala Glu Val Lys Lys Pro Gly Ser
1               5                   10                  15
tcg gtg aag gtc tcc tgc aag gct tct gga ggc atc ttc aac agt aat      96
Ser Val Lys Val Ser Cys Lys Ala Ser Gly Gly Ile Phe Asn Ser Asn
            20                  25                  30
gct atc agc tgg gtg cga cag gcc cct gga caa ggg ctt gag tgg atg      144
Ala Ile Ser Trp Val Arg Gln Ala Pro Gly Gln Gly Leu Glu Trp Met
        35                  40                  45
gga ggg atc atc ggt atc ttt gga aca gca aac tac gca cag aag ttc      192
Gly Gly Ile Ile Gly Ile Phe Gly Thr Ala Asn Tyr Ala Gln Lys Phe
    50                  55                  60
cag ggc aga gta acg att gcc gcg gac caa tcc acg aac aca gtc ttc      240
Gln Gly Arg Val Thr Ile Ala Ala Asp Gln Ser Thr Asn Thr Val Phe
65                  70                  75                  80
atg gag ttg agt agc ctg aca tct gag gac acg gcc gtg tat ttc tgt      288
Met Glu Leu Ser Ser Leu Thr Ser Glu Asp Thr Ala Val Tyr Phe Cys
                85                  90                  95
gcg aga tcc cgt gga tac agc ttt ggt tac ggc acc gac tac ttt gac      336
Ala Arg Ser Arg Gly Tyr Ser Phe Gly Tyr Gly Thr Asp Tyr Phe Asp
            100                 105                 110
tac tgg ggc cag ggc acc ctg gtc acc gtc tcg agc                      372
Tyr Trp Gly Gln Gly Thr Leu Val Thr Val Ser Ser
        115                 120
<210>  232
<211>  124
<212>  PRT
<213>  human
<400>  232
Gln Val Gln Leu Val Gln Ser Gly Ala Glu Val Lys Lys Pro Gly Ser
1               5                   10                  15
Ser Val Lys Val Ser Cys Lys Ala Ser Gly Gly Ile Phe Asn Ser Asn
            20                  25                  30
Ala Ile Ser Trp Val Arg Gln Ala Pro Gly Gln Gly Leu Glu Trp Met
        35                  40                  45
Gly Gly Ile Ile Gly Ile Phe Gly Thr Ala Asn Tyr Ala Gln Lys Phe
    50                  55                  60
Gln Gly Arg Val Thr Ile Ala Ala Asp Gln Ser Thr Asn Thr Val Phe
65                  70                  75                  80
Met Glu Leu Ser Ser Leu Thr Ser Glu Asp Thr Ala Val Tyr Phe Cys
                85                  90                  95
Ala Arg Ser Arg Gly Tyr Ser Phe Gly Tyr Gly Thr Asp Tyr Phe Asp
            100                 105                 110
Tyr Trp Gly Gln Gly Thr Leu Val Thr Val Ser Ser
        115                 120
<210>  233
<211>  321
<212>  DNA
<213>  human
<220>
<221>  CDS
<222>  (1)..(321)
<400>  233
gac atc gtg atg acc cag tct cct tcc acc ctg tct gca tct gta gga      48
Asp Ile Val Met Thr Gln Ser Pro Ser Thr Leu Ser Ala Ser Val Gly
1               5                   10                  15
gac aga gtc acc atc act tgc cgg gcc agt cag agt att agt agt tgg      96
Asp Arg Val Thr Ile Thr Cys Arg Ala Ser Gln Ser Ile Ser Ser Trp
            20                  25                  30
ttg gcc tgg tat cag cag gcc cca ggg aaa gcc cct aag ctc ctg atc      144
Leu Ala Trp Tyr Gln Gln Ala Pro Gly Lys Ala Pro Lys Leu Leu Ile
        35                  40                  45
tat gag gcg tcg aaa tta caa agc ggg gtc cca tca agg ttc agc gcc      192
Tyr Glu Ala Ser Lys Leu Gln Ser Gly Val Pro Ser Arg Phe Ser Ala

50                  55                  60
act gga tct ggg aca gaa ttc act ctc acc atc gcc agc ctg cag cct      240
Thr Gly Ser Gly Thr Glu Phe Thr Leu Thr Ile Ala Ser Leu Gln Pro
65                  70                  75                  80
gag gat ttt gca att tat tac tgc caa cac tac gag gct tac cca ttc      288
Glu Asp Phe Ala Ile Tyr Tyr Cys Gln His Tyr Glu Ala Tyr Pro Phe
                85                  90                  95
agt ttc ggc cct ggg acc aag ttg gat gtc aaa                          321
Ser Phe Gly Pro Gly Thr Lys Leu Asp Val Lys
            100                 105
<210>  234
<211>  107
<212>  PRT
<213>  human
<400>  234
Asp Ile Val Met Thr Gln Ser Pro Ser Thr Leu Ser Ala Ser Val Gly
1               5                   10                  15
Asp Arg Val Thr Ile Thr Cys Arg Ala Ser Gln Ser Ile Ser Ser Trp
            20                  25                  30
Leu Ala Trp Tyr Gln Gln Ala Pro Gly Lys Ala Pro Lys Leu Leu Ile
        35                  40                  45
Tyr Glu Ala Ser Lys Leu Gln Ser Gly Val Pro Ser Arg Phe Ser Ala
    50                  55                  60
Thr Gly Ser Gly Thr Glu Phe Thr Leu Thr Ile Ala Ser Leu Gln Pro
65                  70                  75                  80
Glu Asp Phe Ala Ile Tyr Tyr Cys Gln His Tyr Glu Ala Tyr Pro Phe
                85                  90                  95
Ser Phe Gly Pro Gly Thr Lys Leu Asp Val Lys
            100                 105
<210>  235
<211>  363
<212>  DNA
<213>  human
<220>
<221>  CDS
<222>  (1)..(363)
<400>  235
cag gtg cag ctg gtg cag tct ggg gct gag gtg aag aag cct ggg tcc       48
Gln Val Gln Leu Val Gln Ser Gly Ala Glu Val Lys Lys Pro Gly Ser
1               5                   10                  15
tcg gtg aag gtc tcc tgc gag gcc tct gga gcc acc ttc agc agt aat       96
Ser Val Lys Val Ser Cys Glu Ala Ser Gly Ala Thr Phe Ser Ser Asn
            20                  25                  30
gct ttc agc tgg gtg cgg cag gcc cct gga caa ggg cct gag tgg atg      144
Ala Phe Ser Trp Val Arg Gln Ala Pro Gly Gln Gly Pro Glu Trp Met
        35                  40                  45
gga ggg atc atc act atg ttt aga aaa gca gag tac gca cag aag ttc      192
Gly Gly Ile Ile Thr Met Phe Arg Lys Ala Glu Tyr Ala Gln Lys Phe
    50                  55                  60
cag ggc aga gtc acg att acc gcg ggc gaa ctg ggg agt acg gcc tac      240
Gln Gly Arg Val Thr Ile Thr Ala Gly Glu Leu Gly Ser Thr Ala Tyr
65                  70                  75                  80
atg gag gtg aga agc ctg aca ttt gag gac acg gcc gtg tat tac tgc      288
Met Glu Val Arg Ser Leu Thr Phe Glu Asp Thr Ala Val Tyr Tyr Cys
                85                  90                  95
gcg aga cat agt ggt tac cat ttg atc ggc tac ttt gac tcc tgg ggc      336
Ala Arg His Ser Gly Tyr His Leu Ile Gly Tyr Phe Asp Ser Trp Gly
            100                 105                 110
cag ggc acc ctg gtc acc gtc tcg agc                                  363
Gln Gly Thr Leu Val Thr Val Ser Ser
        115                 120
<210>  236
<211>  121

<212>  PRT
<213>  human
<400>  236
Gln Val Gln Leu Val Gln Ser Gly Ala Glu Val Lys Lys Pro Gly Ser
1               5                   10                  15
Ser Val Lys Val Ser Cys Glu Ala Ser Gly Ala Thr Phe Ser Ser Asn
            20                  25                  30
Ala Phe Ser Trp Val Arg Gln Ala Pro Gly Gln Gly Pro Glu Trp Met
        35                  40                  45
Gly Gly Ile Ile Thr Met Phe Arg Lys Ala Glu Tyr Ala Gln Lys Phe
    50                  55                  60
Gln Gly Arg Val Thr Ile Thr Ala Gly Glu Leu Gly Ser Thr Ala Tyr
65                  70                  75                  80
Met Glu Val Arg Ser Leu Thr Phe Glu Asp Thr Ala Val Tyr Tyr Cys
                85                  90                  95
Ala Arg His Ser Gly Tyr His Leu Ile Gly Tyr Phe Asp Ser Trp Gly
            100                 105                 110
Gln Gly Thr Leu Val Thr Val Ser Ser
        115                 120
<210>  237
<211>  333
<212>  DNA
<213>  human
<220>
<221>  CDS
<222>  (1)..(333)
<400>  237
cag tct gtg ttg acg cag ccg ccc tca gtg tct gcg gcc cca gga cag      48
Gln Ser Val Leu Thr Gln Pro Pro Ser Val Ser Ala Ala Pro Gly Gln
1               5                   10                  15
aag gtc acc atc tcc tgc tct gga acc act tcc aac att gga aat aat      96
Lys Val Thr Ile Ser Cys Ser Gly Thr Thr Ser Asn Ile Gly Asn Asn
            20                  25                  30
tat gtt tcc tgg tac cag caa ctc cca ggg gca gcc ccc aaa ctc ctc      144
Tyr Val Ser Trp Tyr Gln Gln Leu Pro Gly Ala Ala Pro Lys Leu Leu
        35                  40                  45
att tat gac aat aat aag cga ccc tca ggg att cct gac cga ttc tct      192
Ile Tyr Asp Asn Asn Lys Arg Pro Ser Gly Ile Pro Asp Arg Phe Ser
    50                  55                  60
ggc tcc aag tct gac acg tca gcc acc ctg ggc atc acc gga ctc cag      240
Gly Ser Lys Ser Asp Thr Ser Ala Thr Leu Gly Ile Thr Gly Leu Gln
65                  70                  75                  80
act ggg gac gag gcc gat tat tac tgc gga aca tgg gtt agc agc ctg      288
Thr Gly Asp Glu Ala Asp Tyr Tyr Cys Gly Thr Trp Val Ser Ser Leu
                85                  90                  95
agt gtc tgg gtg ttc ggc gga ggg acc aag ctg acc gtc cta ggt          333
Ser Val Trp Val Phe Gly Gly Gly Thr Lys Leu Thr Val Leu Gly
            100                 105                 110
<210>  238
<211>  111
<212>  PRT
<213>  human
<400>  238
Gln Ser Val Leu Thr Gln Pro Pro Ser Val Ser Ala Ala Pro Gly Gln
1               5                   10                  15
Lys Val Thr Ile Ser Cys Ser Gly Thr Thr Ser Asn Ile Gly Asn Asn
            20                  25                  30
Tyr Val Ser Trp Tyr Gln Gln Leu Pro Gly Ala Ala Pro Lys Leu Leu
        35                  40                  45
Ile Tyr Asp Asn Asn Lys Arg Pro Ser Gly Ile Pro Asp Arg Phe Ser
    50                  55                  60
Gly Ser Lys Ser Asp Thr Ser Ala Thr Leu Gly Ile Thr Gly Leu Gln
65                  70                  75                  80

Thr Gly Asp Glu Ala Asp Tyr Tyr Cys Gly Thr Trp Val Ser Ser Leu
                85                  90                  95
Ser Val Trp Val Phe Gly Gly Gly Thr Lys Leu Thr Val Leu Gly
            100                 105                 110
<210>  239
<211>  360
<212>  DNA
<213>  human
<220>
<221>  CDS
<222>  (1)..(360)
<400>  239
gag gtg cag ctg gtg gag tct ggg gct gag gtg aag aag cct ggg tcc        48
Glu Val Gln Leu Val Glu Ser Gly Ala Glu Val Lys Lys Pro Gly Ser
1               5                   10                  15
tcg gtg aag gtc tcc tgc aag gct tcc gga agc atc ttc agc aac tat        96
Ser Val Lys Val Ser Cys Lys Ala Ser Gly Ser Ile Phe Ser Asn Tyr
            20                  25                  30
gcc atc agc tgg gtg cga cag gcc cct gga cag ggg ctt gag tgg atg       144
Ala Ile Ser Trp Val Arg Gln Ala Pro Gly Gln Gly Leu Glu Trp Met
        35                  40                  45
gga ggc atc gtc cct atg ttt ggt aca aca aga ttc gca cag aag ttc       192
Gly Gly Ile Val Pro Met Phe Gly Thr Thr Arg Phe Ala Gln Lys Phe
    50                  55                  60
cag ggc aga gtc acg att acc gcg gac gaa tcc agg agc aca gcc tac       240
Gln Gly Arg Val Thr Ile Thr Ala Asp Glu Ser Arg Ser Thr Ala Tyr
65                  70                  75                  80
atg gag ctg aac aac ctg ata tct gaa gac acg gcc gtg tat tac tgt       288
Met Glu Leu Asn Asn Leu Ile Ser Glu Asp Thr Ala Val Tyr Tyr Cys
                85                  90                  95
gcg aga gct ccc ctc att tat aac tgg tac ttc gat ctc tgg ggc cgt       336
Ala Arg Ala Pro Leu Ile Tyr Asn Trp Tyr Phe Asp Leu Trp Gly Arg
            100                 105                 110
gga acc ctg gtc acc gtc tcg agc                                       360
Gly Thr Leu Val Thr Val Ser Ser
        115                 120
<210>  240
<211>  120
<212>  PRT
<213>  human
<400>  240
Glu Val Gln Leu Val Glu Ser Gly Ala Glu Val Lys Lys Pro Gly Ser
1               5                   10                  15
Ser Val Lys Val Ser Cys Lys Ala Ser Gly Ser Ile Phe Ser Asn Tyr
            20                  25                  30
Ala Ile Ser Trp Val Arg Gln Ala Pro Gly Gln Gly Leu Glu Trp Met
        35                  40                  45
Gly Gly Ile Val Pro Met Phe Gly Thr Thr Arg Phe Ala Gln Lys Phe
    50                  55                  60
Gln Gly Arg Val Thr Ile Thr Ala Asp Glu Ser Arg Ser Thr Ala Tyr
65                  70                  75                  80
Met Glu Leu Asn Asn Leu Ile Ser Glu Asp Thr Ala Val Tyr Tyr Cys
                85                  90                  95
Ala Arg Ala Pro Leu Ile Tyr Asn Trp Tyr Phe Asp Leu Trp Gly Arg
            100                 105                 110
Gly Thr Leu Val Thr Val Ser Ser
        115                 120
<210>  241
<211>  321
<212>  DNA
<213>  human
<220>
<221>  CDS

<222>  (1)..(321)
<400>  241
gaa att gtg ttg acg cag tct cca tct ttt gtg tct gcg tcg gtc gga        48
Glu Ile Val Leu Thr Gln Ser Pro Ser Phe Val Ser Ala Ser Val Gly
1               5                   10                  15
gac aga gtc agc atc act tgc cgg gcg agt cag ggt att agc agc tgg        96
Asp Arg Val Ser Ile Thr Cys Arg Ala Ser Gln Gly Ile Ser Ser Trp
            20                  25                  30
tta gcc tgg tat caa caa aaa ccc ggg aaa gcc cct aga ctc ctg atc       144
Leu Ala Trp Tyr Gln Gln Lys Pro Gly Lys Ala Pro Arg Leu Leu Ile
        35                  40                  45
tat ggt gca tcc aac ttg caa agt ggg gtc cca tca agg ttc agc ggc       192
Tyr Gly Ala Ser Asn Leu Gln Ser Gly Val Pro Ser Arg Phe Ser Gly
    50                  55                  60
agt gga tct ggg aca gat tac act ctc act atc aac agc ctg cag cct       240
Ser Gly Ser Gly Thr Asp Tyr Thr Leu Thr Ile Asn Ser Leu Gln Pro
65                  70                  75                  80
gaa gat ttt gca act tac tac tgt caa cag gct cgc acc ttc ccg gta       288
Glu Asp Phe Ala Thr Tyr Tyr Cys Gln Gln Ala Arg Thr Phe Pro Val
                85                  90                  95
act ttt ggc cag ggg acc aag ctg gag atc aaa                           321
Thr Phe Gly Gln Gly Thr Lys Leu Glu Ile Lys
            100                 105
<210>  242
<211>  107
<212>  PRT
<213>  human
<400>  242
Glu Ile Val Leu Thr Gln Ser Pro Ser Phe Val Ser Ala Ser Val Gly
1               5                   10                  15
Asp Arg Val Ser Ile Thr Cys Arg Ala Ser Gln Gly Ile Ser Ser Trp
            20                  25                  30
Leu Ala Trp Tyr Gln Gln Lys Pro Gly Lys Ala Pro Arg Leu Leu Ile
        35                  40                  45
Tyr Gly Ala Ser Asn Leu Gln Ser Gly Val Pro Ser Arg Phe Ser Gly
    50                  55                  60
Ser Gly Ser Gly Thr Asp Tyr Thr Leu Thr Ile Asn Ser Leu Gln Pro
65                  70                  75                  80
Glu Asp Phe Ala Thr Tyr Tyr Cys Gln Gln Ala Arg Thr Phe Pro Val
                85                  90                  95
Thr Phe Gly Gln Gly Thr Lys Leu Glu Ile Lys
            100                 105
<210>  243
<211>  363
<212>  DNA
<213>  human
<220>
<221>  CDS
<222>  (1)..(363)
<400>  243
cag gta cag ctg cag cag tca ggg gct gac gtg aag aag cct ggg tcc        48
Gln Val Gln Leu Gln Gln Ser Gly Ala Asp Val Lys Lys Pro Gly Ser
1               5                   10                  15
tcg gtg aag gtc tcc tgc aag gct tct gga ggc acc ttc ggc aac tat        96
Ser Val Lys Val Ser Cys Lys Ala Ser Gly Gly Thr Phe Gly Asn Tyr
            20                  25                  30
gct atc agc tgg gtg cga cag gcc cct gga caa ggg ctt gag tgg atg       144
Ala Ile Ser Trp Val Arg Gln Ala Pro Gly Gln Gly Leu Glu Trp Met
        35                  40                  45
gga ggg atc acc cct atc ttt gga cca gca aac tac gca cag agg ttc       192
Gly Gly Ile Thr Pro Ile Phe Gly Pro Ala Asn Tyr Ala Gln Arg Phe
    50                  55                  60
cag ggc aga gtc acg att tcc gcg gac ata tcc acg agg aca gcc tac       240

Gln Gly Arg Val Thr Ile Ser Ala Asp Ile Ser Thr Arg Thr Ala Tyr
65                  70                  75                  80
atg gag ctg agc agc ctg aga tct gag gac acg gcc ata tat tac tgt       288
Met Glu Leu Ser Ser Leu Arg Ser Glu Asp Thr Ala Ile Tyr Tyr Cys
                85                  90                  95
gcg aga cat cct acg tat tat ttt ggt tcg gcg atg gag tac tgg ggc       336
Ala Arg His Pro Thr Tyr Tyr Phe Gly Ser Ala Met Glu Tyr Trp Gly
            100                 105                 110
cag ggc acc ctg gtc acc gtc tcg agc                                   363
Gln Gly Thr Leu Val Thr Val Ser Ser
        115                 120
<210>  244
<211>  121
<212>  PRT
<213>  human
<400>  244
Gln Val Gln Leu Gln Gln Ser Gly Ala Asp Val Lys Lys Pro Gly Ser
1               5                   10                  15
Ser Val Lys Val Ser Cys Lys Ala Ser Gly Gly Thr Phe Gly Asn Tyr
            20                  25                  30
Ala Ile Ser Trp Val Arg Gln Ala Pro Gly Gln Gly Leu Glu Trp Met
        35                  40                  45
Gly Gly Ile Thr Pro Ile Phe Gly Pro Ala Asn Tyr Ala Gln Arg Phe
    50                  55                  60
Gln Gly Arg Val Thr Ile Ser Ala Asp Ile Ser Thr Arg Thr Ala Tyr
65                  70                  75                  80
Met Glu Leu Ser Ser Leu Arg Ser Glu Asp Thr Ala Ile Tyr Tyr Cys
                85                  90                  95
Ala Arg His Pro Thr Tyr Tyr Phe Gly Ser Ala Met Glu Tyr Trp Gly
            100                 105                 110
Gln Gly Thr Leu Val Thr Val Ser Ser
        115                 120
<210>  245
<211>  330
<212>  DNA
<213>  human
<220>
<221>  CDS
<222>  (1)..(330)
<400>  245
tcc tat gag ctg act cag cca ccc tcg gtg tca gtg gcc ccc ggt caa        48
Ser Tyr Glu Leu Thr Gln Pro Pro Ser Val Ser Val Ala Pro Gly Gln
1               5                   10                  15
acg gcc acc atc acc tgt ggg gga gac agg att ggg act gga cct gta        96
Thr Ala Thr Ile Thr Cys Gly Gly Asp Arg Ile Gly Thr Gly Pro Val
            20                  25                  30
cat tgg tat cag cag agg cca ggc cgg gcc cct gtc ctg atc att tat       144
His Trp Tyr Gln Gln Arg Pro Gly Arg Ala Pro Val Leu Ile Ile Tyr
        35                  40                  45
ggc agc act cat cgg ccc tca ggg atc cct gag cga ttc tct ggc tcc       192
Gly Ser Thr His Arg Pro Ser Gly Ile Pro Glu Arg Phe Ser Gly Ser
    50                  55                  60
aag tct gtc aat acg gcc acc ctg tcc atc agc ggg gtc gga gtc ggg       240
Lys Ser Val Asn Thr Ala Thr Leu Ser Ile Ser Gly Val Gly Val Gly
65                  70                  75                  80
gat gag gcc gac tat tac tgt cag gtg tgg gac act tcc act gcc cag       288
Asp Glu Ala Asp Tyr Tyr Cys Gln Val Trp Asp Thr Ser Thr Ala Gln
                85                  90                  95
ccg att gtt ttc ggc gga ggg acc aag ctg acc gtc ctc agt               330
Pro Ile Val Phe Gly Gly Gly Thr Lys Leu Thr Val Leu Ser
            100                 105                 110
<210>  246
<211>  110

<212>  PRT
<213>  human
<400>  246
Ser Tyr Glu Leu Thr Gln Pro Pro Ser Val Ser Val Ala Pro Gly Gln
1               5                   10                  15
Thr Ala Thr Ile Thr Cys Gly Gly Asp Arg Ile Gly Thr Gly Pro Val
            20                  25                  30
His Trp Tyr Gln Gln Arg Pro Gly Arg Ala Pro Val Leu Ile Ile Tyr
        35                  40                  45
Gly Ser Thr His Arg Pro Ser Gly Ile Pro Glu Arg Phe Ser Gly Ser
    50                  55                  60
Lys Ser Val Asn Thr Ala Thr Leu Ser Ile Ser Gly Val Gly Val Gly
65                  70                  75                  80
Asp Glu Ala Asp Tyr Tyr Cys Gln Val Trp Asp Thr Ser Thr Ala Gln
                85                  90                  95
Pro Ile Val Phe Gly Gly Gly Thr Lys Leu Thr Val Leu Ser
            100                 105                 110
<210>  247
<211>  360
<212>  DNA
<213>  human
<220>
<221>  CDS
<222>  (1)..(360)
<400>  247
cag gtg cag ctg gtg cag tct ggg gct gag gta aag aag cct ggg tcc      48
Gln Val Gln Leu Val Gln Ser Gly Ala Glu Val Lys Lys Pro Gly Ser
1               5                   10                  15
tcg gtg aag gtc tcc tgc aag gct tct gga ggc atc ttc aga agt cat      96
Ser Val Lys Val Ser Cys Lys Ala Ser Gly Gly Ile Phe Arg Ser His
            20                  25                  30
gct atc agc tgg gtg cga cag gcc cct gga caa ggg ctt gaa tgg atg      144
Ala Ile Ser Trp Val Arg Gln Ala Pro Gly Gln Gly Leu Glu Trp Met
        35                  40                  45
gga ggg ata atc gct ata ttt ggt aca aca cac tac gca cag cag ttc      192
Gly Gly Ile Ile Ala Ile Phe Gly Thr Thr His Tyr Ala Gln Gln Phe
    50                  55                  60
cag ggc aga gtc aca atc acc gcg gac gaa tcc acg agc aca gtg tac      240
Gln Gly Arg Val Thr Ile Thr Ala Asp Glu Ser Thr Ser Thr Val Tyr
65                  70                  75                  80
atg gag ctg agc acc ctg aga tct gag gac acg gcc gtg tat tac tgt      288
Met Glu Leu Ser Thr Leu Arg Ser Glu Asp Thr Ala Val Tyr Tyr Cys
                85                  90                  95
gcg aga ggg ccg aac tac ttt gag agt tac ttt gac aac tgg ggc cag      336
Ala Arg Gly Pro Asn Tyr Phe Glu Ser Tyr Phe Asp Asn Trp Gly Gln
            100                 105                 110
gga acc ctg gtc acc gtc tcg agc                                      360
Gly Thr Leu Val Thr Val Ser Ser
        115                 120
<210>  248
<211>  120
<212>  PRT
<213>  human
<400>  248
Gln Val Gln Leu Val Gln Ser Gly Ala Glu Val Lys Lys Pro Gly Ser
1               5                   10                  15
Ser Val Lys Val Ser Cys Lys Ala Ser Gly Gly Ile Phe Arg Ser His
            20                  25                  30
Ala Ile Ser Trp Val Arg Gln Ala Pro Gly Gln Gly Leu Glu Trp Met
        35                  40                  45
Gly Gly Ile Ile Ala Ile Phe Gly Thr Thr His Tyr Ala Gln Gln Phe
    50                  55                  60
Gln Gly Arg Val Thr Ile Thr Ala Asp Glu Ser Thr Ser Thr Val Tyr

65                  70                  75                  80
Met Glu Leu Ser Thr Leu Arg Ser Glu Asp Thr Ala Val Tyr Tyr Cys
                85                  90                  95
Ala Arg Gly Pro Asn Tyr Phe Glu Ser Tyr Phe Asp Asn Trp Gly Gln
            100                 105                 110
Gly Thr Leu Val Thr Val Ser Ser
        115                 120
<210>  249
<211>  324
<212>  DNA
<213>  human
<220>
<221>  CDS
<222>  (1)..(324)
<400>  249
gaa att gtg ttg acg cag tct cca gcc acc ctg tct ttg tct cca ggg       48
Glu Ile Val Leu Thr Gln Ser Pro Ala Thr Leu Ser Leu Ser Pro Gly
1               5                   10                  15
gaa aga gcc acc ctc tcc tgc agg gcc agt cag act att agc agc tac       96
Glu Arg Ala Thr Leu Ser Cys Arg Ala Ser Gln Thr Ile Ser Ser Tyr
            20                  25                  30
tta gcc tgg tac caa cag aaa cct ggc cag gct ccc agg ctc ctc atc      144
Leu Ala Trp Tyr Gln Gln Lys Pro Gly Gln Ala Pro Arg Leu Leu Ile
        35                  40                  45
tat ggt gca tcc aac agg gcc act ggc atc cca gcc agg ttc agt ggc      192
Tyr Gly Ala Ser Asn Arg Ala Thr Gly Ile Pro Ala Arg Phe Ser Gly
    50                  55                  60
agt ggg tct ggg aca gac ttc act ctc acc atc aac agc cta gag cct      240
Ser Gly Ser Gly Thr Asp Phe Thr Leu Thr Ile Asn Ser Leu Glu Pro
65                  70                  75                  80
gaa gat ttt gca gtt tat tac tgt cag cag cgt agt tcc tgg cct ccg      288
Glu Asp Phe Ala Val Tyr Tyr Cys Gln Gln Arg Ser Ser Trp Pro Pro
                85                  90                  95
atc acc ttc ggc caa ggg aca cga ctg gag att aaa                      324
Ile Thr Phe Gly Gln Gly Thr Arg Leu Glu Ile Lys
            100                 105
<210>  250
<211>  108
<212>  PRT
<213>  human
<400>  250
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Thr Ile Ser Ser Tyr
            20                  25                  30
Leu Ala Trp Tyr Gln Gln Lys Pro Gly Gln Ala Pro Arg Leu Leu Ile
        35                  40                  45
Tyr Gly Ala Ser Asn Arg Ala Thr Gly Ile Pro Ala Arg Phe Ser Gly
    50                  55                  60
Ser Gly Ser Gly Thr Asp Phe Thr Leu Thr Ile Asn Ser Leu Glu Pro
65                  70                  75                  80
Glu Asp Phe Ala Val Tyr Tyr Cys Gln Gln Arg Ser Ser Trp Pro Pro
                85                  90                  95
Ile Thr Phe Gly Gln Gly Thr Arg Leu Glu Ile Lys
            100                 105
<210>  251
<211>  360
<212>  DNA
<213>  human
<220>
<221>  CDS
<222>  (1)..(360)
<400>  251

cag gtg cag ctg gtg caa tct ggg tct gag gtg aag aag cct ggg tcc 48
Gln Val Gln Leu Val Gln Ser Gly Ser Glu Val Lys Lys Pro Gly Ser
1               5                   10                  15
tcg gtg aag gtc tcc tgc aag gct tct gga ggc att ttc cgc act aat 96
Ser Val Lys Val Ser Cys Lys Ala Ser Gly Gly Ile Phe Arg Thr Asn
            20                  25                  30
gcc atc agc tac gtg cga cag gcc cct gga caa ggg ctt gag tgg atg 144
Ala Ile Ser Tyr Val Arg Gln Ala Pro Gly Gln Gly Leu Glu Trp Met
        35                  40                  45
gga ggg atc acc gct atc ttt ggc aca cca aac tac gca cag aag ttc 192
Gly Gly Ile Thr Ala Ile Phe Gly Thr Pro Asn Tyr Ala Gln Lys Phe
    50                  55                  60
cag ggc aga gtc acc att atc gcg gac gaa tcc acg agc aca gtc tac 240
Gln Gly Arg Val Thr Ile Ile Ala Asp Glu Ser Thr Ser Thr Val Tyr
65                  70                  75                  80
atg gaa ctg ggc agt ctg aca tct gag gac acg gcc gtg tat tat tgt 288
Met Glu Leu Gly Ser Leu Thr Ser Glu Asp Thr Ala Val Tyr Tyr Cys
                85                  90                  95
gcg acc agt ggg acc tac tac gtc tcc tac ttt gac tcc tgg ggc cag 336
Ala Thr Ser Gly Thr Tyr Tyr Val Ser Tyr Phe Asp Ser Trp Gly Gln
            100                 105                 110
gga acc ctg gtc acc gtc tcg agc 360
Gly Thr Leu Val Thr Val Ser Ser
        115                 120
<210> 252
<211> 120
<212> PRT
<213> human
<400> 252
Gln Val Gln Leu Val Gln Ser Gly Ser Glu Val Lys Lys Pro Gly Ser
1               5                   10                  15
Ser Val Lys Val Ser Cys Lys Ala Ser Gly Gly Ile Phe Arg Thr Asn
            20                  25                  30
Ala Ile Ser Tyr Val Arg Gln Ala Pro Gly Gln Gly Leu Glu Trp Met
        35                  40                  45
Gly Gly Ile Thr Ala Ile Phe Gly Thr Pro Asn Tyr Ala Gln Lys Phe
    50                  55                  60
Gln Gly Arg Val Thr Ile Ile Ala Asp Glu Ser Thr Ser Thr Val Tyr
65                  70                  75                  80
Met Glu Leu Gly Ser Leu Thr Ser Glu Asp Thr Ala Val Tyr Tyr Cys
                85                  90                  95
Ala Thr Ser Gly Thr Tyr Tyr Val Ser Tyr Phe Asp Ser Trp Gly Gln
            100                 105                 110
Gly Thr Leu Val Thr Val Ser Ser
        115                 120
<210> 253
<211> 324
<212> DNA
<213> human
<220>
<221> CDS
<222> (1)..(324)
<400> 253
gac atc gtg atg acc cag tct cca ggc acc ctg act ttg tct cca ggg 48
Asp Ile Val Met Thr Gln Ser Pro Gly Thr Leu Thr Leu Ser Pro Gly
1               5                   10                  15
gaa aga gcc acc ctc tcc tgc agg gcc agt cag agt ctt tat agc agc 96
Glu Arg Ala Thr Leu Ser Cys Arg Ala Ser Gln Ser Leu Tyr Ser Ser
            20                  25                  30
cac tta gcc tgg tac cag cag aaa cct ggc cag cct ccc agg ctc ctc 144
His Leu Ala Trp Tyr Gln Gln Lys Pro Gly Gln Pro Pro Arg Leu Leu
        35                  40                  45
atc tat ggt gca tcc acc agg gcc ccg ggc act cca gac agg ttc agt 192

Ile Tyr Gly Ala Ser Thr Arg Ala Pro Gly Thr Pro Asp Arg Phe Ser
    50                  55                  60
ggc agt ggg tct ggg aca gac ttc act ctc acc atc acc gcc ctg gac      240
Gly Ser Gly Ser Gly Thr Asp Phe Thr Leu Thr Ile Thr Ala Leu Asp
65                  70                  75                  80
cct gaa gat tct gca gtg tat tac tgt cag caa tat ggt agc tca ccg      288
Pro Glu Asp Ser Ala Val Tyr Tyr Cys Gln Gln Tyr Gly Ser Ser Pro
                85                  90                  95
atc acc ttc ggc caa ggg acg cga ctg gag att aaa                      324
Ile Thr Phe Gly Gln Gly Thr Arg Leu Glu Ile Lys
            100                 105
<210>  254
<211>  108
<212>  PRT
<213>  human
<400>  254
Asp Ile Val Met Thr Gln Ser Pro Gly Thr Leu Thr Leu Ser Pro Gly
1               5                   10                  15
Glu Arg Ala Thr Leu Ser Cys Arg Ala Ser Gln Ser Leu Tyr Ser Ser
            20                  25                  30
His Leu Ala Trp Tyr Gln Gln Lys Pro Gly Gln Pro Pro Arg Leu Leu
        35                  40                  45
Ile Tyr Gly Ala Ser Thr Arg Ala Pro Gly Thr Pro Asp Arg Phe Ser
    50                  55                  60
Gly Ser Gly Ser Gly Thr Asp Phe Thr Leu Thr Ile Thr Ala Leu Asp
65                  70                  75                  80
Pro Glu Asp Ser Ala Val Tyr Tyr Cys Gln Gln Tyr Gly Ser Ser Pro
                85                  90                  95
Ile Thr Phe Gly Gln Gly Thr Arg Leu Glu Ile Lys
            100                 105
<210>  255
<211>  360
<212>  DNA
<213>  human
<220>
<221>  CDS
<222>  (1)..(360)
<400>  255
gag gtg cag ctg gtg cag tct ggg gct gag gtg aag aag cct ggg tcc       48
Glu Val Gln Leu Val Gln Ser Gly Ala Glu Val Lys Lys Pro Gly Ser
1               5                   10                  15
tcg gtg aag gtc tcc tgc cag gct tcc gga agc atc ttc agc aac tat       96
Ser Val Lys Val Ser Cys Gln Ala Ser Gly Ser Ile Phe Ser Asn Tyr
            20                  25                  30
gcc atc aac tgg gtg cgc cag gcc cct gga cag ggg ctt gag tgg atg      144
Ala Ile Asn Trp Val Arg Gln Ala Pro Gly Gln Gly Leu Glu Trp Met
        35                  40                  45
gga ggc atc gtc cct ata ttt ggt aca aca aga ttc gca cag aag ttc      192
Gly Gly Ile Val Pro Ile Phe Gly Thr Thr Arg Phe Ala Gln Lys Phe
    50                  55                  60
cag ggc aga ctc acg att acc gcg gac gaa ccc agg agc aca gcc tac      240
Gln Gly Arg Leu Thr Ile Thr Ala Asp Glu Pro Arg Ser Thr Ala Tyr
65                  70                  75                  80
atg gag ctg aac aac ctg ata tct gaa gac acg gcc gtg tat tac tgt      288
Met Glu Leu Asn Asn Leu Ile Ser Glu Asp Thr Ala Val Tyr Tyr Cys
                85                  90                  95
gcg aga gct ccc ctc att tat aac tgg tac tac gat ctc tgg ggc cgt      336
Ala Arg Ala Pro Leu Ile Tyr Asn Trp Tyr Tyr Asp Leu Trp Gly Arg
            100                 105                 110
gga acc ctg gtc acc gtc tcg agc                                      360
Gly Thr Leu Val Thr Val Ser Ser
        115                 120
<210>  256

<211>  120
<212>  PRT
<213>  human
<400>  256
Glu Val Gln Leu Val Gln Ser Gly Ala Glu Val Lys Lys Pro Gly Ser
1               5                   10                  15
Ser Val Lys Val Ser Cys Gln Ala Ser Gly Ser Ile Phe Ser Asn Tyr
            20                  25                  30
Ala Ile Asn Trp Val Arg Gln Ala Pro Gly Gln Gly Leu Glu Trp Met
        35                  40                  45
Gly Gly Ile Val Pro Ile Phe Gly Thr Thr Arg Phe Ala Gln Lys Phe
    50                  55                  60
Gln Gly Arg Leu Thr Ile Thr Ala Asp Glu Pro Arg Ser Thr Ala Tyr
65                  70                  75                  80
Met Glu Leu Asn Asn Leu Ile Ser Glu Asp Thr Ala Val Tyr Tyr Cys
                85                  90                  95
Ala Arg Ala Pro Leu Ile Tyr Asn Trp Tyr Tyr Asp Leu Trp Gly Arg
            100                 105                 110
Gly Thr Leu Val Thr Val Ser Ser
        115                 120
<210>  257
<211>  321
<212>  DNA
<213>  human
<220>
<221>  CDS
<222>  (1)..(321)
<400>  257
gaa att gtg ttg acg cag tct cca tcc tcc ctg tct gca tct gta gga      48
Glu Ile Val Leu Thr Gln Ser Pro Ser Ser Leu Ser Ala Ser Val Gly
1               5                   10                  15
gac aga gtc acc atc act tgc cgg gcg agt cag ggc att agc aat tat      96
Asp Arg Val Thr Ile Thr Cys Arg Ala Ser Gln Gly Ile Ser Asn Tyr
            20                  25                  30
tta gcc tgg tat cag cag aaa cca ggg aaa gtt cct aag ctc ctg atc      144
Leu Ala Trp Tyr Gln Gln Lys Pro Gly Lys Val Pro Lys Leu Leu Ile
        35                  40                  45
tat gct gca tcc act ttg caa tca ggg gtc cca tct cgg ttc agt ggc      192
Tyr Ala Ala Ser Thr Leu Gln Ser Gly Val Pro Ser Arg Phe Ser Gly
    50                  55                  60
agt gga tct ggg aca gat ttc act ctc acc atc agc agc ctg cag cct      240
Ser Gly Ser Gly Thr Asp Phe Thr Leu Thr Ile Ser Ser Leu Gln Pro
65                  70                  75                  80
gaa gat gtt gca act tat tac tgt caa aag tat aac agt gcc cct cac      288
Glu Asp Val Ala Thr Tyr Tyr Cys Gln Lys Tyr Asn Ser Ala Pro His
                85                  90                  95
act ttc ggc cct ggg acc aaa gtg gat atc aaa                          321
Thr Phe Gly Pro Gly Thr Lys Val Asp Ile Lys
            100                 105
<210>  258
<211>  107
<212>  PRT
<213>  human
<400>  258
Glu Ile Val Leu Thr Gln Ser Pro Ser Ser Leu Ser Ala Ser Val Gly
1               5                   10                  15
Asp Arg Val Thr Ile Thr Cys Arg Ala Ser Gln Gly Ile Ser Asn Tyr
            20                  25                  30
Leu Ala Trp Tyr Gln Gln Lys Pro Gly Lys Val Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro

65                  70                  75                  80
Glu Asp Val Ala Thr Tyr Tyr Cys Gln Lys Tyr Asn Ser Ala Pro His
                85                  90                  95
Thr Phe Gly Pro Gly Thr Lys Val Asp Ile Lys
            100                 105
<210>  259
<211>  360
<212>  DNA
<213>  human
<220>
<221>  CDS
<222>  (1)..(360)
<400>  259
cag gtg cag ctg gtg cag tct ggg gct gag gtg aag aag cct ggg tcc      48
Gln Val Gln Leu Val Gln Ser Gly Ala Glu Val Lys Lys Pro Gly Ser
1               5                   10                  15
tcg gtg aag ctc tcc tgc aag gct tct ggc ggc acc ctc cga agt tat      96
Ser Val Lys Leu Ser Cys Lys Ala Ser Gly Gly Thr Leu Arg Ser Tyr
            20                  25                  30
gct ctc agc tgg gtg cga cag gcc cct gga gaa ggg ctt gag tgg atg      144
Ala Leu Ser Trp Val Arg Gln Ala Pro Gly Glu Gly Leu Glu Trp Met
        35                  40                  45
gga ggg atc agc gcc atc ttt aat act gca act tac gca cag aac gtc      192
Gly Gly Ile Ser Ala Ile Phe Asn Thr Ala Thr Tyr Ala Gln Asn Val
    50                  55                  60
cag ggc aga gtc acg atc acc gcg gac gag tcc acg aac aca ttt tat      240
Gln Gly Arg Val Thr Ile Thr Ala Asp Glu Ser Thr Asn Thr Phe Tyr
65                  70                  75                  80
ttg gag gtg agc agc ctg aga tct gac gac acg gcc gtc tat tac tgt      288
Leu Glu Val Ser Ser Leu Arg Ser Asp Asp Thr Ala Val Tyr Tyr Cys
                85                  90                  95
gcg aca tcc ggg acc tac tac gtc tca ttt ttt gac tac tgg ggc cag      336
Ala Thr Ser Gly Thr Tyr Tyr Val Ser Phe Phe Asp Tyr Trp Gly Gln
            100                 105                 110
gga acc ctg gtc acc gtc tcg agc                                      360
Gly Thr Leu Val Thr Val Ser Ser
        115                 120
<210>  260
<211>  120
<212>  PRT
<213>  human
<400>  260
Gln Val Gln Leu Val Gln Ser Gly Ala Glu Val Lys Lys Pro Gly Ser
1               5                   10                  15
Ser Val Lys Leu Ser Cys Lys Ala Ser Gly Gly Thr Leu Arg Ser Tyr
            20                  25                  30
Ala Leu Ser Trp Val Arg Gln Ala Pro Gly Glu Gly Leu Glu Trp Met
        35                  40                  45
Gly Gly Ile Ser Ala Ile Phe Asn Thr Ala Thr Tyr Ala Gln Asn Val
    50                  55                  60
Gln Gly Arg Val Thr Ile Thr Ala Asp Glu Ser Thr Asn Thr Phe Tyr
65                  70                  75                  80
Leu Glu Val Ser Ser Leu Arg Ser Asp Asp Thr Ala Val Tyr Tyr Cys
                85                  90                  95
Ala Thr Ser Gly Thr Tyr Tyr Val Ser Phe Phe Asp Tyr Trp Gly Gln
            100                 105                 110
Gly Thr Leu Val Thr Val Ser Ser
        115                 120
<210>  261
<211>  321
<212>  DNA
<213>  human
<220>

<221>  CDS
<222>  (1)..(321)
<400>  261
gat att gtg ttg acg cag tct cct tcc acc ctg tct gca tct gtg gga      48
Asp Ile Val Leu Thr Gln Ser Pro Ser Thr Leu Ser Ala Ser Val Gly
1               5                   10                  15
gac aga gtc acc ctc act tgc cgg gcc agt cag agc att ggt agc ttg      96
Asp Arg Val Thr Leu Thr Cys Arg Ala Ser Gln Ser Ile Gly Ser Leu
            20                  25                  30
atg gcc tgg tat cag cag caa cca ggg aaa ccc cct aaa ctc ctg atc     144
Met Ala Trp Tyr Gln Gln Gln Pro Gly Lys Pro Pro Lys Leu Leu Ile
        35                  40                  45
tat agg gcg tct aat ttg gaa act ggt gtc cca tca agg ttc acc ggc     192
Tyr Arg Ala Ser Asn Leu Glu Thr Gly Val Pro Ser Arg Phe Thr Gly
    50                  55                  60
agt gga tct ggg aca gaa ttc gct ctc aca atc agc agc ctg cag cct     240
Ser Gly Ser Gly Thr Glu Phe Ala Leu Thr Ile Ser Ser Leu Gln Pro
65                  70                  75                  80
gat gat gtt gga acc tat tac tgc caa cac ttt aag act ttt tcc cgg     288
Asp Asp Val Gly Thr Tyr Tyr Cys Gln His Phe Lys Thr Phe Ser Arg
                85                  90                  95
acg ttc ggc caa ggg acc aag gtg gaa atc aaa                         321
Thr Phe Gly Gln Gly Thr Lys Val Glu Ile Lys
            100                 105
<210>  262
<211>  107
<212>  PRT
<213>  human
<400>  262
Asp Ile Val Leu Thr Gln Ser Pro Ser Thr Leu Ser Ala Ser Val Gly
1               5                   10                  15
Asp Arg Val Thr Leu Thr Cys Arg Ala Ser Gln Ser Ile Gly Ser Leu
            20                  25                  30
Met Ala Trp Tyr Gln Gln Gln Pro Gly Lys Pro Pro Lys Leu Leu Ile
        35                  40                  45
Tyr Arg Ala Ser Asn Leu Glu Thr Gly Val Pro Ser Arg Phe Thr Gly
    50                  55                  60
Ser Gly Ser Gly Thr Glu Phe Ala Leu Thr Ile Ser Ser Leu Gln Pro
65                  70                  75                  80
Asp Asp Val Gly Thr Tyr Tyr Cys Gln His Phe Lys Thr Phe Ser Arg
                85                  90                  95
Thr Phe Gly Gln Gly Thr Lys Val Glu Ile Lys
            100                 105
<210>  263
<211>  366
<212>  DNA
<213>  human
<220>
<221>  CDS
<222>  (1)..(366)
<400>  263
cag gta cag ctg cag cag tca ggg gct gag gtg aag cag cct ggg tcc      48
Gln Val Gln Leu Gln Gln Ser Gly Ala Glu Val Lys Gln Pro Gly Ser
1               5                   10                  15
tcg gtg aag gtc tcc tgc aag gct tct gga ggc atc ttc agg agc aac      96
Ser Val Lys Val Ser Cys Lys Ala Ser Gly Gly Ile Phe Arg Ser Asn
            20                  25                  30
gct atc agc tgg gtg cga cag gcc cct gga caa ggg ctt gag tgg atg     144
Ala Ile Ser Trp Val Arg Gln Ala Pro Gly Gln Gly Leu Glu Trp Met
        35                  40                  45
gga ggc atc gtc gct ttg ttt ggt aca gca aac tac gca cag aag ttc     192
Gly Gly Ile Val Ala Leu Phe Gly Thr Ala Asn Tyr Ala Gln Lys Phe
    50                  55                  60

cag ggc aga gtc acg att acc gcg gac gaa tct acg agt aca gtc tac 240
Gln Gly Arg Val Thr Ile Thr Ala Asp Glu Ser Thr Ser Thr Val Tyr
65                  70                  75                  80
atg gaa ctg agc agc ctg aga tct gag gac acg gcc cgc tat tac tgt 288
Met Glu Leu Ser Ser Leu Arg Ser Glu Asp Thr Ala Arg Tyr Tyr Cys
                85                  90                  95
gcg aga aat agt ggc tac cac atc tct ggg ttc tat ctt gac tac tgg 336
Ala Arg Asn Ser Gly Tyr His Ile Ser Gly Phe Tyr Leu Asp Tyr Trp
            100                 105                 110
ggc cag gga acc ctg gtc acc gtc tcg agc 366
Gly Gln Gly Thr Leu Val Thr Val Ser Ser
        115                 120
<210> 264
<211> 122
<212> PRT
<213> human
<400> 264
Gln Val Gln Leu Gln Gln Ser Gly Ala Glu Val Lys Gln Pro Gly Ser
1               5                   10                  15
Ser Val Lys Val Ser Cys Lys Ala Ser Gly Gly Ile Phe Arg Ser Asn
            20                  25                  30
Ala Ile Ser Trp Val Arg Gln Ala Pro Gly Gln Gly Leu Glu Trp Met
        35                  40                  45
Gly Gly Ile Val Ala Leu Phe Gly Thr Ala Asn Tyr Ala Gln Lys Phe
    50                  55                  60
Gln Gly Arg Val Thr Ile Thr Ala Asp Glu Ser Thr Ser Thr Val Tyr
65                  70                  75                  80
Met Glu Leu Ser Ser Leu Arg Ser Glu Asp Thr Ala Arg Tyr Tyr Cys
                85                  90                  95
Ala Arg Asn Ser Gly Tyr His Ile Ser Gly Phe Tyr Leu Asp Tyr Trp
            100                 105                 110
Gly Gln Gly Thr Leu Val Thr Val Ser Ser
        115                 120
<210> 265
<211> 321
<212> DNA
<213> human
<220>
<221> CDS
<222> (1)..(321)
<400> 265
gaa att gtg ttg acg cag tct cca tcc tcc ctg tct gca tct gta gga 48
Glu Ile Val Leu Thr Gln Ser Pro Ser Ser Leu Ser Ala Ser Val Gly
1               5                   10                  15
gac aga gtc acc atc act tgc cgg gcg agt cag ggc att agc aat tat 96
Asp Arg Val Thr Ile Thr Cys Arg Ala Ser Gln Gly Ile Ser Asn Tyr
            20                  25                  30
tta gcc tgg tat cag cag agg cca ggg aaa gtt cct aaa ctc ctg atc 144
Leu Ala Trp Tyr Gln Gln Arg Pro Gly Lys Val Pro Lys Leu Leu Ile
        35                  40                  45
tat act gct tcc act ttg cta tca ggg gtc cca tct cgg ttc agt ggc 192
Tyr Thr Ala Ser Thr Leu Leu Ser Gly Val Pro Ser Arg Phe Ser Gly
    50                  55                  60
ggt gga tct ggg aca gat tac act ctc acc atc agc agc ctg cag cct 240
Gly Gly Ser Gly Thr Asp Tyr Thr Leu Thr Ile Ser Ser Leu Gln Pro
65                  70                  75                  80
gaa gat gtt gca act tat tac tgt caa gag tat aaa agt gtc cct ctc 288
Glu Asp Val Ala Thr Tyr Tyr Cys Gln Glu Tyr Lys Ser Val Pro Leu
                85                  90                  95
act ttc ggc cct ggg acc aaa gtg gag atc aaa 321
Thr Phe Gly Pro Gly Thr Lys Val Glu Ile Lys
            100                 105
<210> 266

<211>  107
<212>  PRT
<213>  human
<400>  266
Glu Ile Val Leu Thr Gln Ser Pro Ser Ser Leu Ser Ala Ser Val Gly
1               5                   10                  15
Asp Arg Val Thr Ile Thr Cys Arg Ala Ser Gln Gly Ile Ser Asn Tyr
            20                  25                  30
Leu Ala Trp Tyr Gln Gln Arg Pro Gly Lys Val Pro Lys Leu Leu Ile
        35                  40                  45
Tyr Thr Ala Ser Thr Leu Leu Ser Gly Val Pro Ser Arg Phe Ser Gly
    50                  55                  60
Gly Gly Ser Gly Thr Asp Tyr Thr Leu Thr Ile Ser Ser Leu Gln Pro
65                  70                  75                  80
Glu Asp Val Ala Thr Tyr Tyr Cys Gln Glu Tyr Lys Ser Val Pro Leu
                85                  90                  95
Thr Phe Gly Pro Gly Thr Lys Val Glu Ile Lys
            100                 105
<210>  267
<211>  366
<212>  DNA
<213>  human
<220>
<221>  CDS
<222>  (1)..(366)
<400>  267
cag gtg cag ctg gtg cag tct ggg gct gag gtg agg aag cct ggg tcc       48
Gln Val Gln Leu Val Gln Ser Gly Ala Glu Val Arg Lys Pro Gly Ser
1               5                   10                  15
tcg gtg aag gtc tcc tgc aag gcg tct gga gtc atc ttc atc aag ttt       96
Ser Val Lys Val Ser Cys Lys Ala Ser Gly Val Ile Phe Ile Lys Phe
            20                  25                  30
gct att agc tgg gtg cga cag gcc cct gga caa ggg ctt gag tgg atg      144
Ala Ile Ser Trp Val Arg Gln Ala Pro Gly Gln Gly Leu Glu Trp Met
        35                  40                  45
ggg ggg atc atc cct atg ttt ggt aca aca aac tac gca cag aag ttc      192
Gly Gly Ile Ile Pro Met Phe Gly Thr Thr Asn Tyr Ala Gln Lys Phe
    50                  55                  60
cag ggc aga gtc acg att acc gcg gac gga tcc acc aac aca gtc tac      240
Gln Gly Arg Val Thr Ile Thr Ala Asp Gly Ser Thr Asn Thr Val Tyr
65                  70                  75                  80
atg gag tta agc agc ctg aca tct gag gac acg gcc gtg tat tac tgt      288
Met Glu Leu Ser Ser Leu Thr Ser Glu Asp Thr Ala Val Tyr Tyr Cys
                85                  90                  95
gcg aaa gag gag gga tat tac tat ggt tcg ggg ccg ctt gac tcc tgg      336
Ala Lys Glu Glu Gly Tyr Tyr Tyr Gly Ser Gly Pro Leu Asp Ser Trp
            100                 105                 110
ggc cag gga acc ctg gtc acc gtc tcg agc                              366
Gly Gln Gly Thr Leu Val Thr Val Ser Ser
        115                 120
<210>  268
<211>  122
<212>  PRT
<213>  human
<400>  268
Gln Val Gln Leu Val Gln Ser Gly Ala Glu Val Arg Lys Pro Gly Ser
1               5                   10                  15
Ser Val Lys Val Ser Cys Lys Ala Ser Gly Val Ile Phe Ile Lys Phe
            20                  25                  30
Ala Ile Ser Trp Val Arg Gln Ala Pro Gly Gln Gly Leu Glu Trp Met
        35                  40                  45
Gly Gly Ile Ile Pro Met Phe Gly Thr Thr Asn Tyr Ala Gln Lys Phe
    50                  55                  60

Gln Gly Arg Val Thr Ile Thr Ala Asp Gly Ser Thr Asn Thr Val Tyr
65                  70                  75                  80
Met Glu Leu Ser Ser Leu Thr Ser Glu Asp Thr Ala Val Tyr Tyr Cys
                85                  90                  95
Ala Lys Glu Glu Gly Tyr Tyr Tyr Gly Ser Gly Pro Leu Asp Ser Trp
            100                 105                 110
Gly Gln Gly Thr Leu Val Thr Val Ser Ser
        115                 120
<210>  269
<211>  339
<212>  DNA
<213>  human
<220>
<221>  CDS
<222>  (1)..(339)
<400>  269
gac atc gtg atg acc cag tct cca gac ttc ctg tct gtg tct ctg ggc      48
Asp Ile Val Met Thr Gln Ser Pro Asp Phe Leu Ser Val Ser Leu Gly
1               5                   10                  15
gag agg gtc acc atc aac tgc aag tcc agt cag act gtt tta aac agg      96
Glu Arg Val Thr Ile Asn Cys Lys Ser Ser Gln Thr Val Leu Asn Arg
            20                  25                  30
tcc aat aat aag aat tac ctt gcc tgg tac cag aag aag cca ggg cag      144
Ser Asn Asn Lys Asn Tyr Leu Ala Trp Tyr Gln Lys Lys Pro Gly Gln
        35                  40                  45
cct cct agg ttg ctc ctt tac tgg tct tct acc cgg gaa tcc ggg gtc      192
Pro Pro Arg Leu Leu Leu Tyr Trp Ser Ser Thr Arg Glu Ser Gly Val
    50                  55                  60
cct gat cgt ttc agt ggc agc ggg tct gag act gat ttc gct ctc acc      240
Pro Asp Arg Phe Ser Gly Ser Gly Ser Glu Thr Asp Phe Ala Leu Thr
65                  70                  75                  80
atc acc aac ctg cag gct gaa gat ttg gca gtt tac ttc tgt cag cag      288
Ile Thr Asn Leu Gln Ala Glu Asp Leu Ala Val Tyr Phe Cys Gln Gln
                85                  90                  95
ttt tat gat atg cct gtc act ttc ggc gga ggg acc aag ctg gag atc      336
Phe Tyr Asp Met Pro Val Thr Phe Gly Gly Gly Thr Lys Leu Glu Ile
            100                 105                 110
aaa                                                                  339
Lys

<210>  270
<211>  113
<212>  PRT
<213>  human
<400>  270
Asp Ile Val Met Thr Gln Ser Pro Asp Phe Leu Ser Val Ser Leu Gly
1               5                   10                  15
Glu Arg Val Thr Ile Asn Cys Lys Ser Ser Gln Thr Val Leu Asn Arg
            20                  25                  30
Ser Asn Asn Lys Asn Tyr Leu Ala Trp Tyr Gln Lys Lys Pro Gly Gln
        35                  40                  45
Pro Pro Arg Leu Leu Leu Tyr Trp Ser Ser Thr Arg Glu Ser Gly Val
    50                  55                  60
Pro Asp Arg Phe Ser Gly Ser Gly Ser Glu Thr Asp Phe Ala Leu Thr
65                  70                  75                  80
Ile Thr Asn Leu Gln Ala Glu Asp Leu Ala Val Tyr Phe Cys Gln Gln
                85                  90                  95
Phe Tyr Asp Met Pro Val Thr Phe Gly Gly Gly Thr Lys Leu Glu Ile
            100                 105                 110
Lys

<210>  271
<211>  363

<212>  DNA
<213>  human
<220>
<221>  CDS
<222>  (1)..(363)
<400>  271
cag gtg cag ctg gtg cag tct ggg gct gac gtg aag aag gct ggg tcc      48
Gln Val Gln Leu Val Gln Ser Gly Ala Asp Val Lys Lys Ala Gly Ser
1               5                   10                  15
tcg gtg aag gtc tcc tgc aag gct tcc gga cgc acc ttc ggc aat tat      96
Ser Val Lys Val Ser Cys Lys Ala Ser Gly Arg Thr Phe Gly Asn Tyr
            20                  25                  30
gct atc agc tgg gtg cga cag gcc cct gga caa gga ctt gag tgg atg      144
Ala Ile Ser Trp Val Arg Gln Ala Pro Gly Gln Gly Leu Glu Trp Met
        35                  40                  45
gga ggg atc acc cct ata ttt gga gca gca aac tac gca cag aag ttc      192
Gly Gly Ile Thr Pro Ile Phe Gly Ala Ala Asn Tyr Ala Gln Lys Phe
    50                  55                  60
cag ggc aga gtc act att tcc gcg gac tta tcc acg aga atg gtc tat      240
Gln Gly Arg Val Thr Ile Ser Ala Asp Leu Ser Thr Arg Met Val Tyr
65                  70                  75                  80
atg gac ttg agc agc ctg aga tct gac gac acg gcc ttc tat tac tgt      288
Met Asp Leu Ser Ser Leu Arg Ser Asp Asp Thr Ala Phe Tyr Tyr Cys
                85                  90                  95
gcg gga cat ccc atg tat cac tat ggt tcg gcg atg gac tac tgg ggc      336
Ala Gly His Pro Met Tyr His Tyr Gly Ser Ala Met Asp Tyr Trp Gly
            100                 105                 110
cag ggc acc ctg gtc acc gtc tcg agc                                  363
Gln Gly Thr Leu Val Thr Val Ser Ser
        115                 120
<210>  272
<211>  121
<212>  PRT
<213>  human
<400>  272
Gln Val Gln Leu Val Gln Ser Gly Ala Asp Val Lys Lys Ala Gly Ser
1               5                   10                  15
Ser Val Lys Val Ser Cys Lys Ala Ser Gly Arg Thr Phe Gly Asn Tyr
            20                  25                  30
Ala Ile Ser Trp Val Arg Gln Ala Pro Gly Gln Gly Leu Glu Trp Met
        35                  40                  45
Gly Gly Ile Thr Pro Ile Phe Gly Ala Ala Asn Tyr Ala Gln Lys Phe
    50                  55                  60
Gln Gly Arg Val Thr Ile Ser Ala Asp Leu Ser Thr Arg Met Val Tyr
65                  70                  75                  80
Met Asp Leu Ser Ser Leu Arg Ser Asp Asp Thr Ala Phe Tyr Tyr Cys
                85                  90                  95
Ala Gly His Pro Met Tyr His Tyr Gly Ser Ala Met Asp Tyr Trp Gly
            100                 105                 110
Gln Gly Thr Leu Val Thr Val Ser Ser
        115                 120
<210>  273
<211>  333
<212>  DNA
<213>  human
<220>
<221>  CDS
<222>  (1)..(333)
<400>  273
cag tct gtg ttg acg cag ccg ccc tca gcg tct ggg acc ccc gga cag      48
Gln Ser Val Leu Thr Gln Pro Pro Ser Ala Ser Gly Thr Pro Gly Gln
1               5                   10                  15
agg gtc agc atc tct tgt ttt gga agc aga tcc aat gtc gga agc agt      96

Arg Val Ser Ile Ser Cys Phe Gly Ser Arg Ser Asn Val Gly Ser Ser
            20                  25                  30
tct gta aac tgg tac cag cag ctc cca gga agg gcc ccc aaa ctc ctc        144
Ser Val Asn Trp Tyr Gln Gln Leu Pro Gly Arg Ala Pro Lys Leu Leu
        35                  40                  45
att caa aga aat gat cag cgg ccc tca ggg gtc cct gac cga ttc tct        192
Ile Gln Arg Asn Asp Gln Arg Pro Ser Gly Val Pro Asp Arg Phe Ser
    50                  55                  60
ggc tcc aag tct ggc acg tca gcc tcc ctg gcc atc agt ggg ctc cag        240
Gly Ser Lys Ser Gly Thr Ser Ala Ser Leu Ala Ile Ser Gly Leu Gln
65                  70                  75                  80
tct gac gat gag tct act tat tat tgt gca gcc tgg gat gac agt gtg        288
Ser Asp Asp Glu Ser Thr Tyr Tyr Cys Ala Ala Trp Asp Asp Ser Val
                85                  90                  95
aag tcc gtg gtg ttc ggc gga ggg acg cag ctg acc gtc cta ggt            333
Lys Ser Val Val Phe Gly Gly Gly Thr Gln Leu Thr Val Leu Gly
            100                 105                 110
<210>  274
<211>  111
<212>  PRT
<213>  human
<400>  274
Gln Ser Val Leu Thr Gln Pro Pro Ser Ala Ser Gly Thr Pro Gly Gln
1               5                   10                  15
Arg Val Ser Ile Ser Cys Phe Gly Ser Arg Ser Asn Val Gly Ser Ser
            20                  25                  30
Ser Val Asn Trp Tyr Gln Gln Leu Pro Gly Arg Ala Pro Lys Leu Leu
        35                  40                  45
Ile Gln Arg Asn Asp Gln Arg Pro Ser Gly Val Pro Asp Arg Phe Ser
    50                  55                  60
Gly Ser Lys Ser Gly Thr Ser Ala Ser Leu Ala Ile Ser Gly Leu Gln
65                  70                  75                  80
Ser Asp Asp Glu Ser Thr Tyr Tyr Cys Ala Ala Trp Asp Asp Ser Val
                85                  90                  95
Lys Ser Val Val Phe Gly Gly Gly Thr Gln Leu Thr Val Leu Gly
            100                 105                 110
<210>  275
<211>  360
<212>  DNA
<213>  human
<220>
<221>  CDS
<222>  (1)..(360)
<400>  275
cag gtg cag ctg gtg cag tct ggg tct gag gtg aag aag cct ggg tcc        48
Gln Val Gln Leu Val Gln Ser Gly Ser Glu Val Lys Lys Pro Gly Ser
1               5                   10                  15
tcg gtg aag gtc tcc tgc aag gct tct gga ggc atc ttc aac agc tat        96
Ser Val Lys Val Ser Cys Lys Ala Ser Gly Gly Ile Phe Asn Ser Tyr
            20                  25                  30
ggt atc agc tgg gtg cga cag gcc cct gga caa ggg ctt gag tgg atg        144
Gly Ile Ser Trp Val Arg Gln Ala Pro Gly Gln Gly Leu Glu Trp Met
        35                  40                  45
ggg ggg atc ctc gct atc ttt gga aca aca aac tac gca cag aag ttc        192
Gly Gly Ile Leu Ala Ile Phe Gly Thr Thr Asn Tyr Ala Gln Lys Phe
    50                  55                  60
cag ggc aga gtc acg att acc gcg gac gac tcc tcg aaa aca gtg tac        240
Gln Gly Arg Val Thr Ile Thr Ala Asp Asp Ser Ser Lys Thr Val Tyr
65                  70                  75                  80
atg gag ctg agc agc ctg aca tct gag gac acg gcc gtg tat tac tgt        288
Met Glu Leu Ser Ser Leu Thr Ser Glu Asp Thr Ala Val Tyr Tyr Cys
                85                  90                  95
gcg agg ggg agt act tat tac tcc agt tac ttt gac cag tgg ggc cag        336

Ala Arg Gly Ser Thr Tyr Tyr Ser Ser Tyr Phe Asp Gln Trp Gly Gln
            100                 105                 110
gga acc ctg gtc acc gtc tcg agc 360
Gly Thr Leu Val Thr Val Ser Ser
        115                 120
<210> 276
<211> 120
<212> PRT
<213> human
<400> 276
Gln Val Gln Leu Val Gln Ser Gly Ser Glu Val Lys Lys Pro Gly Ser
1               5                   10                  15
Ser Val Lys Val Ser Cys Lys Ala Ser Gly Gly Ile Phe Asn Ser Tyr
            20                  25                  30
Gly Ile Ser Trp Val Arg Gln Ala Pro Gly Gln Gly Leu Glu Trp Met
        35                  40                  45
Gly Gly Ile Leu Ala Ile Phe Gly Thr Thr Asn Tyr Ala Gln Lys Phe
    50                  55                  60
Gln Gly Arg Val Thr Ile Thr Ala Asp Asp Ser Ser Lys Thr Val Tyr
65                  70                  75                  80
Met Glu Leu Ser Ser Leu Thr Ser Glu Asp Thr Ala Val Tyr Tyr Cys
                85                  90                  95
Ala Arg Gly Ser Thr Tyr Tyr Ser Ser Tyr Phe Asp Gln Trp Gly Gln
            100                 105                 110
Gly Thr Leu Val Thr Val Ser Ser
        115                 120
<210> 277
<211> 321
<212> DNA
<213> human
<220>
<221> CDS
<222> (1)..(321)
<400> 277
gac atc cag atg acc cag tct cca tcc tcc ctg tct gca tct gta gga 48
Asp Ile Gln Met Thr Gln Ser Pro Ser Ser Leu Ser Ala Ser Val Gly
1               5                   10                  15
gac aga gtc acc atc act tgc cgg gcg agt cag ggc atc cgc aat tct 96
Asp Arg Val Thr Ile Thr Cys Arg Ala Ser Gln Gly Ile Arg Asn Ser
            20                  25                  30
ttg gcc tgg tat cag cag aaa cca ggg caa gtt cct aag ctc cta atc 144
Leu Ala Trp Tyr Gln Gln Lys Pro Gly Gln Val Pro Lys Leu Leu Ile
        35                  40                  45
tat tct gca acc act ttg cga tca ggg gtc cca tac cgc ttc agt ggc 192
Tyr Ser Ala Thr Thr Leu Arg Ser Gly Val Pro Tyr Arg Phe Ser Gly
    50                  55                  60
agt ggg tct ggg aca gat ttc act ctc acc atc agc ggc ctg cag cct 240
Ser Gly Ser Gly Thr Asp Phe Thr Leu Thr Ile Ser Gly Leu Gln Pro
65                  70                  75                  80
gaa gat gtt gga act tat ttc tgt cac agg tat gac agt gcc ccc ctc 288
Glu Asp Val Gly Thr Tyr Phe Cys His Arg Tyr Asp Ser Ala Pro Leu
                85                  90                  95
aca ttc ggc gga ggg acc agg gtg gcg ctc aag 321
Thr Phe Gly Gly Gly Thr Arg Val Ala Leu Lys
            100                 105
<210> 278
<211> 107
<212> PRT
<213> human
<400> 278
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Gly Ile Arg Asn Ser

20                  25                  30
Leu Ala Trp Tyr Gln Gln Lys Pro Gly Gln Val Pro Lys Leu Leu Ile
        35                  40                  45
Tyr Ser Ala Thr Thr Leu Arg Ser Gly Val Pro Tyr Arg Phe Ser Gly
    50                  55                  60
Ser Gly Ser Gly Thr Asp Phe Thr Leu Thr Ile Ser Gly Leu Gln Pro
65                  70                  75                  80
Glu Asp Val Gly Thr Tyr Phe Cys His Arg Tyr Asp Ser Ala Pro Leu
                85                  90                  95
Thr Phe Gly Gly Gly Thr Arg Val Ala Leu Lys
            100                 105
<210>  279
<211>  372
<212>  DNA
<213>  human
<220>
<221>  CDS
<222>  (1)..(372)
<400>  279
cag gtg cag ctg gtg caa tct ggg tct gag gtg aag aag cct ggg tcc      48
Gln Val Gln Leu Val Gln Ser Gly Ser Glu Val Lys Lys Pro Gly Ser
1               5                   10                  15
tcg gtg aag gtc tcc tgc aag gct tct aga ggc atc ttc agc agt tat      96
Ser Val Lys Val Ser Cys Lys Ala Ser Arg Gly Ile Phe Ser Ser Tyr
            20                  25                  30
gct atc agc tgg gtg cga cag gcc cct gga caa ggg ctt gag tgg atg      144
Ala Ile Ser Trp Val Arg Gln Ala Pro Gly Gln Gly Leu Glu Trp Met
        35                  40                  45
gga gcg gtc atc cct atg ttt ggc acg cta aag tat gca gag aat ttc      192
Gly Ala Val Ile Pro Met Phe Gly Thr Leu Lys Tyr Ala Glu Asn Phe
    50                  55                  60
cag ggc aga atc acg ata tcc gcg gac aaa tcc atg agc gca gcc tac      240
Gln Gly Arg Ile Thr Ile Ser Ala Asp Lys Ser Met Ser Ala Ala Tyr
65                  70                  75                  80
atg gag ctg agc agc ctg aga tct gag gac acg gcc gta tat tac tgt      288
Met Glu Leu Ser Ser Leu Arg Ser Glu Asp Thr Ala Val Tyr Tyr Cys
                85                  90                  95
gcg aga aat tac tat ggt tcg ggg act tat ttc aat gat gct ttt gat      336
Ala Arg Asn Tyr Tyr Gly Ser Gly Thr Tyr Phe Asn Asp Ala Phe Asp
            100                 105                 110
att tgg ggc caa ggg aca atg gtc acc gtc tcg agc                      372
Ile Trp Gly Gln Gly Thr Met Val Thr Val Ser Ser
        115                 120
<210>  280
<211>  124
<212>  PRT
<213>  human
<400>  280
Gln Val Gln Leu Val Gln Ser Gly Ser Glu Val Lys Lys Pro Gly Ser
1               5                   10                  15
Ser Val Lys Val Ser Cys Lys Ala Ser Arg Gly Ile Phe Ser Ser Tyr
            20                  25                  30
Ala Ile Ser Trp Val Arg Gln Ala Pro Gly Gln Gly Leu Glu Trp Met
        35                  40                  45
Gly Ala Val Ile Pro Met Phe Gly Thr Leu Lys Tyr Ala Glu Asn Phe
    50                  55                  60
Gln Gly Arg Ile Thr Ile Ser Ala Asp Lys Ser Met Ser Ala Ala Tyr
65                  70                  75                  80
Met Glu Leu Ser Ser Leu Arg Ser Glu Asp Thr Ala Val Tyr Tyr Cys
                85                  90                  95
Ala Arg Asn Tyr Tyr Gly Ser Gly Thr Tyr Phe Asn Asp Ala Phe Asp
            100                 105                 110
Ile Trp Gly Gln Gly Thr Met Val Thr Val Ser Ser

115                 120
<210>  281
<211>  336
<212>  DNA
<213>  human
<220>
<221>  CDS
<222>  (1)..(336)
<400>  281
cag tct gtg ttg acg cag ccg ccc tca ggg tct ggg gcc cca ggg cag       48
Gln Ser Val Leu Thr Gln Pro Pro Ser Gly Ser Gly Ala Pro Gly Gln
1               5                   10                  15
agg gtc acc atc tcc tgc act ggg ggc aac tcc aac atc ggg gca ggt       96
Arg Val Thr Ile Ser Cys Thr Gly Gly Asn Ser Asn Ile Gly Ala Gly
            20                  25                  30
tat gat gta aat ggg tac cag caa ctt cca gga aaa gcc cct aaa ctc      144
Tyr Asp Val Asn Gly Tyr Gln Gln Leu Pro Gly Lys Ala Pro Lys Leu
        35                  40                  45
ctc att tat gga aac aac aat cgg ccc tca ggg gtc cct gac cga ttc      192
Leu Ile Tyr Gly Asn Asn Asn Arg Pro Ser Gly Val Pro Asp Arg Phe
    50                  55                  60
tct ggg tcc aag tca ggc acc tca gcc tcc ctg gcc atc act ggg ctc      240
Ser Gly Ser Lys Ser Gly Thr Ser Ala Ser Leu Ala Ile Thr Gly Leu
65                  70                  75                  80
cag gtt gag gat gag gct cac tat tac tgc cag tca tat gac aat ggc      288
Gln Val Glu Asp Glu Ala His Tyr Tyr Cys Gln Ser Tyr Asp Asn Gly
                85                  90                  95
ctg agt ggc tcg gtt ttt ggc gga ggg acc cag ctg acc gtc cta ggt      336
Leu Ser Gly Ser Val Phe Gly Gly Gly Thr Gln Leu Thr Val Leu Gly
            100                 105                 110
<210>  282
<211>  112
<212>  PRT
<213>  human
<400>  282
Gln Ser Val Leu Thr Gln Pro Pro Ser Gly Ser Gly Ala Pro Gly Gln
1               5                   10                  15
Arg Val Thr Ile Ser Cys Thr Gly Gly Asn Ser Asn Ile Gly Ala Gly
            20                  25                  30
Tyr Asp Val Asn Gly Tyr Gln Gln Leu Pro Gly Lys Ala Pro Lys Leu
        35                  40                  45
Leu Ile Tyr Gly Asn Asn Asn Arg Pro Ser Gly Val Pro Asp Arg Phe
    50                  55                  60
Ser Gly Ser Lys Ser Gly Thr Ser Ala Ser Leu Ala Ile Thr Gly Leu
65                  70                  75                  80
Gln Val Glu Asp Glu Ala His Tyr Tyr Cys Gln Ser Tyr Asp Asn Gly
                85                  90                  95
Leu Ser Gly Ser Val Phe Gly Gly Gly Thr Gln Leu Thr Val Leu Gly
            100                 105                 110
<210>  283
<211>  363
<212>  DNA
<213>  human
<220>
<221>  CDS
<222>  (1)..(363)
<400>  283
cag gtg cag ctg gtg cag tct ggg gct gac gtg aag aag cct ggg tcc       48
Gln Val Gln Leu Val Gln Ser Gly Ala Asp Val Lys Lys Pro Gly Ser
1               5                   10                  15
tcg gtg agg gtc tcc tgc aag gct tct gga cgc acc ttc ggc aat tat       96
Ser Val Arg Val Ser Cys Lys Ala Ser Gly Arg Thr Phe Gly Asn Tyr
            20                  25                  30

gct atc agc tgg gtg cga cag gcc cct gga caa gga ctt gag tgg atg 144
Ala Ile Ser Trp Val Arg Gln Ala Pro Gly Gln Gly Leu Glu Trp Met
        35                  40                  45
gga ggg atc acc cct atc ttt gga gca gca aac tac gca cag aag ttc 192
Gly Gly Ile Thr Pro Ile Phe Gly Ala Ala Asn Tyr Ala Gln Lys Phe
    50                  55                  60
cag ggc aga gtc act att tcc gcg gac tta tcc acg act atg gtc tac 240
Gln Gly Arg Val Thr Ile Ser Ala Asp Leu Ser Thr Thr Met Val Tyr
65                  70                  75                  80
atg gac ttg agc agc ctg aga tct gac gac acg gcc ttc tat tac tgt 288
Met Asp Leu Ser Ser Leu Arg Ser Asp Asp Thr Ala Phe Tyr Tyr Cys
                85                  90                  95
gcg gga cat cca acg tat tac tat ggt tcg ccg atg gac tac tgg ggc 336
Ala Gly His Pro Thr Tyr Tyr Tyr Gly Ser Pro Met Asp Tyr Trp Gly
            100                 105                 110
cag gga acc ctg gtc acc gtc tcg agc 363
Gln Gly Thr Leu Val Thr Val Ser Ser
        115                 120
<210> 284
<211> 121
<212> PRT
<213> human
<400> 284
Gln Val Gln Leu Val Gln Ser Gly Ala Asp Val Lys Lys Pro Gly Ser
1               5                   10                  15
Ser Val Arg Val Ser Cys Lys Ala Ser Gly Arg Thr Phe Gly Asn Tyr
            20                  25                  30
Ala Ile Ser Trp Val Arg Gln Ala Pro Gly Gln Gly Leu Glu Trp Met
        35                  40                  45
Gly Gly Ile Thr Pro Ile Phe Gly Ala Ala Asn Tyr Ala Gln Lys Phe
    50                  55                  60
Gln Gly Arg Val Thr Ile Ser Ala Asp Leu Ser Thr Thr Met Val Tyr
65                  70                  75                  80
Met Asp Leu Ser Ser Leu Arg Ser Asp Asp Thr Ala Phe Tyr Tyr Cys
                85                  90                  95
Ala Gly His Pro Thr Tyr Tyr Tyr Gly Ser Pro Met Asp Tyr Trp Gly
            100                 105                 110
Gln Gly Thr Leu Val Thr Val Ser Ser
        115                 120
<210> 285
<211> 321
<212> DNA
<213> human
<220>
<221> CDS
<222> (1)..(321)
<400> 285
gaa att gtg ttg acg cag tct cca tcc tcc ctg tct gca tct gta gga 48
Glu Ile Val Leu Thr Gln Ser Pro Ser Ser Leu Ser Ala Ser Val Gly
1               5                   10                  15
gac aga gtc acc atc act tgc cgg gca agt cag agt att gcc acc tat 96
Asp Arg Val Thr Ile Thr Cys Arg Ala Ser Gln Ser Ile Ala Thr Tyr
            20                  25                  30
tta aat tgg tat cag cag aaa cca ggc aag gcc cct aac ctc ctg atc 144
Leu Asn Trp Tyr Gln Gln Lys Pro Gly Lys Ala Pro Asn Leu Leu Ile
        35                  40                  45
tat gct gca tcc aat ttg caa agt ggg gtc cca tcc cgg ttc agt ggc 192
Tyr Ala Ala Ser Asn Leu Gln Ser Gly Val Pro Ser Arg Phe Ser Gly
    50                  55                  60
agt gga tct ggg aca gat ttc act ctc acc atc agc agt ctg cag cct 240
Ser Gly Ser Gly Thr Asp Phe Thr Leu Thr Ile Ser Ser Leu Gln Pro
65                  70                  75                  80
gaa gat ttt gcg act tac gtc tgt cta cag agt tac agt gcc act ctc 288

Glu Asp Phe Ala Thr Tyr Val Cys Leu Gln Ser Tyr Ser Ala Thr Leu
                85                  90                  95
act ttc ggc gga ggg acc aag gtg gag atc aaa                         321
Thr Phe Gly Gly Gly Thr Lys Val Glu Ile Lys
            100                 105
<210>  286
<211>  107
<212>  PRT
<213>  human
<400>  286
Glu Ile Val Leu Thr Gln Ser Pro Ser Ser Leu Ser Ala Ser Val Gly
1               5                   10                  15
Asp Arg Val Thr Ile Thr Cys Arg Ala Ser Gln Ser Ile Ala Thr Tyr
            20                  25                  30
Leu Asn Trp Tyr Gln Gln Lys Pro Gly Lys Ala Pro Asn Leu Leu Ile
        35                  40                  45
Tyr Ala Ala Ser Asn Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Val Cys Leu Gln Ser Tyr Ser Ala Thr Leu
                85                  90                  95
Thr Phe Gly Gly Gly Thr Lys Val Glu Ile Lys
            100                 105
<210>  287
<211>  360
<212>  DNA
<213>  human
<220>
<221>  CDS
<222>  (1)..(360)
<400>  287
cag gtg cag ctg gtg cag tct ggg gct gag gtg aag aag cct ggg tcc      48
Gln Val Gln Leu Val Gln Ser Gly Ala Glu Val Lys Lys Pro Gly Ser
1               5                   10                  15
tcg gtg aag gtc tcc tgc aag gct tcc gga gac ata ttc aac aag ttt      96
Ser Val Lys Val Ser Cys Lys Ala Ser Gly Asp Ile Phe Asn Lys Phe
            20                  25                  30
gct att act tgg gtg cga cag gcc cct gga caa ggg ctt gag tgg atg     144
Ala Ile Thr Trp Val Arg Gln Ala Pro Gly Gln Gly Leu Glu Trp Met
        35                  40                  45
gga ggg atc acc gcc ctc ttt gcc aca aca agc tac gca cag aag ttc     192
Gly Gly Ile Thr Ala Leu Phe Ala Thr Thr Ser Tyr Ala Gln Lys Phe
    50                  55                  60
cag gac aga gtc acg att aat tgg gac gaa tct acg acc act gcc ttc     240
Gln Asp Arg Val Thr Ile Asn Trp Asp Glu Ser Thr Thr Thr Ala Phe
65                  70                  75                  80
atg gag ctg agc agc ctg aga ttt gag gac acg gcc gtc tat tac tgt     288
Met Glu Leu Ser Ser Leu Arg Phe Glu Asp Thr Ala Val Tyr Tyr Cys
                85                  90                  95
gcg aga gga cca aat tac tat gag acc tac ctt gac aac tgg ggc cag     336
Ala Arg Gly Pro Asn Tyr Tyr Glu Thr Tyr Leu Asp Asn Trp Gly Gln
            100                 105                 110
ggc acc ctg gtc acc gtc tcg agc                                     360
Gly Thr Leu Val Thr Val Ser Ser
        115                 120
<210>  288
<211>  120
<212>  PRT
<213>  human
<400>  288
Gln Val Gln Leu Val Gln Ser Gly Ala Glu Val Lys Lys Pro Gly Ser
1               5                   10                  15

Ser Val Lys Val Ser Cys Lys Ala Ser Gly Asp Ile Phe Asn Lys Phe
            20                  25                  30
Ala Ile Thr Trp Val Arg Gln Ala Pro Gly Gln Gly Leu Glu Trp Met
        35                  40                  45
Gly Gly Ile Thr Ala Leu Phe Ala Thr Thr Ser Tyr Ala Gln Lys Phe
    50                  55                  60
Gln Asp Arg Val Thr Ile Asn Trp Asp Glu Ser Thr Thr Thr Ala Phe
65                  70                  75                  80
Met Glu Leu Ser Ser Leu Arg Phe Glu Asp Thr Ala Val Tyr Tyr Cys
                85                  90                  95
Ala Arg Gly Pro Asn Tyr Tyr Glu Thr Tyr Leu Asp Asn Trp Gly Gln
            100                 105                 110
Gly Thr Leu Val Thr Val Ser Ser
        115                 120
<210>  289
<211>  327
<212>  DNA
<213>  human
<220>
<221>  CDS
<222>  (1)..(327)
<400>  289
gaa att gtg ttg acg cag tct cct acc atc ttg tct gcg tct gta gga       48
Glu Ile Val Leu Thr Gln Ser Pro Thr Ile Leu Ser Ala Ser Val Gly
1               5                   10                  15
gac aga gtc acc atc act tgc cgg gcc agt cag agt att agt aag tgg       96
Asp Arg Val Thr Ile Thr Cys Arg Ala Ser Gln Ser Ile Ser Lys Trp
            20                  25                  30
ttg gcc tgg tat caa caa aaa cca ggg aaa gtc cct aaa ctc ctg atc      144
Leu Ala Trp Tyr Gln Gln Lys Pro Gly Lys Val Pro Lys Leu Leu Ile
        35                  40                  45
tat atg gcg tct acc tta gaa agt ggg gtc ccg tcg agg ttc agc ggc      192
Tyr Met Ala Ser Thr Leu Glu Ser Gly Val Pro Ser Arg Phe Ser Gly
    50                  55                  60
agt gga tct ggg tca gag ttc act ctc acc atc agc agc ctg cag cct      240
Ser Gly Ser Gly Ser Glu Phe Thr Leu Thr Ile Ser Ser Leu Gln Pro
65                  70                  75                  80
ggt gat ttt gca act tat tac tgc caa cag tat aat agt tat cct cgg      288
Gly Asp Phe Ala Thr Tyr Tyr Cys Gln Gln Tyr Asn Ser Tyr Pro Arg
                85                  90                  95
acg ttc ggc caa ggg acc aag gtg gag atc aac gtc aaa                  327
Thr Phe Gly Gln Gly Thr Lys Val Glu Ile Asn Val Lys
            100                 105
<210>  290
<211>  109
<212>  PRT
<213>  human
<400>  290
Glu Ile Val Leu Thr Gln Ser Pro Thr Ile Leu Ser Ala Ser Val Gly
1               5                   10                  15
Asp Arg Val Thr Ile Thr Cys Arg Ala Ser Gln Ser Ile Ser Lys Trp
            20                  25                  30
Leu Ala Trp Tyr Gln Gln Lys Pro Gly Lys Val Pro Lys Leu Leu Ile
        35                  40                  45
Tyr Met Ala Ser Thr Leu Glu Ser Gly Val Pro Ser Arg Phe Ser Gly
    50                  55                  60
Ser Gly Ser Gly Ser Glu Phe Thr Leu Thr Ile Ser Ser Leu Gln Pro
65                  70                  75                  80
Gly Asp Phe Ala Thr Tyr Tyr Cys Gln Gln Tyr Asn Ser Tyr Pro Arg
                85                  90                  95
Thr Phe Gly Gln Gly Thr Lys Val Glu Ile Asn Val Lys
            100                 105
<210>  291

<211>  15
<212>  PRT
<213> Artificial Sequence
<220>
<223> Description of Artificial Sequence: Linker
<400>  291
Gly Gly Gly Gly Ser Gly Gly Gly Gly Ser Gly Gly Gly Gly Ser
1               5                   10                  15